# EUROPEAN PATENT APPLICATION

(11) **EP 3 998 286 A1**
(43) Date of publication of application: **18.05.2022**
(21) Application number: 19936731.9
(22) Date of filing: 11.07.2019
(51) Int. Cl.: C07K 16/46, C12N 15/09, A61K 39/395, A61P 35/00, G01N 33/00

(54) **TETRAVALENT SYMMETRIC BISPECIFIC ANTIBODY**

(71) Applicant: Wuhan Yzy Biopharma Co., Ltd., Wuhan, Hubei 430075 (CN)
(72) Inventor: ZHANG, Jing, Wuhan, Hubei 430075 (CN); FANG, Lijuan, Wuhan, Hubei 430075 (CN); YAN, Yongxiang, Wuhan, Hubei 430075 (CN); ZENG, Liang, Wuhan, Hubei 430075 (CN); ZHOU, Pengfei, Wuhan, Hubei 430075 (CN)
(74) Representative: Altmann Stößel Dick Patentanwälte PartG mbB
(86) International application number: PCT/CN2019/095603
(87) International publication number: WO 2021/003739

(57) **Abstract**

The present invention relates to a bispecific antibody, which is a trtravalent symmetrical bispecific antibody with a structure of F(ab)₂-(Fv)₂-Fc and comprises two identical fusion heavy chains and two identical fusion light chains.

## Description

### FIELD OF THE INVENTION

The present invention relates to the field of antibodies. Specifically, the present invention relates to bispecific antibodies, pharmaceutical compositions containing them, and uses thereof.

### BACKGROUND OF THE INVENTION

Bispecific antibodies (BsAbs) are antibodies or antibody-like molecules having two different binding specificities. Bispecific antibodies are widely used in biomedicine, especially in immunotherapy for tumors.

Bispecific antibodies can be prepared by methods such as chemical engineering, cell engineering, and genetic engineering. The advantage of genetic engineering is that antibodies can be easily modified, such that many different forms of bispecific antibody fragments, including bispecific IgG (BsIgG), appended IgG, bispecific antibody fragments (BsAb fragments), bispecific fusion proteins and bispecific antibody conjugates (BsAb conjugates), can be designed and produced (see Christoph Spiess, Qianting Zhai, Paul J. Carter, Alternative molecular formats and therapeutic applications for bispecific antibodies. Molecular Immunology 67 (2015) 95-106).

The first type: bispecific IgG, which has a similar structure and molecular weight to natural monoclonal antibodies, but has two different Fabs. (1) In early Triomab technology, rat and mouse hybridomas are re-fused to form a "hybrid-hybridoma" to produce mouse and rat chimeric bispecific antibodies. Purification is performed according to the selectivity of rat and mouse Fc to affinity filler, and then the product of interest is obtained. This technology has disadvantages of low expression amount and low purification yield. (2) In the later stage, Fc is modified by antibody engineering technology such as Lucine zipper, knobs-into-holes or electrostatic steering, so that a heterodimer is formed by two different heavy chains, and then the light chain and the heavy chain are paired correctly by using common light chains, "knobs-into-holes" or electrostatic steering technology, or the correct product of interest is obtained by designing different light chain subtypes κ chain and λ chain and purifying with an affinity filler specific to κ chain and λ chain. Such type of bispecific antibodies have a pharmacokinetics close to that of natural antibodies, but have the disadvantage that it is difficult to identify and remove impurities.

The second type: appended IgG, wherein a natural monoclonal antibody IgG is used as a backbone, and an antigen binding domain (such as a variable region fragment (Fv), single-chain variable region fragment (ScFv), antigen-binding fragment (Fab) or polypeptide, etc.) is fused at the N-terminus or C-terminus of the heavy or light chains of antibody; this type of bispecific antibodies have a molecular weight greater than 150kDa and are mainly of symmetrical structure. The pharmacokinetics of appended IgG is similar to that of natural antibodies, but the appended IgG has a longer half-life, and is convenient for purification. The disadvantages of appended IgG are low expression amount and insufficient stability, etc..

The third type: bispecific antibody fragments, wherein antibody variable region fragments are remained, and part or even all of the antibody constant region are deleted; heavy chain variable region fragments (VH) and light chain variable region fragments (VL) are generally linked to form a ScFv through a linker protein, or are spontaneously paired to form a Fv. The molecular weight of this type of bispecific antibodies does not exceed 150kDa, the half-life thereof is short. The disadvantages of bispecific antibody fragments are as following: it is difficult for the expression level of bispecific antibody fragment to reach that of the monoclonal antibody, the molecule is not stable enough, and the purification yield is low, etc..

The fourth type: bispecific fusion proteins, wherein antibody variable region fragments are retained, and other proteins or pharmaceutical molecules (such as T cell receptor constant regions, human serum albumin, or toxin proteins and the like) are fused. The molecular weight of this type of bispecific antibodies is between 75kDa and 160kDa, the purification process is challenging, and the molecular stability also has certain risks.

The fifth type: bispecific antibody conjugates, wherein two different monoclonal antibodies or ScFv are coupled together by chemical methods. The preparation process of this type of antibodies is complicated and has a low final yield, and thus this type of antibodies is substantially not used in the world at present.

All of the above-mentioned types of bispecific antibodies have some disadvantages, thereby leading to potential risks in clinical use of the products. To eliminate these risks, the present disclosure has invented a novel bispecific antibody.

### SUMMARY OF THE INVENTION

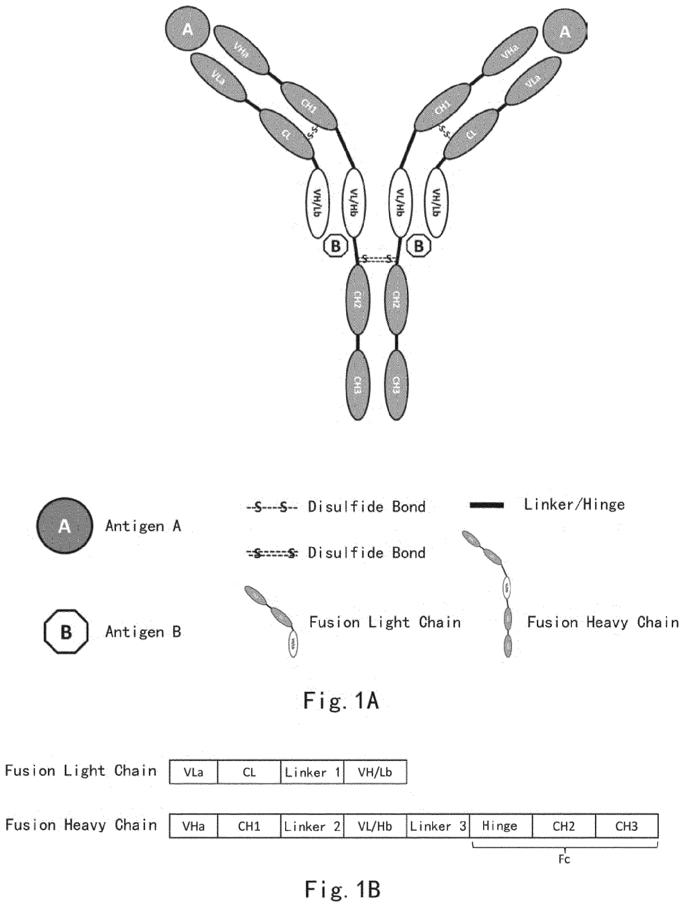

The present disclosure provides a method for construction and preparation of a novel bispecific antibody. The specific structure of the bispecific antibody is shown in Figure 1. All of the antibodies in the present disclosure have a human IgG constant region, and belong to appended IgG or bispecific antibody fragments.

The affinity, stability, biological activity and efficacy of the novel bispecific antibody have been tested in the present disclosure, and compared with three existing bispecific antibodies. The results show that the new bispecific antibody has a better stability, biological activity and efficacy, and the expression and preparation thereof are more convenient.

### TECHNICAL SOLUTIONS

The bispecific antibody of the present disclosure has two identical fusion heavy chains and two identical fusion light chains, wherein the two fusion heavy chains form a pair, and the fusion light chain and the fusion heavy chain form a pair.

In some aspects, the fusion heavy chains of the antibody are bound by one or more disulfide bonds.

In some aspects, the fusion light chain and the fusion heavy chain of the antibody are bound by one or more disulfide bonds.

In some aspects, the fusion heavy chain of the antibody has a heavy chain variable region of antibody a (VHa), a first constant region (CH1), a light chain variable region of antibody b (VLb), and a Fc fragment. Wherein the VLb is located between the CH1 and the Fc, and is linked by a linker or a peptide bond. The fusion light chain of the antibody has a light chain variable region of antibody a (VLa), a light chain constant region (CL) and a heavy chain variable region of antibody b (VHb). Wherein, the VHb is located at the C-terminus of CL and is linked by a linker or a peptide bond.

In some aspects, the fusion heavy chain of the antibody has a heavy chain variable region of antibody a (VHa), a first constant region (CH1), a heavy chain variable region of antibody b (VHb), and a Fc fragment. Wherein the VHb is located between the CH1 and the Fc, and is linked by a linker or a peptide bond. The fusion light chain of the antibody has a light chain variable region of antibody a (VLa), a light chain constant region (CL) and a light chain variable region of antibody b (VLb). Wherein, the VLb is located at the C-terminus of CL and is linked by a linker or a peptide bond.

In some aspects, the VHa-VLa pair is specific to an antigen A, which includes but is not limited to tumor cell surface antigens, immune cell surface antigens, viruses, bacteria, endotoxins, cytokines, such as CD3, SLAMF7, CD38, BCMA, CD16a, CEA, PD-L1, PD-1, CTLA-4, TIGIT, LAG-3, VEGF, B7-H3, TGF-β, IL-10, etc.

In some aspects, the VHb-VLb pair is specific to an antigen B, which includes but is not limited to tumor cell surface antigens, immune cell surface antigens, viruses, bacteria, endotoxins, cytokines, such as CD3, SLAMF7, CD38, BCMA, CD16a, CEA, PD-L1, PD-1, CTLA-4, TIGIT, LAG-3, VEGF, B7-H3, TGF-β, IL-10, etc.

Specifically, in one aspect, the present disclosure provides a bispecific antibody, comprising two identical fusion heavy chains and two identical fusion light chains, wherein the two fusion heavy chains form a pair, and the fusion light chain and the fusion heavy chain form a pair, wherein the fusion heavy chain comprises a heavy chain variable region of antibody a (VHa), a first constant region CHI, a variable region 1 of antibody b and a Fc fragment, and the variable region 1 of antibody b is linked to the CH1 through a linker 2 or a peptide bond, or is linked to the Fc through a linker 3 or a peptide bond. The fusion light chain of the bispecific antibody comprises a light chain variable region of antibody a (VLa), a light chain constant region CL and a variable region 2 of antibody b, wherein the variable region 2 of antibody b is linked to a C terminus of CL through a linker 1 or a peptide bond, wherein,
(1) when the variable region 1 of antibody b is the heavy chain variable region of antibody b (VHb), the variable region 2 of antibody b is the light chain variable region of antibody b (VLb);
   or
(2) when the variable region 1 of antibody b is the light chain variable region of antibody b (VLb), the variable region 2 of antibody b is the heavy chain variable region of antibody b (VHb),
wherein, the VHa-VLa pair targets to the antigen A, and the VHb-VLb pair targets to the antigen B.

In one embodiment, the structure of the bispecific antibody of the present disclosure is F(ab)₂-(Fv)₂-Fc, wherein the F(ab)₂ includes two VHa, two CHI, two VLa and two CL; the (Fv)₂ includes two VH/VLb and two VL/VHb, wherein positions of the VH/VLb and the VL/VHb are interchangeable; and the Fc fragment includes a hinge region and a second constant region CH2 and a third constant region CH3.

In one embodiment, the bispecific antibody symmetrically has a structure shown in formula I from N-terminus to C-terminus: 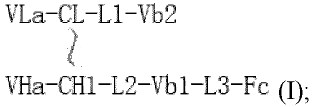 wherein,
"∼" is a disulfide bond or a covalent bond;
"-" is a peptide bond;
L1, L2, and L3 are each independently a peptide bond or a linker or a hinge;
VHa is a heavy chain variable region of antibody a;
VLa is a light chain variable region of antibody a;
CH1 is a first constant region;
Fc is a Fc fragment containing a hinge region;
CL is a light chain constant region;
Vb1 is a variable region 1 of antibody b;
Vb2 is a variable region 2 of antibody b.

In one embodiment, the bispecific antibody symmetrically has a structure shown in Formula II or Formula III from N-terminus to C-terminus: 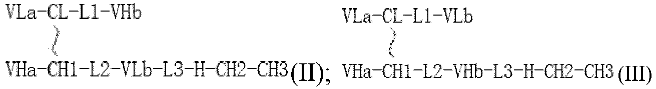 wherein,
"~" is a disulfide bond or a covalent bond;
"-" is a peptide bond;
L1, L2, and L3 are each independently a peptide bond or a linker;
VHa is a heavy chain variable region of antibody a;
VLa is a light chain variable region of antibody a;
CL is a light chain constant region;
H is a hinge region;
CHI, CH2, and CH3 are a first constant region, a second constant region, and a third constant region, respectively;
VHb is a heavy chain variable region of antibody b;
VLb is a light chain variable region of antibody b.

In one embodiment, the bispecific antibody comprises:
(1) a fusion heavy chain, wherein the fusion heavy chain includes a heavy chain variable region of antibody a (VHa), a first constant region CHI, a linker 2 (or a peptide bond), and a heavy chain variable region of antibody b (VHb), a linker 3 (or a peptide bond) and a Fc fragment in sequence from N-terminus to C-terminus; and
(2) a fusion light chain, wherein the fusion light chain includes a light chain variable region of antibody a (VLa), a light chain constant region CL, a linker 1 (or a peptide bond) and a light chain variable region of antibody b (VLb) in sequence from N-terminus to C-terminus.

In one embodiment, the bispecific antibody comprises:
(1) a fusion heavy chain, wherein the fusion heavy chain includes a heavy chain variable region of antibody a (VHa), a first constant region CHI, a linker 2 (or a peptide bond), and a light chain variable region of antibody b (VLb), a linker 3 (or a peptide bond) and a Fc fragment in sequence from N terminus to C terminus; and
(2) a fusion light chain, wherein the fusion light chain includes a light chain variable region of antibody a (VLa), a light chain constant region CL, a linker 1 (or a peptide bond) and a heavy chain variable region of antibody b (VHb) in sequence from N-terminus to C-terminus.

In one embodiment, the VHa-VLa pair forms one or more interchain disulfide bonds, and the VHb-VLb pair forms one or more interchain disulfide bonds.

In one embodiment, the variable region 1 of antibody b is a light chain variable region of antibody b (VLb), and the variable region 2 of antibody b is a heavy chain variable region of antibody b (VHb).

In one embodiment, the linkers 1 to 3 may be the same or different, and the linkers 1 to 3 are each independently selected from a group consisting of SEQ ID NOs: 69 to 90, preferably selected from SEQ ID NOs: 71, 72, 73, 74, 75, 76, 77, 78, 79, 80, 82, 83, 85, 86, 87, 88, 89 or 90.

In one embodiment, the sequence of the hinge region is selected from a group consisting of SEQ ID NOs: 91 to 103.

In one embodiment, the sequence of CL is selected from a group consisting of SEQ ID NOs: 104 to 110.

In one embodiment, the sequence of CH1 is selected from a group consisting of SEQ ID NOs: 111 to 114.

In one embodiment, the sequence of CH2 is selected from a group consisting of SEQ ID NOs: 115 to 119.

In one embodiment, the sequence of CH3 is selected from a group consisting of SEQ ID NOs: 120 to 128.

In one embodiment, the antigen A and antigen B may be the same or different, preferably, the antigen A or antigen B are different or represent different epitopes on the same antigen.

In one embodiment, the antigen A and the antigen B are each independently selected from a group consisting of: immune cell surface antigens, tumor antigens, viruses, bacteria, endotoxins, cytokines, or a combination thereof.

In one embodiment, the antigen A and/or antigen B are selected from PD-L1, PD-1, VEGFA, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, GD3, GM2, RANKL, CD3 and/or CD16a.

In one embodiment, the antigen A and/or the antigen B are selected from SEQ ID NOs: 129 to 145.

In one embodiment, the antigen A and/or antigen B is PD-1; preferably, one of the antigen A and antigen B is PD-1, and the other one is selected from a group consisting of PD-L1, PD-1, VEGFA, IL-10, IL-10R, BCMA, VEGF, TGF-β, CTLA-4, LAG-3, TIGIT, CEA, CD38, SLAMF7, B7-H3, HER2, CD3 or CD16a.

In one embodiment, the antigen A and antigen B are selected from a group consisting of:
PD-L1 and VEGF,
PD-1 and VEGF,
PD-L1 and TGF-β,
PD-1 and TGF-β,
PD-1 and CTLA-4,
PD-1 and LAG-3,
PD-1 and TIGIT,
PD-1 and IL-10,
SLAMF7 and CD16a, and
Her2 and Her2.

In one embodiment, the VHa, VLa, VHb and/or VLb are derived from an antibody selected from a group consisting of animal-derived antibodies (such as murine antibodies), chimeric antibodies, and humanized antibodies; preferably, the humanized antibodies include fully humanized antibodies and partially humanized antibodies.

In one embodiment, the VHa and/or VHb comprise any one of the following sequences:
a) an amino acid sequence shown in any one of SEQ ID NOs: 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 146, 148;
b) an amino acid sequence having a sequence identity of 80%, 85%, 90%, 91%, 92%, 93%, 94%, 95%, 96%, 97%, 98%, 99% or more to the amino acid sequence of a);
c) an amino acid sequence that has one or more (preferably one or several, more preferably 1, 2 or 3) amino acid alterations from the amino acid sequence of a); and/or
   the VLa and/or VLb include any one of the following sequences:
d) an amino acid sequence shown in any one of SEQ ID NOs: 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 147, 149;
e) an amino acid sequence having a sequence identity of 80%, 85%, 90%, 91%, 92%, 93%, 94%, 95%, 96%, 97%, 98%, 99% or more to the amino acid sequence of d);
f) an amino acid sequence has one or more (preferably one or several, more preferably 1, 2, or 3) amino acid alterations from the amino acid sequence of d).

In one embodiment, the bispecific antibody is selected from a group consisting of Y100-A1, Y100-A2, Y100-A3, Y100-A4, Y100-A5, Y100-A6, Y100-A7, Y100-A8, Y100 -A9, Y100-A10, Y100-A11, Y100-AC1, Y100-AC2, Y100-AC3, Y101-A1, Y101-A2, Y101-A3, Y101-A4, Y101-A5, Y103-A1, Y103-A2 , Y103-A3, Y104-A1, Y104-A2, Y104-A3, Y105-A1, Y105-A2, Y105-A3, Y106-A1, Y106-A2, Y106-A3, Y106-A4, Y106-A5, Y110 -A1, Y110-A2, Y110-A3, Y110-A4, Y116-A1, Y116-A2, Y116-A3, Y100-B1, Y100-B2, Y100-B3, Y100-B4, Y100-B5, Y100-B6, Y100-B7, Y100-B8, Y100-B9, Y100-B10, Y100-B11, Y100-B12, Y100-B13, Y100-BC1, Y100-BC2, Y100-BC3, Y101-B1, Y101-B2, Y101 -B3, Y101-B4, Y101-B5, Y101-B6, Y103-B1, Y103-B2, Y103-B3, Y104-B1, Y104-B2, Y104-B3, Y105-B1, Y105-B2, Y105-B3 , Y106-B1, Y106-B2, Y106-B3, Y106-B4, Y106-B5, Y110-B1, Y110-B2, Y110-B3, Y110-B4, Y110-B5, Y116-B1, Y116-B2, Y116 -B3, Y140-A1, Y140-A2, Y140-A3, Y140-A4, Y140-B1, Y140-B2, Y140-B3, Y140-B4, or a combination thereof.

In one embodiment, the bispecific antibody is selected from those shown in any one of Table 27 and Table 34.

In one embodiment, the bispecific antibody is selected from a group consisting of Y100-B6, Y100-B7, Y100-B8, Y100-B9, Y100-B10, Y100-B11, Y100-B12, Y100-BC1, Y100 -BC2, Y100-BC3, Y101-B1, Y101-B2, Y101-B3, Y101-B4, Y101-B5, Y101-B6, Y103-B1, Y103-B2, Y103-B3, Y104-B1, Y104-B2 , Y104-B3, Y105-B1, Y105-B2, Y105-B3, Y106-B1, Y106-B2, Y106-B3, Y106-B4, Y106-B5, Y110-B1, Y110-B2, Y110-B3, Y110 -B4, Y110-B5, Y116-B1, Y116-B2, Y116-B3, Y140-B1, Y140-B2, Y140-B3, Y140-B4, or a combination thereof.

In one embodiment, the bispecific antibody is selected from a group consisting of Y100-B7, Y100-B9, Y101-B2, Y101-B4, Y103-B2, Y104-B2, Y105-B2, Y106-B2, Y106 -B4, Y110-B2, Y110-B4, Y116-B2, or a combination thereof.

In one embodiment, the bispecific antibody is selected from a group consisting of:
(1) a fusion light chain includes SEQ ID NO: 58, SEQ ID NO: 104, SEQ ID NO: 82 and SEQ ID NO: 42; a fusion heavy chain includes SEQ ID NO: 57, SEQ ID NO: 111, SEQ ID NO: 82, SEQ ID NO: 41, SEQ ID NO: 82, SEQ ID NO: 91, SEQ ID NO: 117 and SEQ ID NO: 120;
(2) a fusion light chain includes SEQ ID NO: 58, SEQ ID NO: 104, SEQ ID NO: 89 and SEQ ID NO: 42; a fusion heavy chain includes SEQ ID NO: 57, SEQ ID NO: 111, SEQ ID NO: 83, SEQ ID NO: 41, SEQ ID NO: 74, SEQ ID NO: 91, SEQ ID NO: 117 and SEQ ID NO: 120;
(3) a fusion light chain includes SEQ ID NO: 58, SEQ ID NO: 104, SEQ ID NO: 85 and SEQ ID NO: 42; a fusion heavy chain includes SEQ ID NO: 57, SEQ ID NO: 111, SEQ ID NO: 85, SEQ ID NO: 41, SEQ ID NO: 74, SEQ ID NO: 91, SEQ ID NO: 117 and SEQ ID NO: 120;
(4) a fusion light chain includes SEQ ID NO: 58, SEQ ID NO: 104, SEQ ID NO: 89 and SEQ ID NO: 46; a fusion heavy chain includes SEQ ID NO: 57, SEQ ID NO: 111, SEQ ID NO: 83, SEQ ID NO: 45, SEQ ID NO: 74, SEQ ID NO: 91, SEQ ID NO: 117 and SEQ ID NO: 120;
(5) a fusion light chain includes SEQ ID NO: 58, SEQ ID NO: 104, SEQ ID NO: 85 and SEQ ID NO: 46; a fusion heavy chain includes SEQ ID NO: 57, SEQ ID NO: 111, SEQ ID NO: 85, SEQ ID NO: 45, SEQ ID NO: 74, SEQ ID NO: 91, SEQ ID NO: 117 and SEQ ID NO: 120;
(6) a fusion light chain includes SEQ ID NO: 42, SEQ ID NO: 104, SEQ ID NO: 89 and SEQ ID NO: 58; a fusion heavy chain includes SEQ ID NO: 41, SEQ ID NO: 111, SEQ ID NO: 83, SEQ ID NO: 57, SEQ ID NO: 74, SEQ ID NO: 91, SEQ ID NO: 117 and SEQ ID NO: 120;
(7) a fusion light chain includes SEQ ID NO: 42, SEQ ID NO: 104, SEQ ID NO: 85 and SEQ ID NO: 58; a fusion heavy chain includes SEQ ID NO: 41, SEQ ID NO: 111, SEQ ID NO: 85, SEQ ID NO: 57, SEQ ID NO: 74, SEQ ID NO: 91, SEQ ID NO: 117 and SEQ ID NO: 120;
(8) a fusion light chain includes SEQ ID NO: 46, SEQ ID NO: 104, SEQ ID NO: 89 and SEQ ID NO: 58; a fusion heavy chain includes SEQ ID NO: 45, SEQ ID NO: 111, SEQ ID NO: 83, SEQ ID NO: 57, SEQ ID NO: 74, SEQ ID NO: 91, SEQ ID NO: 117 and SEQ ID NO: 120;
(9) a fusion light chain includes SEQ ID NO: 46, SEQ ID NO: 104, SEQ ID NO: 85 and SEQ ID NO: 58; a fusion heavy chain includes SEQ ID NO: 45, SEQ ID NO: 111, SEQ ID NO: 85, SEQ ID NO: 57, SEQ ID NO: 74, SEQ ID NO: 91, SEQ ID NO: 117 and SEQ ID NO: 120;
(10) a fusion light chain includes SEQ ID NO: 42, SEQ ID NO: 104, SEQ ID NO: 85 and SEQ ID NO: 54; a fusion heavy chain includes SEQ ID NO: 41, SEQ ID NO: 111, SEQ ID NO: 85, SEQ ID NO: 53, SEQ ID NO: 74, SEQ ID NO: 91, SEQ ID NO: 117 and SEQ ID NO: 120;
(11) a fusion light chain includes SEQ ID NO: 42, SEQ ID NO: 104, SEQ ID NO: 85 and SEQ ID NO: 58; a fusion heavy chain includes SEQ ID NO: 41, SEQ ID NO: 111, SEQ ID NO: 85, SEQ ID NO: 57, SEQ ID NO: 74, SEQ ID NO: 91, SEQ ID NO: 115 and SEQ ID NO: 120;
(12) a fusion light chain includes SEQ ID NO: 42, SEQ ID NO: 104, SEQ ID NO: 89 and SEQ ID NO: 68; a fusion heavy chain includes SEQ ID NO: 41, SEQ ID NO: 111, SEQ ID NO: 83, SEQ ID NO: 67, SEQ ID NO: 74, SEQ ID NO: 91, SEQ ID NO: 117 and SEQ ID NO: 120;
(13) a fusion light chain includes SEQ ID NO: 68, SEQ ID NO: 104, SEQ ID NO: 85 and SEQ ID NO: 58; a fusion heavy chain includes SEQ ID NO: 67, SEQ ID NO: 111, SEQ ID NO: 85, SEQ ID NO: 57, SEQ ID NO: 74, SEQ ID NO: 91, SEQ ID NO: 117 and SEQ ID NO: 120;
(14) a fusion light chain includes SEQ ID NO: 42, SEQ ID NO: 104, SEQ ID NO: 89 and SEQ ID NO: 60; a fusion heavy chain includes SEQ ID NO: 41, SEQ ID NO: 111, SEQ ID NO: 83, SEQ ID NO: 59, SEQ ID NO: 74, SEQ ID NO: 91, SEQ ID NO: 117 and SEQ ID NO: 120;
(15) a fusion light chain includes SEQ ID NO: 42, SEQ ID NO: 104, SEQ ID NO: 85 and SEQ ID NO: 60; a fusion heavy chain includes SEQ ID NO: 41, SEQ ID NO: 111, SEQ ID NO: 85, SEQ ID NO: 59, SEQ ID NO: 74, SEQ ID NO: 91, SEQ ID NO: 117 and SEQ ID NO: 120;
(16) a fusion light chain includes SEQ ID NO: 46, SEQ ID NO: 104, SEQ ID NO: 89 and SEQ ID NO: 60; a fusion heavy chain includes SEQ ID NO: 45, SEQ ID NO: 111, SEQ ID NO: 83, SEQ ID NO: 59, SEQ ID NO: 74, SEQ ID NO: 91, SEQ ID NO: 117 and SEQ ID NO: 120;
(17) a fusion light chain includes SEQ ID NO: 46, SEQ ID NO: 104, SEQ ID NO: 85 and SEQ ID NO: 60; a fusion heavy chain includes SEQ ID NO: 45, SEQ ID NO: 111, SEQ ID NO: 85, SEQ ID NO: 59, SEQ ID NO: 74, SEQ ID NO: 91, SEQ ID NO: 117 and SEQ ID NO: 120;
(18) a fusion light chain includes SEQ ID NO: 42, SEQ ID NO: 104, SEQ ID NO: 85 and SEQ ID NO: 60; a fusion heavy chain includes SEQ ID NO: 41, SEQ ID NO: 111, SEQ ID NO: 85, SEQ ID NO: 59, SEQ ID NO: 74, SEQ ID NO: 91, SEQ ID NO: 115 and SEQ ID NO: 120;
(19) a fusion light chain includes SEQ ID NO: 42, SEQ ID NO: 104, SEQ ID NO: 89 and SEQ ID NO: 34; a fusion heavy chain includes SEQ ID NO: 41, SEQ ID NO: 111, SEQ ID NO: 83, SEQ ID NO: 33, SEQ ID NO: 74, SEQ ID NO: 91, SEQ ID NO: 117 and SEQ ID NO: 120;
(20) a fusion light chain includes SEQ ID NO: 42, SEQ ID NO: 104, SEQ ID NO: 85 and SEQ ID NO: 34; a fusion heavy chain includes SEQ ID NO: 41, SEQ ID NO: 111, SEQ ID NO: 85, SEQ ID NO: 33, SEQ ID NO: 74, SEQ ID NO: 91, SEQ ID NO: 117 and SEQ ID NO: 120;
(21) a fusion light chain includes SEQ ID NO: 42, SEQ ID NO: 104, SEQ ID NO: 85 and SEQ ID NO: 34; a fusion heavy chain includes SEQ ID NO: 41, SEQ ID NO: 111, SEQ ID NO: 85, SEQ ID NO: 33, SEQ ID NO: 74, SEQ ID NO: 91, SEQ ID NO: 115 and SEQ ID NO: 120;
(22) a fusion light chain includes SEQ ID NO: 38, SEQ ID NO: 104, SEQ ID NO: 89 and SEQ ID NO: 28; a fusion heavy chain includes SEQ ID NO: 37, SEQ ID NO: 111, SEQ ID NO: 83, SEQ ID NO: 27, SEQ ID NO: 74, SEQ ID NO: 91, SEQ ID NO: 117 and SEQ ID NO: 120;
(23) a fusion light chain includes SEQ ID NO: 38, SEQ ID NO: 104, SEQ ID NO: 85 and SEQ ID NO: 28; a fusion heavy chain includes SEQ ID NO: 37, SEQ ID NO: 111, SEQ ID NO: 85, SEQ ID NO: 27, SEQ ID NO: 74, SEQ ID NO: 91, SEQ ID NO: 117 and SEQ ID NO: 120;
(24) a fusion light chain includes SEQ ID NO: 38, SEQ ID NO: 104, SEQ ID NO: 85 and SEQ ID NO: 28; a fusion heavy chain includes SEQ ID NO: 37, SEQ ID NO: 111, SEQ ID NO: 85, SEQ ID NO: 27, SEQ ID NO: 74, SEQ ID NO: 91, SEQ ID NO: 115 and SEQ ID NO: 120;
(25) a fusion light chain includes SEQ ID NO: 38, SEQ ID NO: 104, SEQ ID NO: 89 and SEQ ID NO: 30; a fusion heavy chain includes SEQ ID NO: 37, SEQ ID NO: 111, SEQ ID NO: 83, SEQ ID NO: 29, SEQ ID NO: 74, SEQ ID NO: 91, SEQ ID NO: 117 and SEQ ID NO: 120;
(26) a fusion light chain includes SEQ ID NO: 38, SEQ ID NO: 104, SEQ ID NO: 85 and SEQ ID NO: 30; a fusion heavy chain includes SEQ ID NO: 37, SEQ ID NO: 111, SEQ ID NO: 85, SEQ ID NO: 29, SEQ ID NO: 74, SEQ ID NO: 91, SEQ ID NO: 117 and SEQ ID NO: 120;
(27) a fusion light chain includes SEQ ID NO: 38, SEQ ID NO: 104, SEQ ID NO: 85 and SEQ ID NO: 30; a fusion heavy chain includes SEQ ID NO: 37, SEQ ID NO: 111, SEQ ID NO: 85, SEQ ID NO: 29, SEQ ID NO: 74, SEQ ID NO: 91, SEQ ID NO: 115 and SEQ ID NO: 120;
(28) a fusion light chain includes SEQ ID NO: 38, SEQ ID NO: 104, SEQ ID NO: 89 and SEQ ID NO: 58; a fusion heavy chain includes SEQ ID NO: 37, SEQ ID NO: 111, SEQ ID NO: 83, SEQ ID NO: 57, SEQ ID NO: 74, SEQ ID NO: 91, SEQ ID NO: 117 and SEQ ID NO: 120;
(29) a fusion light chain includes SEQ ID NO: 38, SEQ ID NO: 104, SEQ ID NO: 85 and SEQ ID NO: 58; a fusion heavy chain includes SEQ ID NO: 37, SEQ ID NO: 111, SEQ ID NO: 85, SEQ ID NO: 57, SEQ ID NO: 74, SEQ ID NO: 91, SEQ ID NO: 117 and SEQ ID NO: 120;
(30) a fusion light chain includes SEQ ID NO: 38, SEQ ID NO: 104, SEQ ID NO: 89 and SEQ ID NO: 60; a fusion heavy chain includes SEQ ID NO: 37, SEQ ID NO: 111, SEQ ID NO: 83, SEQ ID NO: 59, SEQ ID NO: 74, SEQ ID NO: 91, SEQ ID NO: 117 and SEQ ID NO: 120;
(31) a fusion light chain includes SEQ ID NO: 38, SEQ ID NO: 104, SEQ ID NO: 85 and SEQ ID NO: 60; a fusion heavy chain includes SEQ ID NO: 37, SEQ ID NO: 111, SEQ ID NO: 85, SEQ ID NO: 59, SEQ ID NO: 74, SEQ ID NO: 91, SEQ ID NO: 117 and SEQ ID NO: 120;
(32) a fusion light chain includes SEQ ID NO: 38, SEQ ID NO: 104, SEQ ID NO: 85 and SEQ ID NO: 60; a fusion heavy chain includes SEQ ID NO: 37, SEQ ID NO: 111, SEQ ID NO: 85, SEQ ID NO: 59, SEQ ID NO: 74, SEQ ID NO: 91, SEQ ID NO: 115 and SEQ ID NO: 120;
(33) a fusion light chain includes SEQ ID NO: 38, SEQ ID NO: 104, SEQ ID NO: 89 and SEQ ID NO: 64; a fusion heavy chain includes SEQ ID NO: 37, SEQ ID NO: 111, SEQ ID NO: 83, SEQ ID NO: 63, SEQ ID NO: 74, SEQ ID NO: 91, SEQ ID NO: 117 and SEQ ID NO: 120;
(34) a fusion light chain includes SEQ ID NO: 38, SEQ ID NO: 104, SEQ ID NO: 85 and SEQ ID NO: 64; a fusion heavy chain includes SEQ ID NO: 37, SEQ ID NO: 111, SEQ ID NO: 85, SEQ ID NO: 63, SEQ ID NO: 74, SEQ ID NO: 91, SEQ ID NO: 117 and SEQ ID NO: 120;
(35) a fusion light chain includes SEQ ID NO: 46, SEQ ID NO: 104, SEQ ID NO: 89 and SEQ ID NO: 64; a fusion heavy chain includes SEQ ID NO: 45, SEQ ID NO: 111, SEQ ID NO: 83, SEQ ID NO: 63, SEQ ID NO: 74, SEQ ID NO: 91, SEQ ID NO: 117 and SEQ ID NO: 120;
(36) a fusion light chain includes SEQ ID NO: 46, SEQ ID NO: 104, SEQ ID NO: 85 and SEQ ID NO: 64; a fusion heavy chain includes SEQ ID NO: 45, SEQ ID NO: 111, SEQ ID NO: 85, SEQ ID NO: 63, SEQ ID NO: 74, SEQ ID NO: 91, SEQ ID NO: 115 and SEQ ID NO: 120;
(37) a fusion light chain includes SEQ ID NO: 50, SEQ ID NO: 104, SEQ ID NO: 89 and SEQ ID NO: 48; a fusion heavy chain includes SEQ ID NO: 49, SEQ ID NO: 111, SEQ ID NO: 83, SEQ ID NO: 47, SEQ ID NO: 74, SEQ ID NO: 91, SEQ ID NO: 117 and SEQ ID NO: 120;
(38) a fusion light chain includes SEQ ID NO: 50, SEQ ID NO: 104, SEQ ID NO: 85 and SEQ ID NO: 48; a fusion heavy chain includes SEQ ID NO: 49, SEQ ID NO: 111, SEQ ID NO: 85, SEQ ID NO: 47, SEQ ID NO: 74, SEQ ID NO: 91, SEQ ID NO: 117 and SEQ ID NO: 120;
(39) a fusion light chain includes SEQ ID NO: 50, SEQ ID NO: 104, SEQ ID NO: 85 and SEQ ID NO: 48; a fusion heavy chain includes SEQ ID NO: 49, SEQ ID NO: 111, SEQ ID NO: 85, SEQ ID NO: 47, SEQ ID NO: 74, SEQ ID NO: 91, SEQ ID NO: 115 and SEQ ID NO: 120;
(40) a fusion light chain includes SEQ ID NO: 58, SEQ ID NO: 104, SEQ ID NO: 82 and SEQ ID NO: 41; a fusion heavy chain includes SEQ ID NO: 57, SEQ ID NO: 111, SEQ ID NO: 82, SEQ ID NO: 42, SEQ ID NO: 82, SEQ ID NO: 91, SEQ ID NO: 117 and SEQ ID NO: 120;
(41) a fusion light chain includes SEQ ID NO: 58, SEQ ID NO: 104, SEQ ID NO: 89 and SEQ ID NO: 41; a fusion heavy chain includes SEQ ID NO: 57, SEQ ID NO: 111, SEQ ID NO: 83, SEQ ID NO: 42, SEQ ID NO: 74, SEQ ID NO: 91, SEQ ID NO: 117 and SEQ ID NO: 120;
(42) a fusion light chain includes SEQ ID NO: 58, SEQ ID NO: 104, SEQ ID NO: 85 and SEQ ID NO: 41; a fusion heavy chain includes SEQ ID NO: 57, SEQ ID NO: 111, SEQ ID NO: 85, SEQ ID NO: 42, SEQ ID NO: 74, SEQ ID NO: 91, SEQ ID NO: 117 and SEQ ID NO: 120;
(43) a fusion light chain includes SEQ ID NO: 58, SEQ ID NO: 104, SEQ ID NO: 89 and SEQ ID NO: 45; a fusion heavy chain includes SEQ ID NO: 57, SEQ ID NO: 111, SEQ ID NO: 83, SEQ ID NO: 46, SEQ ID NO: 74, SEQ ID NO: 91, SEQ ID NO: 117 and SEQ ID NO: 120;
(44) a fusion light chain includes SEQ ID NO: 58, SEQ ID NO: 104, SEQ ID NO: 85 and SEQ ID NO: 45; a fusion heavy chain includes SEQ ID NO: 57, SEQ ID NO: 111, SEQ ID NO: 85, SEQ ID NO: 46, SEQ ID NO: 74, SEQ ID NO: 91, SEQ ID NO: 117 and SEQ ID NO: 120;
(45) a fusion light chain includes SEQ ID NO: 42, SEQ ID NO: 104, SEQ ID NO: 89 and SEQ ID NO: 57; a fusion heavy chain includes SEQ ID NO: 41, SEQ ID NO: 111, SEQ ID NO: 83, SEQ ID NO: 58, SEQ ID NO: 74, SEQ ID NO: 91, SEQ ID NO: 117 and SEQ ID NO: 120;
(46) a fusion light chain includes SEQ ID NO: 42, SEQ ID NO: 104, SEQ ID NO: 85 and SEQ ID NO: 57; a fusion heavy chain includes SEQ ID NO: 41, SEQ ID NO: 111, SEQ ID NO: 85, SEQ ID NO: 58, SEQ ID NO: 74, SEQ ID NO: 91, SEQ ID NO: 117 and SEQ ID NO: 120;
(47) a fusion light chain includes SEQ ID NO: 46, SEQ ID NO: 104, SEQ ID NO: 89 and SEQ ID NO: 57; a fusion heavy chain includes SEQ ID NO: 45, SEQ ID NO: 111, SEQ ID NO: 83, SEQ ID NO: 58, SEQ ID NO: 74, SEQ ID NO: 91, SEQ ID NO: 117 and SEQ ID NO: 120;
(48) a fusion light chain includes SEQ ID NO: 46, SEQ ID NO: 104, SEQ ID NO: 85 and SEQ ID NO: 57; a fusion heavy chain includes SEQ ID NO: 45, SEQ ID NO: 111, SEQ ID NO: 85, SEQ ID NO: 58, SEQ ID NO: 74, SEQ ID NO: 91, SEQ ID NO: 117 and SEQ ID NO: 120;
(49) a fusion light chain includes SEQ ID NO: 42, SEQ ID NO: 104, SEQ ID NO: 85 and SEQ ID NO: 53; a fusion heavy chain includes SEQ ID NO: 41, SEQ ID NO: 111, SEQ ID NO: 85, SEQ ID NO:54, SEQ ID NO:74, SEQ ID NO:91, SEQ ID NO: 117 and SEQ ID NO: 120;
(50) a fusion light chain includes SEQ ID NO: 42, SEQ ID NO: 104, SEQ ID NO: 84 and SEQ ID NO: 57; a fusion heavy chain includes SEQ ID NO: 41, SEQ ID NO: 111, SEQ ID NO: 84, SEQ ID NO: 58, SEQ ID NO: 74, SEQ ID NO: 91, SEQ ID NO: 117 and SEQ ID NO: 120;
(51) a fusion light chain includes SEQ ID NO: 42, SEQ ID NO: 104, SEQ ID NO: 86 and SEQ ID NO: 57; a fusion heavy chain includes SEQ ID NO: 41, SEQ ID NO: 111, SEQ ID NO: 86, SEQ ID NO: 58, SEQ ID NO: 74, SEQ ID NO: 91, SEQ ID NO: 117 and SEQ ID NO: 120;
(52) a fusion light chain includes SEQ ID NO: 42, SEQ ID NO: 104, SEQ ID NO: 85 and SEQ ID NO: 57; a fusion heavy chain includes SEQ ID NO: 41, SEQ ID NO: 111, SEQ ID NO: 85, SEQ ID NO: 58, SEQ ID NO: 74, SEQ ID NO: 91, SEQ ID NO: 115 and SEQ ID NO: 120;
(53) a fusion light chain includes SEQ ID NO: 42, SEQ ID NO: 104, SEQ ID NO: 85 and SEQ ID NO: 67; a fusion heavy chain includes SEQ ID NO: 41, SEQ ID NO: 111, SEQ ID NO: 85, SEQ ID NO: 68, SEQ ID NO: 74, SEQ ID NO: 91, SEQ ID NO: 117 and SEQ ID NO: 120;
(54) a fusion light chain includes SEQ ID NO: 68, SEQ ID NO: 104, SEQ ID NO: 85 and SEQ ID NO: 57; a fusion heavy chain includes SEQ ID NO: 67, SEQ ID NO: 111, SEQ ID NO: 85, SEQ ID NO: 58, SEQ ID NO: 74, SEQ ID NO: 91, SEQ ID NO: 117 and SEQ ID NO: 120;
(55) a fusion light chain includes SEQ ID NO: 68, SEQ ID NO: 104, SEQ ID NO: 85 and SEQ ID NO: 57; a fusion heavy chain includes SEQ ID NO: 67, SEQ ID NO: 111, SEQ ID NO: 85, SEQ ID NO: 58, SEQ ID NO: 74, SEQ ID NO: 91, SEQ ID NO: 115 and SEQ ID NO: 120;
(56) a fusion light chain includes SEQ ID NO: 42, SEQ ID NO: 104, SEQ ID NO: 89 and SEQ ID NO: 59; a fusion heavy chain includes SEQ ID NO: 41, SEQ ID NO: 111, SEQ ID NO: 83, SEQ ID NO: 60, SEQ ID NO: 74, SEQ ID NO: 91, SEQ ID NO: 117 and SEQ ID NO: 120;
(57) a fusion light chain includes SEQ ID NO: 42, SEQ ID NO: 104, SEQ ID NO: 85 and SEQ ID NO: 59; a fusion heavy chain includes SEQ ID NO: 41, SEQ ID NO: 111, SEQ ID NO: 85, SEQ ID NO: 60, SEQ ID NO: 74, SEQ ID NO: 91, SEQ ID NO: 117 and SEQ ID NO: 120;
(58) a fusion light chain includes SEQ ID NO: 46, SEQ ID NO: 104, SEQ ID NO: 89 and SEQ ID NO: 59; a fusion heavy chain includes SEQ ID NO: 45, SEQ ID NO: 111, SEQ ID NO: 83, SEQ ID NO: 60, SEQ ID NO: 74, SEQ ID NO: 91, SEQ ID NO: 117 and SEQ ID NO: 120;
(59) a fusion light chain includes SEQ ID NO: 46, SEQ ID NO: 104, SEQ ID NO: 85 and SEQ ID NO: 59; a fusion heavy chain includes SEQ ID NO: 45, SEQ ID NO: 111, SEQ ID NO: 85, SEQ ID NO: 60, SEQ ID NO: 74, SEQ ID NO: 91, SEQ ID NO: 117 and SEQ ID NO: 120;
(60) a fusion light chain includes SEQ ID NO: 42, SEQ ID NO: 104, SEQ ID NO: 84 and SEQ ID NO: 59; a fusion heavy chain includes SEQ ID NO: 41, SEQ ID NO: 111, SEQ ID NO: 84, SEQ ID NO: 60, SEQ ID NO: 74, SEQ ID NO: 91, SEQ ID NO: 117 and SEQ ID NO: 120;
(61) a fusion light chain includes SEQ ID NO: 42, SEQ ID NO: 104, SEQ ID NO: 86 and SEQ ID NO: 59; a fusion heavy chain includes SEQ ID NO: 41, SEQ ID NO: 111, SEQ ID NO: 86, SEQ ID NO: 60, SEQ ID NO: 74, SEQ ID NO: 91, SEQ ID NO: 117 and SEQ ID NO: 120;
(62) a fusion light chain includes SEQ ID NO: 42, SEQ ID NO: 104, SEQ ID NO: 89 and SEQ ID NO: 33; a fusion heavy chain includes SEQ ID NO: 41, SEQ ID NO: 111, SEQ ID NO: 83, SEQ ID NO: 34, SEQ ID NO: 74, SEQ ID NO: 91, SEQ ID NO: 117 and SEQ ID NO: 120;
(63) a fusion light chain includes SEQ ID NO: 42, SEQ ID NO: 104, SEQ ID NO: 86 and SEQ ID NO: 33; a fusion heavy chain includes SEQ ID NO: 41, SEQ ID NO: 111, SEQ ID NO: 86, SEQ ID NO: 34, SEQ ID NO: 74, SEQ ID NO: 91, SEQ ID NO: 117 and SEQ ID NO: 120;
(64) a fusion light chain includes SEQ ID NO: 42, SEQ ID NO: 104, SEQ ID NO: 86 and SEQ ID NO: 33; a fusion heavy chain includes SEQ ID NO: 41, SEQ ID NO: 111, SEQ ID NO: 86, SEQ ID NO: 34, SEQ ID NO: 74, SEQ ID NO: 91, SEQ ID NO: 115 and SEQ ID NO: 120;
(65) a fusion light chain includes SEQ ID NO: 38, SEQ ID NO: 104, SEQ ID NO: 89 and SEQ ID NO: 27; a fusion heavy chain includes SEQ ID NO: 37, SEQ ID NO: 111, SEQ ID NO: 83, SEQ ID NO: 28, SEQ ID NO: 74, SEQ ID NO: 91, SEQ ID NO: 117 and SEQ ID NO: 120;
(66) a fusion light chain includes SEQ ID NO: 38, SEQ ID NO: 104, SEQ ID NO: 85 and SEQ ID NO: 27; a fusion heavy chain includes SEQ ID NO: 37, SEQ ID NO: 111, SEQ ID NO: 85, SEQ ID NO: 28, SEQ ID NO: 74, SEQ ID NO: 91, SEQ ID NO: 117 and SEQ ID NO: 120;
(67) a fusion light chain includes SEQ ID NO: 38, SEQ ID NO: 104, SEQ ID NO: 85 and SEQ ID NO: 27; a fusion heavy chain includes SEQ ID NO: 37, SEQ ID NO: 111, SEQ ID NO: 85, SEQ ID NO: 28, SEQ ID NO: 74, SEQ ID NO: 91, SEQ ID NO: 115 and SEQ ID NO: 120;
(68) a fusion light chain includes SEQ ID NO: 38, SEQ ID NO: 104, SEQ ID NO: 89 and SEQ ID NO: 29; a fusion heavy chain includes SEQ ID NO: 37, SEQ ID NO: 111, SEQ ID NO: 83, SEQ ID NO: 30, SEQ ID NO: 74, SEQ ID NO: 91, SEQ ID NO: 117 and SEQ ID NO: 120;
(69) a fusion light chain includes SEQ ID NO: 38, SEQ ID NO: 104, SEQ ID NO: 85 and SEQ ID NO: 29; a fusion heavy chain includes SEQ ID NO: 37, SEQ ID NO: 111, SEQ ID NO: 85, SEQ ID NO: 30, SEQ ID NO: 74, SEQ ID NO: 91, SEQ ID NO: 117 and SEQ ID NO: 120;
(70) a fusion light chain includes SEQ ID NO: 38, SEQ ID NO: 104, SEQ ID NO: 85 and SEQ ID NO: 29; a fusion heavy chain includes SEQ ID NO: 37, SEQ ID NO: 111, SEQ ID NO: 85, SEQ ID NO: 30, SEQ ID NO: 74, SEQ ID NO: 91, SEQ ID NO: 115 and SEQ ID NO: 120;
(71) a fusion light chain includes SEQ ID NO: 38, SEQ ID NO: 104, SEQ ID NO: 89 and SEQ ID NO: 57; a fusion heavy chain includes SEQ ID NO: 37, SEQ ID NO: 111, SEQ ID NO: 83, SEQ ID NO: 58, SEQ ID NO: 74, SEQ ID NO: 91, SEQ ID NO: 117 and SEQ ID NO: 120;
(72) a fusion light chain includes SEQ ID NO: 38, SEQ ID NO: 104, SEQ ID NO: 85 and SEQ ID NO: 57; a fusion heavy chain includes SEQ ID NO: 37, SEQ ID NO: 111, SEQ ID NO: 85, SEQ ID NO: 58, SEQ ID NO: 74, SEQ ID NO: 91, SEQ ID NO: 117 and SEQ ID NO: 120;
(73) a fusion light chain includes SEQ ID NO: 38, SEQ ID NO: 104, SEQ ID NO: 89 and SEQ ID NO: 59; a fusion heavy chain includes SEQ ID NO: 37, SEQ ID NO: 111, SEQ ID NO: 83, SEQ ID NO: 60, SEQ ID NO: 74, SEQ ID NO: 91, SEQ ID NO: 117 and SEQ ID NO: 120;
(74) a fusion light chain includes SEQ ID NO: 38, SEQ ID NO: 104, SEQ ID NO: 85 and SEQ ID NO: 59; a fusion heavy chain includes SEQ ID NO: 37, SEQ ID NO: 111, SEQ ID NO: 85, SEQ ID NO: 60, SEQ ID NO: 74, SEQ ID NO: 91, SEQ ID NO: 117 and SEQ ID NO: 120;
(75) a fusion light chain includes SEQ ID NO: 38, SEQ ID NO: 104, SEQ ID NO: 85 and SEQ ID NO: 59; a fusion heavy chain includes SEQ ID NO: 37, SEQ ID NO: 111, SEQ ID NO: 85, SEQ ID NO: 60, SEQ ID NO: 74, SEQ ID NO: 91, SEQ ID NO: 115 and SEQ ID NO: 120;
(76) a fusion light chain includes SEQ ID NO: 38, SEQ ID NO: 104, SEQ ID NO: 89 and SEQ ID NO: 63; a fusion heavy chain includes SEQ ID NO: 37, SEQ ID NO: 111, SEQ ID NO: 83, SEQ ID NO: 64, SEQ ID NO: 74, SEQ ID NO: 91, SEQ ID NO: 117 and SEQ ID NO: 120;
(77) a fusion light chain includes SEQ ID NO: 38, SEQ ID NO: 104, SEQ ID NO: 85 and SEQ ID NO: 63; a fusion heavy chain includes SEQ ID NO: 37, SEQ ID NO: 111, SEQ ID NO: 85, SEQ ID NO: 64, SEQ ID NO: 74, SEQ ID NO: 91, SEQ ID NO: 115 and SEQ ID NO: 120;
(78) a fusion light chain includes SEQ ID NO: 46, SEQ ID NO: 104, SEQ ID NO: 89 and SEQ ID NO: 63; a fusion heavy chain includes SEQ ID NO: 45, SEQ ID NO: 111, SEQ ID NO: 83, SEQ ID NO: 64, SEQ ID NO: 74, SEQ ID NO: 91, SEQ ID NO: 117 and SEQ ID NO: 120;
(79) a fusion light chain includes SEQ ID NO: 46, SEQ ID NO: 104, SEQ ID NO: 85 and SEQ ID NO: 63; a fusion heavy chain includes SEQ ID NO: 45, SEQ ID NO: 111, SEQ ID NO: 85, SEQ ID NO: 64, SEQ ID NO: 74, SEQ ID NO: 91, SEQ ID NO: 117 and SEQ ID NO: 120;
(80) a fusion light chain includes SEQ ID NO: 46, SEQ ID NO: 104, SEQ ID NO: 85 and SEQ ID NO: 63; a fusion heavy chain includes SEQ ID NO: 45, SEQ ID NO: 111, SEQ ID NO: 85, SEQ ID NO: 64, SEQ ID NO: 74, SEQ ID NO: 91, SEQ ID NO: 115 and SEQ ID NO: 120;
(80) a fusion light chain includes SEQ ID NO: 50, SEQ ID NO: 104, SEQ ID NO: 89 and SEQ ID NO: 47; a fusion heavy chain includes SEQ ID NO: 49, SEQ ID NO: 111, SEQ ID NO: 83, SEQ ID NO: 48, SEQ ID NO: 74, SEQ ID NO: 91, SEQ ID NO: 117 and SEQ ID NO: 120;
(81) a fusion light chain includes SEQ ID NO: 50, SEQ ID NO: 104, SEQ ID NO: 85 and SEQ ID NO: 47; a fusion heavy chain includes SEQ ID NO: 49, SEQ ID NO: 111, SEQ ID NO: 85, SEQ ID NO: 48, SEQ ID NO: 74, SEQ ID NO: 91, SEQ ID NO: 117 and SEQ ID NO: 120;
(82) a fusion light chain includes SEQ ID NO: 50, SEQ ID NO: 104, SEQ ID NO: 85 and SEQ ID NO: 47; a fusion heavy chain includes SEQ ID NO: 49, SEQ ID NO: 111, SEQ ID NO: 85, SEQ ID NO: 48, SEQ ID NO: 74, SEQ ID NO: 91, SEQ ID NO: 115 and SEQ ID NO: 120;
(83) a fusion light chain includes SEQ ID NO: 147, SEQ ID NO: 104, SEQ ID NO: 85 and SEQ ID NO: 149; a fusion heavy chain includes SEQ ID NO: 146, SEQ ID NO: 111, SEQ ID NO: 85, SEQ ID NO: 148, SEQ ID NO: 82, SEQ ID NO: 91, SEQ ID NO: 117 and SEQ ID NO: 120;
(84) a fusion light chain includes SEQ ID NO: 149, SEQ ID NO: 104, SEQ ID NO: 85 and SEQ ID NO: 147; a fusion heavy chain includes SEQ ID NO: 148, SEQ ID NO: 111, SEQ ID NO: 85, SEQ ID NO: 146, SEQ ID NO: 82, SEQ ID NO: 91, SEQ ID NO: 117 and SEQ ID NO: 120;
(85) a fusion light chain includes SEQ ID NO: 147, SEQ ID NO: 104, SEQ ID NO: 85 and SEQ ID NO: 149; a fusion heavy chain includes SEQ ID NO: 146, SEQ ID NO: 111, SEQ ID NO: 85, SEQ ID NO: 148, SEQ ID NO: 82, SEQ ID NO: 91, SEQ ID NO: 115 and SEQ ID NO: 120;
(86) a fusion light chain comprises SEQ ID NO: 149, SEQ ID NO: 104, SEQ ID NO: 85 and SEQ ID NO: 147; a fusion heavy chain comprises SEQ ID NO: 148, SEQ ID NO: 111, SEQ ID NO: 85, SEQ ID NO: 146, SEQ ID NO: 82, SEQ ID NO: 91, SEQ ID NO: 115 and SEQ ID NO: 120;
(87) a fusion light chain includes SEQ ID NO: 147, SEQ ID NO: 104, SEQ ID NO: 85 and SEQ ID NO: 148; a fusion heavy chain includes SEQ ID NO: 146, SEQ ID NO: 111, SEQ ID NO: 85, SEQ ID NO: 149, SEQ ID NO: 87, SEQ ID NO: 91, SEQ ID NO: 117 and SEQ ID NO: 120;
(88) a fusion light chain includes SEQ ID NO: 149, SEQ ID NO: 104, SEQ ID NO: 85 and SEQ ID NO: 146; a fusion heavy chain includes SEQ ID NO: 148, SEQ ID NO: 111, SEQ ID NO: 85, SEQ ID NO: 147, SEQ ID NO: 82, SEQ ID NO: 91, SEQ ID NO: 117 and SEQ ID NO: 120;
(89) a fusion light chain includes SEQ ID NO: 147, SEQ ID NO: 104, SEQ ID NO: 85 and SEQ ID NO: 148; a fusion heavy chain includes SEQ ID NO: 146, SEQ ID NO: 111, SEQ ID NO: 85, SEQ ID NO: 149, SEQ ID NO: 82, SEQ ID NO: 91, SEQ ID NO: 115 and SEQ ID NO: 120; or
(90) a fusion light chain includes SEQ ID NO: 149, SEQ ID NO: 104, SEQ ID NO: 85 and SEQ ID NO: 146; a fusion heavy chain includes SEQ ID NO: 148, SEQ ID NO: 111, SEQ ID NO: 85, SEQ ID NO: 147, SEQ ID NO: 82, SEQ ID NO: 91, SEQ ID NO: 115 and SEQ ID NO: 120.

In one embodiment, the bispecific antibody has an activity of simultaneously binding to the antigen A and the antigen B.

In one embodiment, the bispecific antibody is a biparatopic antibody.

In another aspect, the present disclosure provides a conjugate or a fusion protein comprising the bispecific antibodies of the present disclosure.

In a specific embodiment, the conjugate or fusion protein comprises a substance A conjugated or fused to the antibody, and the substance A is selected from a group consisting of therapeutic agents, prodrugs, proteins (such as enzymes), viruses, lipids, biological response modifiers (such as immunomodulators), PEG, hormones, oligonucleotides, diagnostic agents, cytotoxic agents which can be drugs or toxins, ultrasound enhancers, non-radioactive markers, detectable markers, such as chemiluminescent labeling compounds (such as luminol, isoluminol, thermal acridinium ester, imidazole, acridinium salt and oxalate), or fluorescence emitting metals (such as 152Eu, or lanthanide labels).

In one embodiment, the conjugate or fusion protein is a monomer, dimer or multimer.

In another aspect, the present disclosure provides a pharmaceutical composition comprising the bispecific antibody of the present disclosure or the conjugate or fusion protein of the present disclosure.

In one embodiment, the pharmaceutical composition further comprises a pharmaceutically acceptable carrier.

In one embodiment, dosage form of the pharmaceutical composition includes a dosage form for gastrointestinal administration or a dosage form for parenteral administration; preferably, the dosage form of the pharmaceutical composition is an injection, including intravenous injection, intravenous drip, subcutaneous injection, local injection, intramuscular injection, intratumoral injection, intraperitoneal injection, intracranial injection, or intracavity injection.

In yet another aspect, the present disclosure provides a polynucleotide encoding the bispecific antibody of the present disclosure.

In one embodiment, the polynucleotide has a first polynucleotide encoding the fusion light chain of the bispecific antibody and a second polynucleotide encoding the fusion heavy chain. Preferably, the ratio of the first polynucleotide to the second polynucleotide is 1:1.

In another aspect, the present disclosure provides a vector comprising the polynucleotide of the present disclosure; preferably, the vector comprises: plasmids, phages, yeast plasmids, plant cell viruses, mammalian cell viruses such as adenoviruses, adeno-associated viruses, retroviruses, or a combination thereof.

In yet another aspect, the present disclosure provides a cell comprising the polynucleotide of the disclosure.

In one embodiment, the cell comprises the vector of the present disclosure, or genome of the cell is integrated with the polynucleotide of the present disclosure.

In one embodiment, the cell is selected from a group consisting of *Escherichia coli, Bacillus subtilis,* yeast cells, insect cells, mammalian cells, or a combination thereof; preferably is a mammalian cell, such as a CHO-S cell or 293E cell.

In yet another aspect, the present disclosure provides use of the bispecific antibody or the conjugate or fusion protein of the present disclosure in the treatment of tumors (cancers) or in the preparation of drugs for the treatment of tumors (cancers).

In one embodiment, the disease is cancer or tumor. Preferably, the tumor is selected from a group consisting of hematological tumors, solid tumors, or a combination thereof.

In another aspect, the present disclosure provides use of the bispecific antibody or the conjugate or fusion protein of the present disclosure in the preparation of reagents or kits for detecting tumors (cancers).

In yet another aspect, the present disclosure provides a method for preparing the bispecific antibody of the present disclosure, including the steps:
(i) culturing the cells of the present disclosure under suitable conditions to obtain a mixture containing the bispecific antibody of the present disclosure; and
(ii) purifying and/or separating the mixture obtained in step (i) to obtain the bispecific antibody of the present disclosure.

In one embodiment, the purification comprises: affinity chromatography, ion exchange chromatography, hydrophobic chromatography, molecular sieve chromatography, or a combination thereof.

In another aspect, the present disclosure provides a method for treating diseases, comprising the steps of: administering a therapeutically effective amount of the bispecific antibody of the present disclosure, or the conjugate or fusion protein of the present disclosure, or the pharmaceutical composition of the present disclosure, or a combination thereof to a subject in need.

In one embodiment, the subject is a human or non-human mammal.

### BRIEF DESCRIPTION OF THE DRAWINGS

**Figure 1** is a schematic diagram of the antibody structure of bispecific antibody structure 1 (A) and a schematic diagram of the protein primary structure of each component of the antibody (B), wherein the variable region 1 of antibody b is represented as VL/Hb, and the variable region 2 of antibody b is represented as VH/Lb.
**Figure 2** is a schematic diagram of the antibody structure of bispecific antibody structure 2 (A) and a schematic diagram of the protein primary structure of each component of the antibody (B).
**Figure 3** is a schematic diagram of the antibody structure of bispecific antibody structure 3 (A) and a schematic diagram of the protein primary structure of each component of the antibody (B).
**Figure 4** is a schematic diagram of the antibody structure of bispecific antibody structure 4 (A) and a schematic diagram of the protein primary structure of each component of the antibody (B).

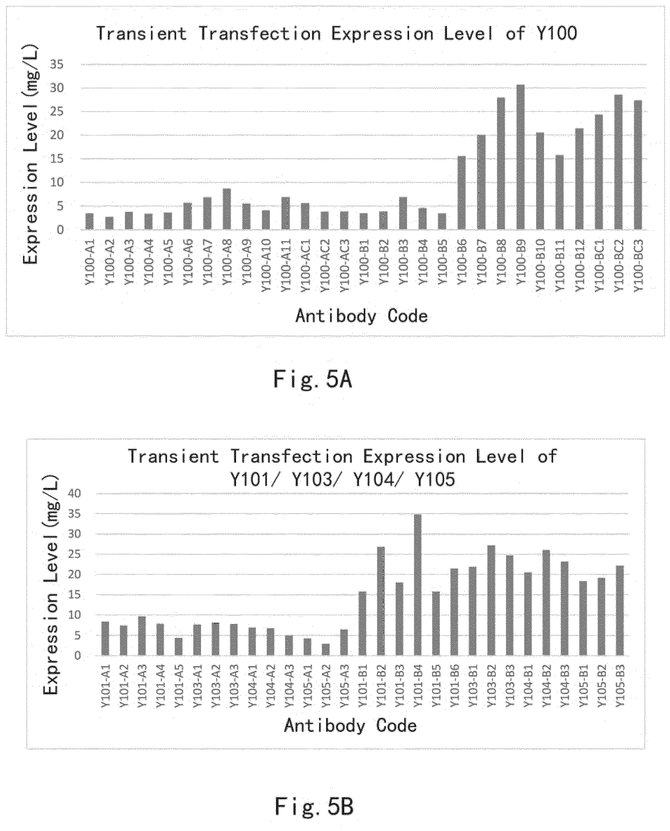

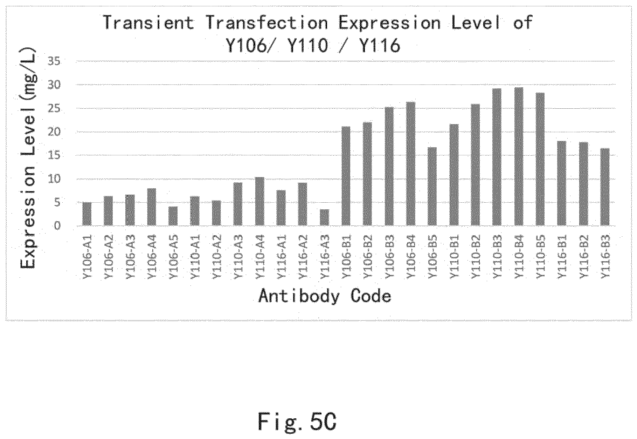

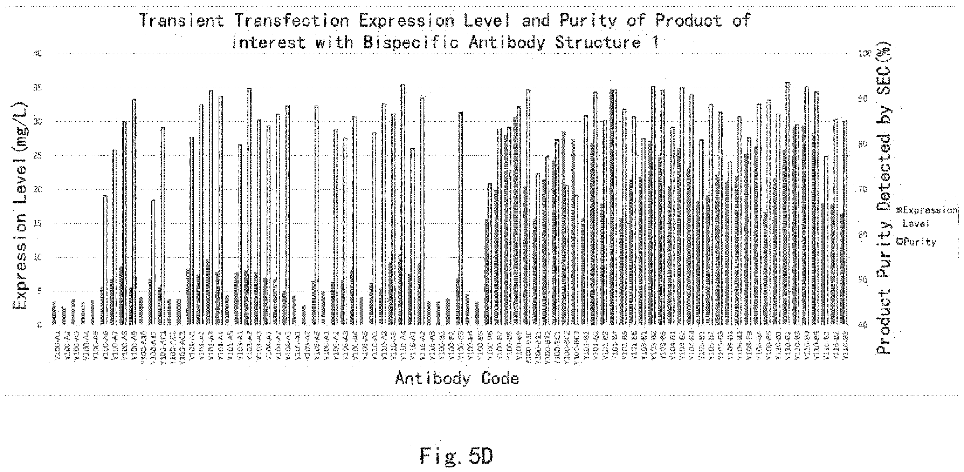

**Figure 5** shows antibodies with bispecific antibody structure 1. (A) shows the transient transfection expression level of Y100-A series and Y100-B series antibodies in CHO cells. (B) shows the transient transfection expression levels of Y101/Y103/Y104/Y105-A series and Y101/Y103/Y104/Y105-B series antibodies in CHO cells. (C) shows the transient transfection expression level of Y106/Y110/Y116-A series and Y106/Y110/Y116-B series antibodies in CHO cells. Figure 5D shows the purity detected by HPLC-SEC after proteinA affinity chromatography for Y100/Y101/Y103/Y104/Y105/Y106/Y110/Y116-B series antibodies that are expressed at a level of greater than 5 mg/L.
**Figure 6** shows the transient transfection expression level of Y200/Y201/Y203/Y204/Y205/Y206/Y210/Y216-A and B series antibodies of bispecific antibody structure 2 in CHO cells and the purity detected by HPLC-SEC after proteinA affinity chromatography.
**Figure 7** shows the transient transfection expression levels of Y300/Y304/Y316-A series, B series, C series and D series antibodies of bispecific antibody structure 3 in CHO cells, and the purity detected by HPLC-SEC after proteinA affinity chromatography.
**Figure 8** shows the transient transfection expression levels of Y400/Y404/Y416-A series and B series antibodies of bispecific antibody structure 4 in CHO cells, and the purity detected by HPLC-SEC after proteinA affinity chromatography.

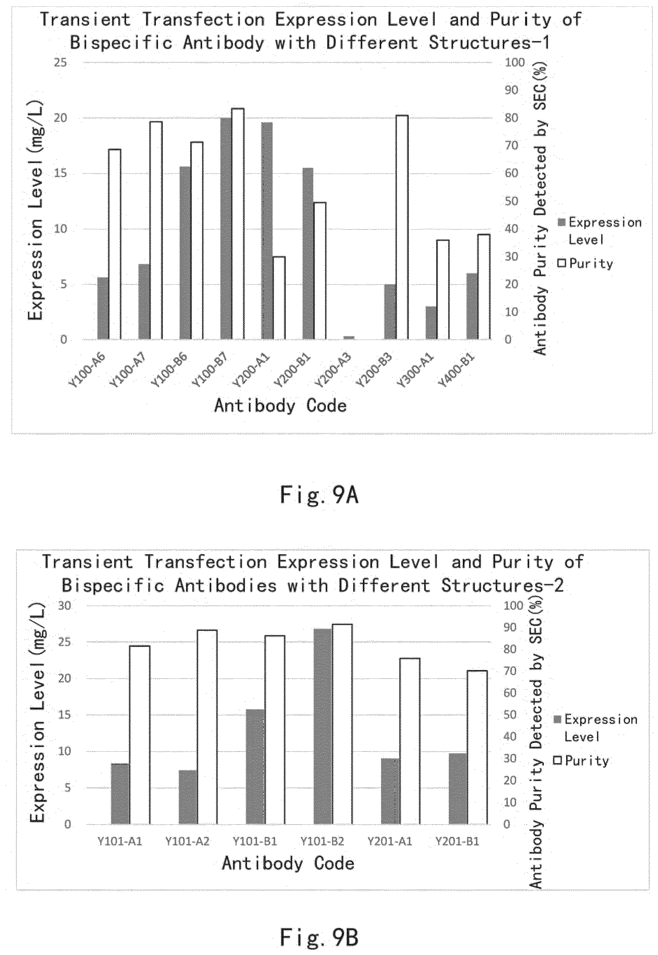

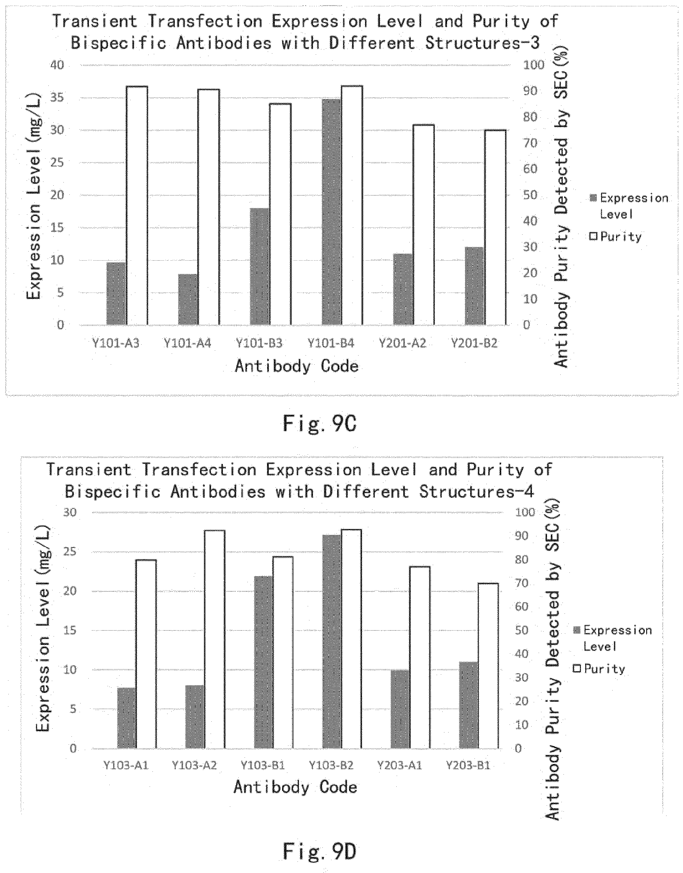

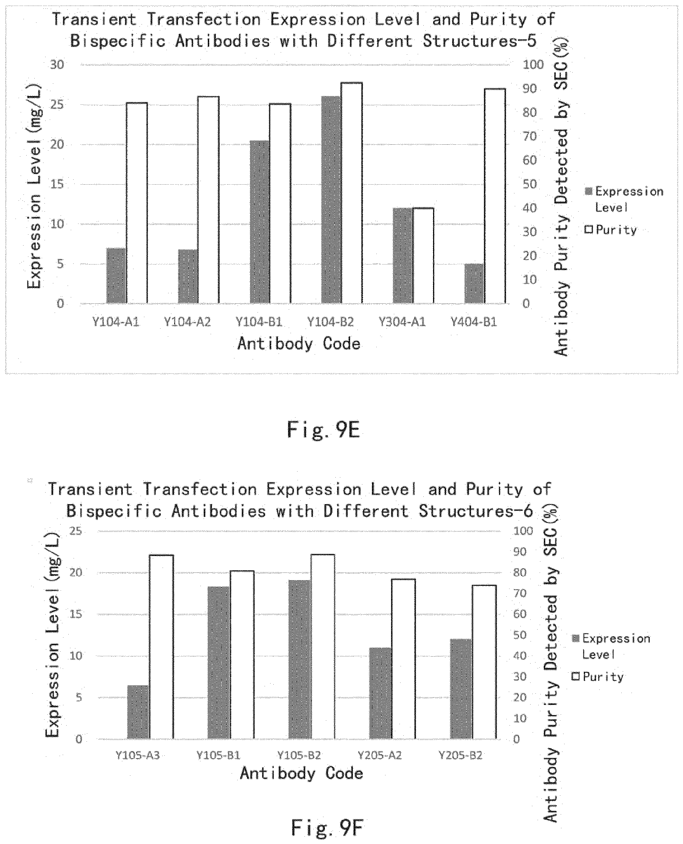

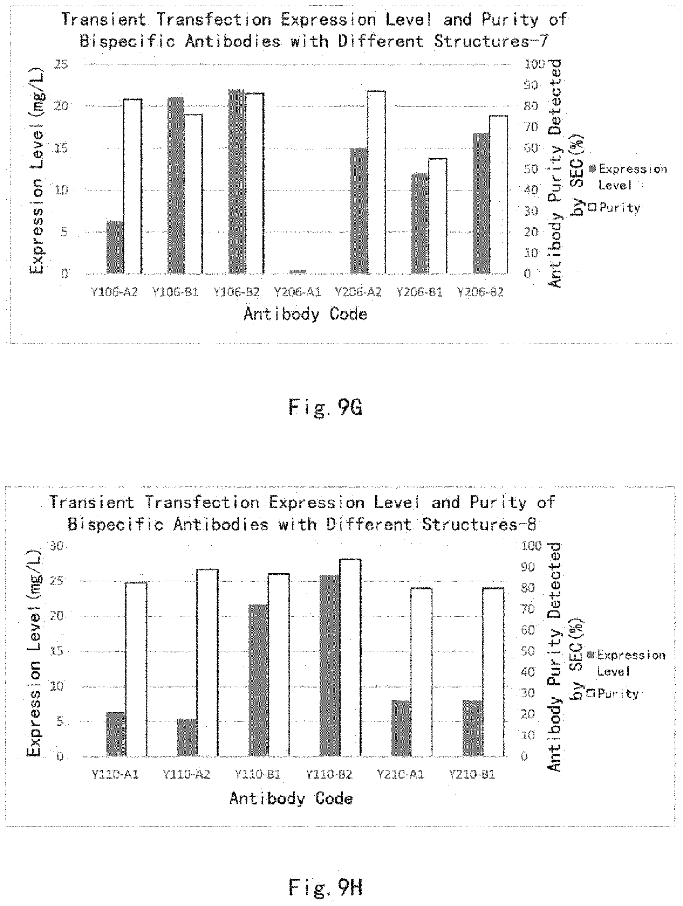

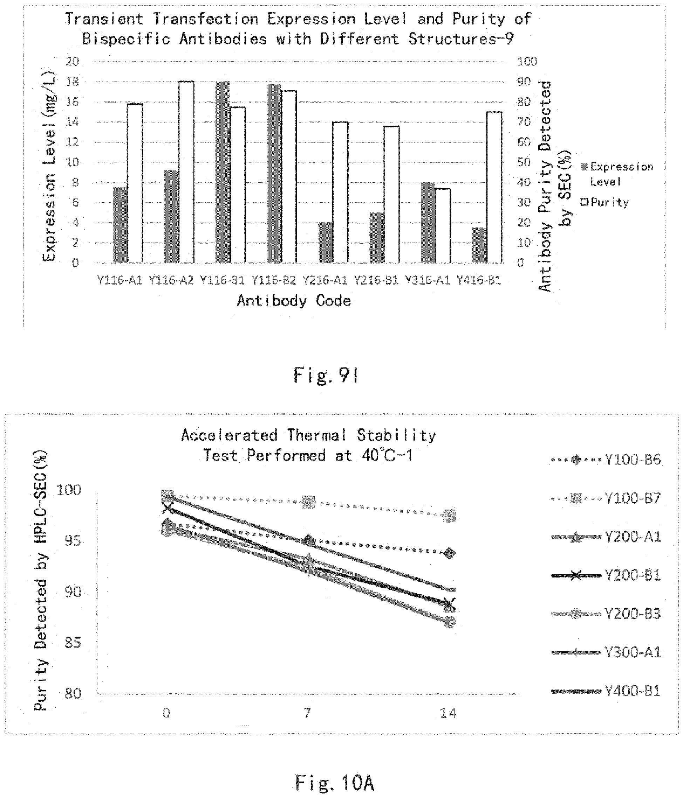

**Figure 9** is a comparison of the expression level and purity of antibodies of the bispecific antibody structures 1 to 4 with the same target and antibody variable region sequence. (A) The expression level and purity of antibodies of bispecific antibody structure 1 to 4 with the S70 antibody variable region sequence (SEQ ID NOs: 41 and 42) and G631 antibody variable region sequence (SEQ ID NOs: 57 and 58). (B) The expression level and purity of antibodies of bispecific antibody structures 1 and 2 with the S70 antibody variable region sequence (SEQ ID NOs: 41 and 42) and 3G12 antibody variable region sequence (SEQ ID NOs: 59 and 60). (C) The expression level and purity of antibodies of bispecific antibody structures 1 and 2 with 12A4 antibody variable region sequence (SEQ ID NOs: 45 and 46) and 3G12 antibody variable region sequence (SEQ ID NOs: 59 and 60). (D) The expression level and purity of antibodies of bispecific antibody structures 1 and 2 with the S70 antibody variable region sequence (SEQ ID NOs: 41 and 42) and LAG35 antibody variable region sequence (SEQ ID NOs: 33 and 34). (E) The expression level and purity of antibodies of bispecific antibody structures 1, 3 and 4 with 5C4 antibody variable region sequence (SEQ ID NOs: 37 and 38) and Yervoy antibody variable region sequence (SEQ ID NOs: 27 and 28). (F) The expression level and purity of antibodies of bispecific antibody structures 1 and 2 with 5C4 antibody variable region sequence (SEQ ID NOs: 37 and 38) and 10A7 antibody variable region sequence (SEQ ID NOs: 29 and 30). (G) The expression level and purity of antibodies of bispecific antibody structure 1 and 2 with 5C4 antibody variable region sequence (SEQ ID NOs: 37 and 38) and G631 antibody variable region sequence (SEQ ID NOs: 57 and 58). (H) The expression level and purity of antibodies of bispecific antibody structures 1 and 2 with 5C4 antibody variable region sequence (SEQ ID NOs: 37 and 38) and B-N10 antibody variable region sequence (SEQ ID NOs: 63 and 64). (I) The expression level and purity of antibodies of bispecific antibody structures 1 to 4 with Elotuzumab antibody variable region sequence (SEQ ID NOs: 49 and 50) and NM3E2 antibody variable region sequence (SEQ ID NOs: 47 and 48).

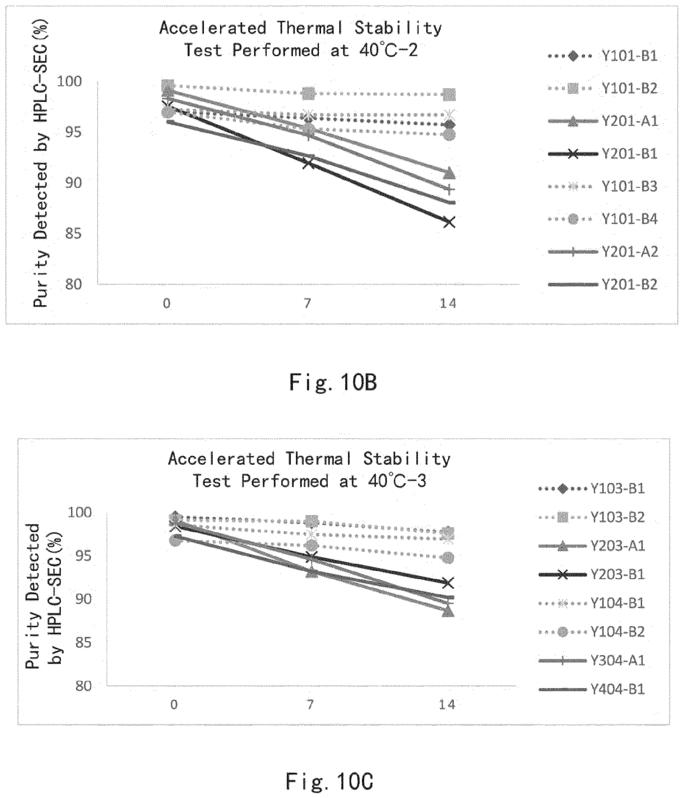

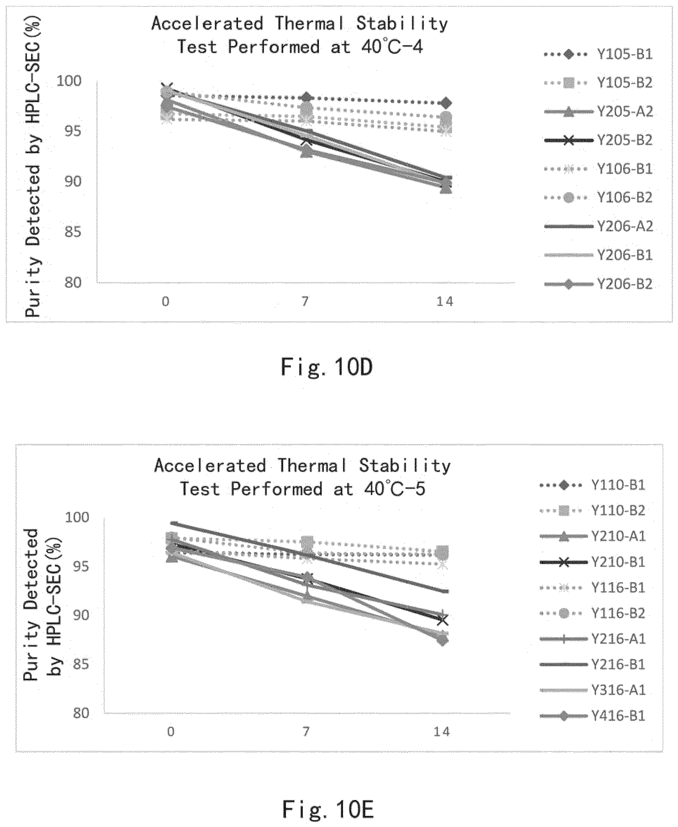

**Figure 10** shows the accelerated thermal stability test of different bispecific antibody structures at 40°C. (A) Purity of Y100-B6, Y100-B7, Y200-A1, Y200-B1, Y200-B3, Y300-A1 and Y400-B1 detected by HPLC-SEC after treatment at 40°C for 0, 7 and 14 days; (B) purity of Y101-B1, Y101-B2, Y201-A1, Y201-B1, Y101-B3, Y101-B4, Y201-A2 and Y201-B2 detected by HPLC-SEC after treatment at 40°C for 0, 7 and 14 days; (C) purity of Y103-B1, Y103-B2, Y203-A1, Y203-B1, Y104-B1, Y104-B2, Y304-A1 and Y404-B1 detected by HPLC-SEC after treatment at 40°C for 0, 7 and 14 days; (D) purity of Y105-B1, Y105-B2, Y205-A2, Y205-B2, Y106-B1, Y106-B2, Y206-A2, Y206-B1 and Y206-B2 detected by HPLC-SEC after treatment at 40°C for 0, 7 and 14 days; (E) purity of Y110-B1, Y110-B2, Y210-A1, Y210-B1, Y116-B1, Y116-B2, Y216-A1, Y216-B1, Y316-A1, Y416-B1 detected by HPLC-SEC after treatment at 40°C for 0, 7 and 14 days.

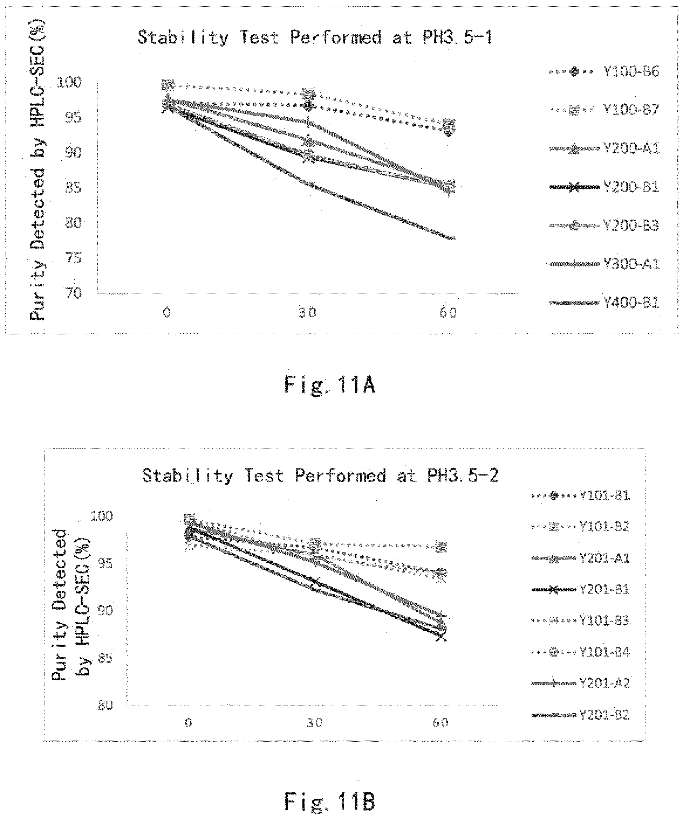

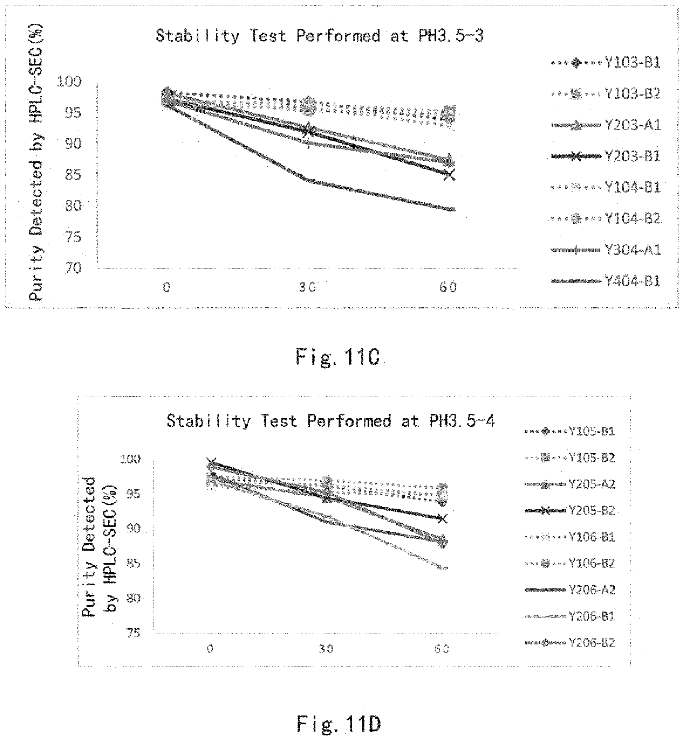

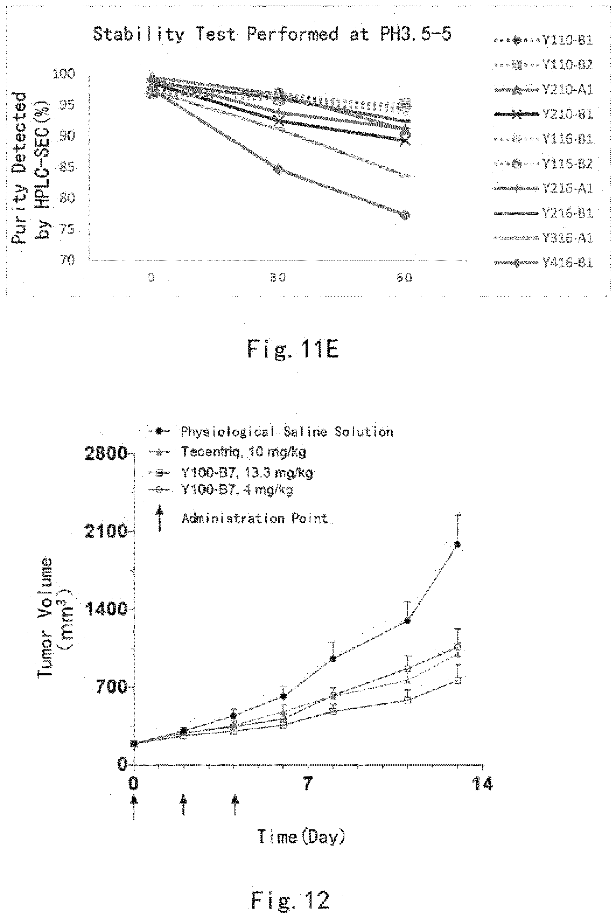

**Figure 11** shows the acid resistance test of different bispecific antibody structures. The pH value of the used acidic solution is 3.5. Figure 11A shows the purity of Y100-B6, Y100-B7, Y200-A1, Y200-B1, Y200-B3, Y300-A1 and Y400-B1 detected by HPLC-SEC after being treated under low pH condition for 0, 30 and 60 minutes; Figure 11B shows the purity of Y101-B1, Y101-B2, Y201-A1, Y201-B1, Y101-B3, Y101-B4, Y201-A2 and Y201-B2 detected by HPLC-SEC after treatment under low pH condition for 0, 30 and 60 minutes; Figure 11C shows purity of Y103-B1, Y103-B2, Y203-A1, Y203-B1, Y104-B1, Y104-B2, Y304-A1 and Y404-B1 detected by HPLC-SEC after treatment under low pH condition for 0, 30 and 60 minutes; Figure 11D shows purity of Y105-B1, Y105-B2, Y205-A2, Y205-B2, Y106-B1, Y106-B2, Y206-A2, Y206-B1 and Y206-B2 detected by HPLC-SEC after treatment under low pH condition for 0, 30 and 60 minutes; Figure 11E shows purity of Y110-B1, Y110-B2, Y210-A1, Y210-B1, Y116-B1, Y116-B2, Y216-A1, Y216-B1, Y316-A1, Y416-B1 detected by HPLC-SEC after treatment under low pH condition for 0, 30 and 60 minutes.
**Figure 12** shows the results of in vivo pharmacodynamic experiment of Y100-B7 with bispecific antibody structure 1. A mouse colon cancer MC38 is used as a tumor cell line, and a female C57BL/6 mouse is used as mouse strain.
**Figure 13** shows the results of in vivo pharmacodynamic experiment of Y101-B2 with bispecific antibody structure 1. A mouse colon cancer MC38 is used as tumor cell line, and a female C57BL/6 mouse is used as mouse strain.

### DEFINITIONS

It is to be noted that the term "a" or "an" entity refers to one or more of that entity; for example, "a bispecific antibody" shall be understood to represent one or more bispecific antibodies. As such, the terms "a" (or "an"), "one or more," and "at least one" can be used interchangeably herein.

As used herein, the term "polypeptide" is intended to encompass a singular "polypeptide" as well as plural "polypeptides", and refers to a molecule composed of monomers (amino acids) linearly linked by amide bonds (also known as peptide bonds). The term "polypeptide" refers to any chain or chains of two or more amino acids, and does not refer to a specific length of the product. Thus, peptides, dipeptides, tripeptides, oligopeptides, "protein," "amino acid chain," or any other term used to refer to a chain or chains of two or more amino acids, are all included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non- naturally occurring amino acids. A polypeptide may be derived from a natural biological source or may be produced by recombinant technology, but is not necessarily translated from a specified nucleic acid sequence. It may be generated in any manner, including by chemical synthesis.

As used herein, the term "recombinant" as it pertains to polypeptides or polynucleotides refers to a form of the polypeptide or polynucleotide that does not exist naturally, a non-limiting example of which can be achieved by combining polynucleotides or polypeptides that would not normally occur together.

"Homology" or "identity" or "similarity" refers to sequence similarity between two peptides or between two nucleic acid molecules. Homology can be determined by comparing positions in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, though preferably less than 25% identity, with one of the sequences of the present disclosure.

A polynucleotide or polynucleotide region (or a polypeptide or polypeptide region) having a certain percentage (for example, 60 %, 65 %, 70 %, 75 %, 80 %, 85 %, 90 %, 95 %, 98 % or 99 %) of "sequence identity" to another sequence means that, when aligned, such percentage of bases (or amino acids) are the same in comparing the two sequences. This alignment and the percent homology or sequence identity can be determined by using software programs known in the art, for example those described in Ausubel et al. eds. (2007) Current Protocols in Molecular Biology. Preferably, default parameters are used for alignment. One alignment program is BLAST, using default parameters. In particular, programs are BLASTN and BLASTP, which use the following default parameters: Genetic code = standard; filter = none; strand = both; cutoff = 60; expect = 10; Matrix = BLOSUM62; Descriptions = 50 sequences; sort by = HIGH SCORE; Databases = non-redundant, GenBank + EMBL + DDBJ + PDB + GenBank CDS translations + SwissProtein + SPupdate + PIR. Detailed information of these programs can be found at the following Internet address: http://www.ncbi.nlm.nih.gov/blast/Blast.cgi, last accessed on May 21, 2008. Biologically equivalent polynucleotides are those having the above specific percent of homology and encoding a polypeptide having the same or similar biological activity.

The term "encode" as it is applied to polynucleotides refers to a polynucleotide which "encodes" a polypeptide and which, in its native state or when manipulated by methods well known to those skilled in the art, can be transcribed and/or translated to produce the mRNA for the polypeptide and/or a fragment thereof. An antisense strand is a complement of such a nucleic acid, and an encoding sequence can be deduced therefrom.

As used herein, an "antibody" or "antigen-binding polypeptide" refers to a polypeptide or a polypeptide complex that specifically recognizes and binds to an antigen. An antibody can be an intact antibody and any antigen binding fragment or a single chain thereof. Thus the term "antibody" includes any protein or peptide containing a specific molecule, wherein the specific molecule comprises at least a portion of an immunoglobulin molecule having biological activity of binding to an antigen. Examples of such include, but are not limited to a complementary determining region (CDR) of a heavy or light chain or a ligand binding portion thereof, a heavy chain or light chain variable region, a heavy chain or light chain constant region, a framework (FR) region, or any portion thereof, or at least one portion of a binding protein.

The term "antibody fragment" or "antigen-binding fragment", as used herein, refers a portion of an antibody, such as F(ab')₂, F(ab)₂, Fab', Fab, Fv, Fd, Fv, dAb, Fab/c, complementarity determining region (CDR) fragments, disulfide bond-linked Fvs (sdFv), single chain antibodies (for example, scFv), bivalent antibodies or domain antibodies and the like. Regardless of structure, the antibody fragment binds with the same antigen that is recognized by an intact antibody. The term "antibody fragment" includes aptamers, spiegelmers, and diabodies. The term "antibody fragment" also includes any synthetic or genetically engineered protein that acts like an antibody by binding to a specific antigen to form a complex.

A "single-chain variable fragment" or "scFv" refers to a fusion protein of the variable regions of the heavy (VH) and light chains (VL) of immunoglobulins. In some aspects, the regions are connected with a short linker peptide of 10 to about 25 amino acids. The linker can be rich in glycine for flexibility, as well as serine or threonine for solubility, and can either connect the N-terminus of the VH with the C-terminus of the VL, or vice versa. This protein retains the specificity of the original immunoglobulin, but the constant region is removed and the linker is introduced. ScFv molecules are known in the art and are described, e.g., in US patent 5,892,019.

The term antibody encompasses a wide variety of polypeptides that can be biochemically recognized. Those skilled in the art will appreciate that heavy chains are classified as gamma, mu, alpha, delta, or epsilon (γ , µ ,α ,δ ,ε ) with some subclasses among them ( e.g., γ1- γ 4). It is the nature of this chain that determines the "class" of the antibody as IgG, IgM, IgA IgGor IgE, respectively. The immunoglobulin subclasses (isotypes) e.g., IgG1, IgG2, IgG3, IgG4, IgG5, etc. are well characterized and functionally specific. Modified versions of each of these classes and isotypes are readily discernable to those skilled in the art in view of the present disclosure and, accordingly, are within the scope of the present disclosure. All immunoglobulin classes are clearly within the scope of the present disclosure, the following discussion will generally be directed to the IgG class of immunoglobulin molecules. With regard to IgG, a standard immunoglobulin molecule comprises two identical light chain polypeptides with a molecular weight of approximately 23,000 Daltons, and two identical heavy chain polypeptides with a molecular weight of 53,000-70,000. The four chains are typically joined by disulfide bonds in a "Y" configuration, wherein the light chains bracket the heavy chains starting at the mouth of the "Y" and extending through the variable region.

Antibodies, antigen-binding polypeptides, variants or derivatives thereof in the present disclosure include, but are not limited to, polyclonal, monoclonal, multispecific, human, humanized, primatized, or chimeric antibodies, single chain antibodies, antigen-binding fragments, e.g., Fab, Fab' and F(ab')2, Fd, Fvs, single-chain Fvs (scFv), single-chain antibodies, disulfide-linked Fvs (sdFv), fragments comprising either a VL or VH domain, fragments produced by a Fab expression library, and anti- idiotypic (anti-Id) antibodies. Immunoglobulin or antibody molecules of the disclosure can be of any type (e.g., IgG, IgE, IgM, IgD, IgA, and IgY), any class (e.g., IgG1, IgG2, IgG3, IgG4, IgAl and IgA2) or subclass of immunoglobulin molecule.

Light chains are classified as either kappa or lambda (κ, λ). Each class of heavy chain may be bound to either a kappa or lambda light chain. In general, the light and heavy chains are covalently bound to each other, and the "tail" portions of the two heavy chains are bound to each other by covalent disulfide bonds or non-covalent bonds when the immunoglobulins are generated either by hybridomas, B cells or genetically engineered host cells. In the heavy chain, the amino acid sequences extend from an N-terminus at the forked ends of the Y configuration to the C-terminus at the bottom of each chain.

Both the light and heavy chains are divided into structural regions and functional homology regions. The terms "constant" and "variable" are used functionally. In this regard, it will be appreciated that the variable domains of both the light (VL) and heavy (VH) chain determine the antigen recognition and specificity. Conversely, the constant domains of the light chain (CL) and the heavy chain (CH1, CH2 or CH3) confer important biological properties such as secretion, transplacental mobility, Fc receptor binding, complement binding, and the like. Generally, the number of the constant region domains increases as they become more distal from the antigen-binding site or amino-terminus of the antibody. The N-terminal portion is a variable region and the C-terminal portion is a constant region; the CH3 and CL domains actually comprise the carboxy-terminus of the heavy and light chain, respectively.

As mentioned above, the variable region allows the antibody to selectively recognize and specifically bind to the epitope on the antigen. That is, the VL domain and VH domain, or the subclasses of complementary determining regions (CDR), of an antibody are combined to form a variable region that defines a three-dimensional antigen-binding site. This tetravalent antibody structure forms an antigen binding site that is present at the end of each arm in the Y configuration. More specifically, the antigen-binding site is defined by three CDRs on each of the VH and VL chains (i.e., CDR-H1, CDR-H2, CDR-H3, CDR-L1, CDR-L2, and CDR-L3). In some examples, for example, certain immunoglobulins derived from camelid species or engineered based on camelid immunoglobulins, an intact immunoglobulin molecule thereof may consist of only heavy chains without light chains. See, for example, Hamers-Casterman et al., Nature 363:446-448 (1993).

In naturally occurring antibodies, the six "complementary determining regions" or "CDRs" present in each antigen-binding domain are short, non-contiguous amino acid sequences that are specifically positioned to form the antigen-binding domain. The remaining amino acids in the antigen-binding domain, called "framework" region, show less intramolecular variability. The framework regions mainly adopt a β-sheet configuration, and the CDRs form loops which connect the β-sheet structure and sometimes form a part of the β-sheet structure. Therefore, the framework regions are used to form a scaffold that provides for positioning the CDRs in correct direction by inter-chain, non-covalent interactions. The antigen-binding domain formed by the positioned CDR defines a surface complementary to epitope on the immunoreactive antigen. This complementary surface promotes the non-covalent binding of the antibody to its homologous epitope. Those skilled in the art can easily identify the amino acids of the CDR and framework regions for any given heavy chain or light chain variable region since they have been clearly defined (see, "Sequences of Proteins of Immunological Interest," Kabat, E., et al., U.S. Department of Health and Human Services, (1983); Chothia and Lesk, J. Mol. Biol., 196:901-917 (1987), the full text of which is incorporated herein by reference).

In the case where there are two or more definitions of a term that are used and/or accepted within the art, the definitions of the term as used herein are intended to include all meanings, unless explicitly stated to the contrary. A specific example is the use of the term "complementary determining region" ("CDR") to describe the non-contiguous antigen binding sites present in the variable regions of both heavy chain and light chain polypeptides. This specific region has been described by Kabat et al.,U.S. Department of Health and Human Services, "Sequences of Proteins of Immunological Interest" (1983) and by Chothia et al.,J. Mol. Biol. 196:901-917 (1987), the full text of which is incorporated herein by reference. According to the definition of Kabat and Chothia, CDR includes overlapping amino acid residues or amino acid substructures when compared with each other. However, the application of each definition to refer to a CDR of an antibody or variant thereof will be within the scope of the term as defined and used herein. Appropriate amino acid residues which encompass the CDRs as defined by each of the references cited above are set forth in the table below for comparison. The exact number of residues which encompass a particular CDR will vary depending on the sequence and size of the CDR. Those skilled in the art can routinely determine which residues comprise a particular CDR if the amino acid sequence of the variable region of the antibody is provided.

**[Table 1] Definition of antibody variable region**

| | **Kabat** | **Chothia** |
|---|---|---|
| CDR-H1 | 31-35 | 26-32 |
| CDR-H2 | 50-65 | 52-58 |
| CDR-H3 | 95-102 | 95-102 |
| CDR-L1 | 24-34 | 26-32 |
| CDR-L2 | 50-56 | 50-52 |
| CDR-L3 | 89-97 | 91-96 |

Kabat *et al*. also defined a numbering system for variable domain sequences, which is applicable to any kind of antibody. Those skilled in the art can use this "Kabat numbering" system to any variable domain sequence unambiguously, without depending on any experimental data other than the sequence itself. The "Kabat numbering" as used herein refers to the numbering system described by Kabat et al., U.S. Department of Health and Human Services, "Sequence of Proteins of Immunological Interest" (1983).

In addition to the above table, the CDR regions described by the Kabat numbering system are as follows: CDR-H1 starts at the amino acid approximately at position 31 (i.e., approximately 9 residues after the first cysteine residue) and includes approximately 5 to 7 amino acids, and ends at the next tryptophan residue. CDR-H2 starts at the fifteenth residue after the end of CDR-H1, includes approximately 16 to 19 amino acids, and ends at the next arginine or lysine residue. CDR-H3 starts at approximately the thirty-third amino acid residue after the end of CDR-H2; includes 3 to 25 amino acids; and ends at the sequence W-G-X-G, wherein X is any amino acid. CDR-L1 starts at the residue approximately at position 24 (i.e., following a cysteine residue); includes approximately 10 to 17 residues; and ends at the next tryptophan residue. CDR-L2 starts at approximately the sixteenth residue after the end of CDR-L1 and includes approximately 7 residues. CDR-L3 starts at approximately the thirty-third residue after the end of CDR-L2 (i.e., following a cysteine residue); includes approximately 7 to 11 residues and ends at the sequence F or WGXG, wherein X is any amino acid.

The antibodies described herein can be from any animal origin, including birds and mammals. Preferably, the antibodies are human, murine, donkey, rabbit, goat, guinea pig, camel, llama, horse or chicken antibodies. In another embodiment, the variable region may be derived from a condricthoid (e.g., from a shark).

The term "heavy chain constant region" as used herein includes amino acid sequences derived from immunoglobulin heavy chains. A polypeptide comprising a heavy chain constant region comprises at least one of the following: a CH1 domain, a hinge (for example, upper hinge region, middle hinge region, and/or lower hinge region) domain, a CH2 domain, a CH3 domain, or a variant or fragment thereof. For example, the antigen-binding polypeptide for use in the present disclosure may comprise a polypeptide chain comprising a CH1 domain; a polypeptide comprising a CH1 domain, at least a portion of a hinge domain and a CH2 domain; a polypeptide chain comprising a CH1 domain and a CH3 domain; a polypeptide chain comprising a CH1 domain, at least a portion of a hinge domain and a CH3 domain, or a polypeptide chain comprising a CH1 domain, at least a portion of a hinge domain, a CH2 domain, and a CH3 domain. In another embodiment, the polypeptide of the present disclosure comprises a polypeptide chain comprising a CH3 domain. In addition, the antibodies used in the present disclosure may lack at least a portion of a CH2 domain (for example, all or a portion of the CH2 domain). As set forth above, it will be understood by those skilled in the art that the heavy chain constant regions may be modified so that they differ in amino acid sequence from naturally occurring immunoglobulin molecules.

The heavy chain constant regions of the antibody disclosed herein can be derived from different immunoglobulin molecules. For example, a heavy chain constant region of a polypeptide may include a CH1 domain derived from an IgG1 molecule and a hinge region derived from an IgG3 molecule. In another example, a heavy chain constant region may include a hinge region that is partly derived from an IgG1 molecule and partly from an IgG3 molecule. In another example, a heavy chain portion may comprise a chimeric hinge that is partly derived from an IgG1 molecule and partly derived from an IgG4 molecule.

The term "light chain constant region" as used herein includes an amino acid sequence derived from the light chain of an antibody. Preferably, the light chain constant region includes at least one of a constant kappa domain and a constant lambda domain.

A "light chain-heavy chain pair" refers to a collection of light chain and heavy chain that can form a dimer through a disulfide bond between the CL domain of the light chain and the CH1 domain of the heavy chain.

As previously indicated, the subunit structures and three-dimensional structures of the constant regions of various immunoglobulins are known. As used herein, the term "VH domain" includes the amino terminus variable domain of a heavy chain of an immunoglobulin, and the term "CHI domain" includes a first (mostly amino terminus) constant region of a heavy chain of an immunoglobulin. The CH1 domain is adjacent to the VH domain and is the amino terminus of the hinge region of heavy chain molecule of an immunoglobulin.

The term "CH2 domain" as used herein includes a portion of a heavy chain molecule that ranges, for example, from a residue at about position 244 to a residue at position 360 of an antibody according to a conventional numbering system (residues at position 244 to 360, according to Kabat numbering system; and residues at position 231-340, according to EU numbering system; see Kabat et al., U.S. Department of Health and Human Services, "Sequences of Proteins of Immunological Interest" (1983). The CH2 domain is unique because it does not pair with another domain tightly. On the contrary, two N-linked branched carbohydrate chains are inserted between the two CH2 domains of an intact natural IgG molecule. It is documented that the CH3 domain extends from the CH2 domain to the C-terminus of the IgG molecule, and comprises about 108 residues.

The term "hinge region" as used herein includes the portion of a heavy chain molecule that links the CH1 domain to the CH2 domain. The hinge region comprises about 25 residues and is flexible, thereby allowing the two N-terminus antigen-binding regions to move independently. The hinge regions can be divided into three different domains: upper hinge domain, middle hinge domain, and lower hinge domain (Roux et al., J. Immunol 161:4083 (1998)).

The term "disulfide bond" as used herein includes a covalent bond formed between two sulfur atoms. The amino acid cysteine comprises a thiol group, which can form a disulfide bond or bridge with a second thiol group. In most naturally occurring IgG molecules, the CH1 and CL regions are linked by a disulfide bond and the two heavy chains are linked by two disulfide bonds, at positions corresponding to positions 239 and 242 under the Kabat numbering system (position 226 or 229, according to EU numbering system).

The term "chimeric antibody" as used herein is intended to refer to any antibody in which the immunoreactive regions or sites are obtained or derived from a first species and the constant regions (which may be intact, partial or modified according to the present disclosure) are obtained from a second species. In certain embodiments the target binding regions or sites will be derived from a non-human origin (e.g., mouse or primate) and the constant region will be derived from a human.

As used herein, "percent humanization" is calculated by determining the number of framework amino acid differences (ie, non-CDR differences) between the humanized domain and the germline domain, and subtracting that number from the total number of amino acids, then being divided by the total number of amino acids and multiplied by 100.

The so-called "specific binding" or "specific to" usually means that an antibody binds to an antigen epitope via its antigen-binding domain, and that this binding entails some complementarity between the antigen-binding domain and the antigen epitope. According to this definition, an antibody is considered to "specifically bind" to an antigen epitope, when it binds to the antigen epitope and the binding via the antigen-binding domain is easier than that via binding to a random, unrelated antigen epitope. The term "specificity" is used herein to determine the affinity of a certain antibody to bind to a certain antigen epitope. For example, antibody "A" may be considered to have a higher specificity for a given antigen epitope than that of antibody "B", or antibody "A" may be said to have higher specificity when bind to epitope "C" than when bind to the related epitope "D".

The term "treating" ("treat" or "treatment") as used herein refers to both therapeutic treatment and prophylactic or preventive measures, wherein the object is to prevent or slow down (lessen) an undesired physiological change or disorder, such as cancer progression. Beneficial or desired clinical outcomes include, but are not limited to, alleviating symptoms, diminishing the degree of disease, stabilizing (for example, preventing it from worsening) disease state, delaying or slowing the disease progression, alleviating or palliating the disease state, and alleviating (whether partial or total), regardless of whether detectable or undetectable. "Treatment" can also mean prolonging survival as compared to expected survival without treatment. Those in need of treatment include those that already have a disease or symptom and those that are prone to have a disease or symptom or those in which a disease or symptom is to be prevented.

The embodiments of the present disclosure provide a variety of bispecific antibodies, which comprise two different or identical antigen-binding polypeptide units. The antibody domain that binds to the antigen is Fab, or ScFv, or non-covalent pairing (Fv) between a variable region of the heavy chain (VH) and a variable region of the light chain (VL). In particular, all of these bispecific antibodies have a Fc fragment of antibody heavy chain constant region, and the Fc comprises: (1) a hinge, (2) a second constant region of heavy chain (CH2), and a third constant region of heavy chain (CH3).

Any of the aforementioned antibodies or polypeptides may further include additional polypeptides, for example, an encoded polypeptide as described herein, a signal peptide at the N-terminus of the antibody used to direct secretion, or other heterologous polypeptides as described herein.

It will also be understood by those skilled in the art that the antibodies as described herein can be modified such that they vary in amino acid sequence from the naturally occurring binding polypeptides from which they are derived. For example, a polypeptide or amino acid sequence derived from a specified protein may be similar to the original sequence, for example, having a certain percentage of identity to the original sequence, for example, it may have an identity of 60%, 70%, 75%, 80%, 85%, 90%, 95%, 98%, or 99% to the original sequence.

In addition, nucleotide or amino acid substitutions, deletions, or insertions leading to conservative substitutions or changes in the "non-essential" amino acid regions may be performed. For example, a polypeptide or amino acid sequence derived from a specified protein may be identical to the original sequence, except for one or more independent amino acid substitutions, insertions, or deletions, for example, 1, 2, 3, 4, 5, 6, 7, 8, 9, 10, 15, 20 or more independent amino acid substitutions, insertions, or deletions. In some embodiments, a polypeptide or amino acid sequence derived from a specified protein has 1 to 5, 1 to 10, 1 to 15 or 1 to 20 independent amino acid substitutions, insertions, or deletions as compared to the original sequence.

In other embodiments, the antigen binding polypeptides of the present disclosure may comprise conservative amino acid substitutions.

A "conservative amino acid substitution" is one in which an amino acid residue is substituted by an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (for example, glycine, asparagine, glutamine, serine, threonine, tyrosine, cysteine), non-polar side chains (for example, alanine, valine, leucine, isoleucine, proline, phenylalanine, methionine, tryptophan), β-branched side chains (for example, threonine, valine, isoleucine) and aromatic side chains (e.g. tyrosine, phenylalanine, tryptophan, histidine). Therefore, non-essential amino acid residues of immunoglobulin polypeptides are preferably substituted by other amino acid residues from the same side chain family. In another embodiment, a string of amino acids may be substituted by a structurally similar string of amino acids that differs in order and/or composition of the side chain family.

Non-limiting examples of conservative amino acid substitutions are provided in the table below, wherein a similarity score of 0 or higher indicates a conservative substitution between the two amino acids.

**[Table 2] Non-limiting list of conservative amino acid substitutions**

| | **C** | **G** | **P** | **S** | **A** | **T** | **D** | **E** | **N** | **Q** | **H** | **K** | **R** | **V** | **M** | **I** | **L** | **F** | **Y** | **w** |
|---|---|---|---|---|---|---|---|---|---|---|---|---|---|---|---|---|---|---|---|---|
| **w** | -8 | -7 | -6 | -2 | -6 | -5 | -7 | -7 | -4 | -5 | -3 | -3 | 2 | -6 | -4 | -5 | -2 | 0 | 0 | 17 |
| **Y** | 0 | -5 | -5 | -3 | -3 | -3 | -4 | -4 | -2 | -4 | 0 | -4 | -5 | -2 | -2 | -1 | -1 | 7 | 10 | |
| **F** | -4 | -5 | -5 | -3 | -4 | -3 | -6 | -5 | -4 | -5 | -2 | -5 | -4 | -1 | 0 | 1 | 2 | 9 | | |
| **L** | -6 | -4 | -3 | -3 | -2 | -2 | -4 | -3 | -3 | -2 | -2 | -3 | -3 | 2 | 4 | 2 | 6 | | | |
| **I** | -2 | -3 | -2 | -1 | -1 | 0 | -2 | -2 | -2 | -2 | -2 | -2 | -2 | 4 | 2 | 5 | | | | |
| **M** | -5 | -3 | -2 | -2 | -1 | -1 | -3 | -2 | 0 | -1 | -2 | 0 | 0 | 2 | 6 | | | | | |
| **V** | -2 | -1 | -1 | -1 | 0 | 0 | -2 | -2 | -2 | -2 | -2 | -2 | -2 | 4 | | | | | | |
| **R** | -4 | -3 | 0 | 0 | -2 | -1 | -1 | -1 | 0 | 1 | 2 | 3 | 6 | | | | | | | |
| **K** | -5 | -2 | -1 | 0 | -1 | 0 | 0 | 0 | 1 | 1 | 0 | 5 | | | | | | | | |
| **H** | -3 | -2 | 0 | -1 | -1 | -1 | 1 | 1 | 2 | 3 | 6 | | | | | | | | | |
| **Q** | -5 | -1 | 0 | -1 | 0 | -1 | 2 | 2 | 1 | 4 | | | | | | | | | | |
| **N** | -4 | 0 | -1 | 1 | 0 | 0 | 2 | 1 | 2 | | | | | | | | | | | |
| **E** | -5 | 0 | -1 | 0 | 0 | 0 | 3 | 4 | | | | | | | | | | | | |
| **D** | -5 | 1 | -1 | 0 | 0 | 0 | 4 | | | | | | | | | | | | | |
| **T** | -2 | 0 | 0 | 1 | 1 | 3 | | | | | | | | | | | | | | |
| **A** | -2 | 1 | 1 | 1 | 2 | | | | | | | | | | | | | | | |
| **S** | 0 | 1 | 1 | 1 | | | | | | | | | | | | | | | | |
| **P** | -3 | -1 | 6 | | | | | | | | | | | | | | | | | |
| **G** | -3 | 5 | | | | | | | | | | | | | | | | | | |
| **C** | 12 | | | | | | | | | | | | | | | | | | | |

In some embodiments, the antibody may be conjugated to therapeutic agents, prodrugs, peptides, proteins, enzymes, viruses, lipids, biological response modifiers, pharmaceutical agents, or PEG.

The antibody may be linked or fused to a therapeutic agent, which may include detectable labels, such as radioactive labels, immunomodulators, hormones, enzymes, oligonucleotides, photoactive therapeutic or diagnostic agents, cytotoxicity agents, which can be drugs or toxins, ultrasound enhancers, non-radioactive labels, a combination thereof and other such components known in the art.

The antibody can be detectably labeled by coupling it to chemiluminescent compounds. Then, the presence of the chemiluminescent-labeled antigen-binding polypeptide is determined by detecting the luminescence produced during the chemical reaction. Examples of particularly useful chemiluminescent labeling compounds are luminol, isoluminol, theromatic acridinium ester, imidazole, acridinium salt and oxalate ester.

The antibodies can also be detectably labeled by using fluorescence emitting metals such as 152Eu, or other lanthanide labels. These metals can be attached to the antibody by using the following metal chelating groups, such as diethylenetriaminepentaacetic acid (DTPA) or ethylenediaminetetraacetic acid (EDTA). Techniques for linking various moieties to antibodies are well known, see, for example, Arnon et al., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. (1985); Hellstrom et al., "Antibodies For Drug Delivery", in Controlled Drug Delivery (Second Edition), Robinson et al. (edited), Marcel Dekker, Inc., pp. 623-53 (1987); Thorpe, "Antibody Carriers Of Cytotoxic Agents In Cancer Therapy: A Review", in Monoclonal Antibodies '84: Biological And Clinical Applications, Pinchera et al. (edited), pp. 475-506 (1985); "Analysis, Results, And Future Prospective Of The Therapeutic Use Of Radiolabeled Antibody In Cancer Therapy", in Monoclonal Antibodies For Cancer Detection And Therapy, Baldwin et al. (edited), Academic Press pp. 303-16 (1985), and Thorpe et al., "The Preparation And Cytotoxic Properties Of Antibody-Toxin Conjugates", Immunol. Rev. (52:119-58 (1982)).

### Method for Preparing Antibodies

Methods for preparing antibodies are well known in the art and are described herein. In some embodiments, both the variable and constant regions of the antigen binding polypeptides of the present disclosure are fully human. Fully human antibodies can be prepared by using techniques described in the prior art, and as described herein. For example, fully human antibodies against a specific antigen can be prepared by administering the antigen to a transgenic animal which has been modified to produce such an antibody in response to antigen stimulation, but whose endogenous loci have been disabled. Exemplary techniques that can be used to produce such antibodies are described in US Patent Nos: 6,150,584; 6,458,592; 6,420,140, the entire contents of which are incorporated herein by reference.

The binding specificity of the antigen-binding polypeptide of the present disclosure can be measured by in vitro experiments, such as immunoprecipitation, radioimmunoassay (RIA) or enzyme-linked immunosorbent assay (ELISA).

Alternatively, the technique described for the preparation of single-chain units (U.S. Patent No. 4,694,778; Bird, Science 242:423-442 (1988); Huston et al. Proc. Natl. Acad. Sci. USA 55:5879-5883 (1988); and Ward et al., Nature 334:544-554 (1989)) can be used to produce single-chain units of the present disclosure. Single-chain units are formed by linking the heavy and light chain fragments of the Fv region via an amino acid bridge, resulting in a single-chain fusion peptide. Techniques of synthesizing functional Fv fragments in *E. coli* can also be used (Skerra et al., Science 242: 1038-1041 (1988)).

Examples of techniques that can be used to produce single-chain Fvs (scFvs) and antibodies include those described in U.S. Patent Nos. 4,946,778 and 5,258,498; Huston et al., Methods in Enzymology 203:46-88 (1991); Shu et al., Proc. Natl. Sci . USA 90:1995-1999 (1993); and Skerra et al., Science 240:1038-1040 (1988). For certain applications, including *in vivo* use of antibodies in humans and *in vitro* detection assays, it may be preferable to use chimeric, humanized or human antibodies. Chimeric antibodies are molecules in which different portions of the antibody are derived from different animal species, such as antibodies containing a variable region from a murine monoclonal antibody and a human immunoglobulin constant region. Methods for producing chimeric antibodies are known in the art. See, for example, Morrison, Science 229:1202 (1985); Oi et al., BioTechniques 4:214 (1986); Gillies et al., J. Immunol. Methods 125:191-202 (1989); U.S. Patent Nos. 5,807,715; 4,816,567 and 4,816,397, the entire content of which is incorporated herein by reference.

A humanized antibody is an antibody molecule that is derived from a non-human species and binds the desired antigen, and the antibody molecule has one or more complementary determining regions (CDR) from the non-human species and a framework region from a human immunoglobulin molecule. Generally, framework residues in the human framework region will be changed by substitution with corresponding residues from the CDR donor antibody, preferably to increase the antigen-binding ability. These framework substitutions are identified by methods known in the art, for example, by modeling of the interactions between CDR and framework residues to identify framework residues that are important for antigen binding and sequence comparison, so as to identify abnormal framework residues at specific positions. (See, for example, U.S. Patent No. 5,585,089 by Queen et al.; Nature 332:323 (1988) by Riechmann *et al*., the entire contents of which are incorporated herein by reference). Antibodies can be humanized by using a variety of techniques known in the art including, for example, CDR-grafting (EP 239,400; PCT Publication No. WO 91/09967; U.S. Patent Nos. 5,225,539; 5,530,101 and 5,585,089), veneering or resurfacing (EP 592,106; EP 519,596; Padlan, Molecular Immunology 28(4/5):489-498 (1991); Studnicka et al., Protein Engineering 7(6):805-814 (1994); Roguska et al., Proc. Natl. Sci. USA 91:969-973 (1994)), and chain shuffling (US Patent No. 5,565,332, the entire contents of which are incorporated herein by reference).

By using conventional recombinant DNA technology, one or more CDRs of the antigen-binding polypeptide of the present disclosure can be inserted into the framework region, for example, inserted into the human framework region to humanize non-human antibodies. The framework region may be a naturally occurring or shared framework region, and is preferably a human framework region (see, for example, Chothia et al., J. Mol. Biol. 278:457-479 (1998), a list of human framework regions). Preferably, the polynucleotide produced by the combination of the framework region and the CDR encodes a polypeptide which specifically binds to at least one antigen epitope of the desired polypeptide, for example, LIGHT. Preferably, one or more amino acid substitutions can be performed within the framework region, and, preferably, the amino acid substitutions improve the binding of an antibody to its antigen. In addition, this method can be used to make amino acid substitutions or deletions of one or more variable region cysteine residues (the cysteine residues are involved in the formation of intrachain disulfide bonds), thereby generating antibody molecules lacking one or more intrachain disulfide bonds. Other alterations made to the polynucleotides are included within the scope of the present disclosure and within the scope of the prior art.

In addition, techniques for producing "chimeric antibodies" by splicing genes from mouse antibody molecules can be used (Morrison et al., Proc. Natl. Acad. Sci. USA: 851-855 (1984); Neuberger et al., Nature 372:604-608 (1984); Takeda et al. Nature 314:452-454 (1985)), and the molecule with an appropriate antigen-specificity will be linked to the human antibody molecule gene with appropriate biological activity. As used herein, a chimeric antibody is a molecule in which different parts are derived from different animal species, such as antibodies containing a variable region from a murine monoclonal antibody and a human immunoglobulin constant region.

However, another efficient method for producing recombinant antibodies is disclosed in Newman, Biotechnology 10: 1455-1460 (1992). Specifically, this technique resulted in the generation of primatized antibodies containing monkey variable domains and human constant sequences. This document is incorporated herein by reference in its entirety. In addition, this technique is also described in commonly assigned U.S. Patent Nos. 5,658,570, 5,693,780, and 5,756,096, each of which is incorporated herein by reference.

Alternatively, antibody-producing cell lines can be selected and cultured by using techniques well known to those skilled in the art. Such techniques are described in various laboratory manuals and primary publications. In this regard, techniques suitable for use herein are described, for example, in Current Protocols in Immunology, edited by Coligan et al., Green Publishing Associates and Wiley-Interscience, John Wiley and Sons, New York (1991), which are incorporated by reference in their entirety, including supplementary references.

In addition, standard techniques known to those skilled in the art can be used to introduce mutations into the nucleotide sequences encoding the antibodies of the present disclosure, including, but not limited to, site-directed mutagenesis and PCR-mediated mutations which result in amino acid substitutions. Preferably, the variants (including derivatives), relative to the reference variable heavy chain region, CDR-H1, CDR-H2, CDR-H3, light chain variable region, CDR-L1, CDR-L2 or CDR- L3, encode less than 50 amino acid substitutions, less than 40 amino acid substitutions, less than 30 amino acid substitutions, less than 25 amino acid substitutions, less than 20 amino acid substitutions, less than 15 amino acid substitutions, and less than 10 amino acid substitutions, less than 5 amino acid substitutions, less than 4 amino acid substitutions, less than 3 amino acid substitutions, or less than 2 amino acid substitutions. Alternatively, mutations can be randomly introduced along all or part of the encoding sequence, for example, by saturation mutagenesis, and the resulting mutants can be screened for biological activity to identify mutations that retain activity.

### Antibody structure information

Monospecific antibodies are symmetrical antibodies, including two identical light chains and two identical heavy chains. The light chain and the heavy chain are linked by a disulfide bond and target a corresponding antigen; the heavy chain and the heavy chain are linked by a disulfide bond; the entire antibody has a "Y" configuration. The light chain includes a light chain variable region (VL) and a light chain constant region (Lc), and the heavy chain includes a heavy chain variable region (VH) and a heavy chain constant region, wherein the heavy chain constant region includes a CH1 and a Fc, and the Fc includes a hinge, a CH2 and a CH3.

**The bispecific antibody structure** 1 is F(ab)₂-(Fv)₂-Fc, which is a tetravalent symmetrical bispecific antibody, including two identical fusion heavy chains and two identical fusion light chains, wherein the two fusion heavy chains form a pair, the fusion light chain and the fusion heavy chain form a pair, and each pair forms one or more interchain disulfide bonds. The fusion heavy chain of the antibody comprises a heavy chain variable region of antibody a (VHa), a first constant region (CH1), a variable region 1 of antibody b and a Fc fragment. The variable region 1 of antibody b is located between CH1 and Fc, and linked by a linker. The fusion light chain of the antibody comprises a light chain variable region of antibody a (VLa), a light chain constant region (CL) and a variable region 2 of antibody b. The variable region 2 of antibody b is located at the C-terminus of CL and is linked by a linker; wherein, (1) the variable region 1 of antibody b is a heavy chain variable region (VHb), the variable region 2 of antibody b is a light chain variable region (VLb), and the antibody is called a A series of bispecific antibody structure 1, or (2) the variable region 1 of antibody b is a light chain variable region (VLb), the variable region 2 of antibody b is a heavy chain variable region (VHb), and the antibody is called a B series of bispecific antibody structure 1. Wherein, the VHa-VLa pair targets the antigen A, and the VHb-VLb pair targets the antigen B.

Figure 1A is a schematic diagram of the structure of bispecific antibody structure 1, and Figure 1B is a schematic diagram of the primary protein structure of each component of the antibody.

**The bispecific antibody structure 2** is IgG(H)-ScFv, which is a tetravalent symmetrical bispecific antibody, including two identical fusion heavy chains and two identical light chains, wherein the two fusion heavy chains form a pair, the light chain and the fusion heavy chain form a pair, and each pair forms one or more interchain disulfide bonds; the fusion heavy chain includes a heavy chain variable region (VHa), a first constant region of the heavy chain (CH1), a Fc and a ScFv, wherein the ScFv is located at the C-terminus of Fc and linked by a linker. The light chain-heavy chain pair targets the antigen A, and the ScFv targets the antigen B.

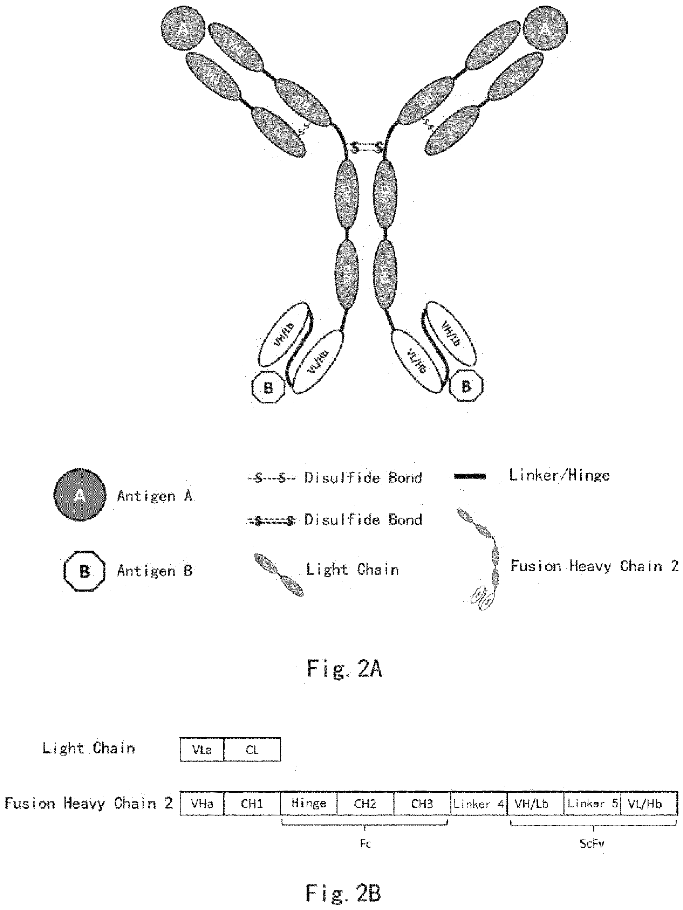

Figure 2A is a schematic diagram of the structure of bispecific antibody structure 2, and Figure 2B is a schematic diagram of the primary protein structure of each component of the antibody.

**The bispecific antibody structure 3** is Tandem-ScFv-Fc, which is a tetravalent symmetrical bispecific antibody, including two identical fusion peptides. The fusion peptide includes a ScFv (ScFv-a) targeting the antigen A, a (ScFv-b) targeting the antigen B, and a Fc, wherein the ScFv-b is located between the ScFv-a and the Fc and is linked by a linker or hinge.

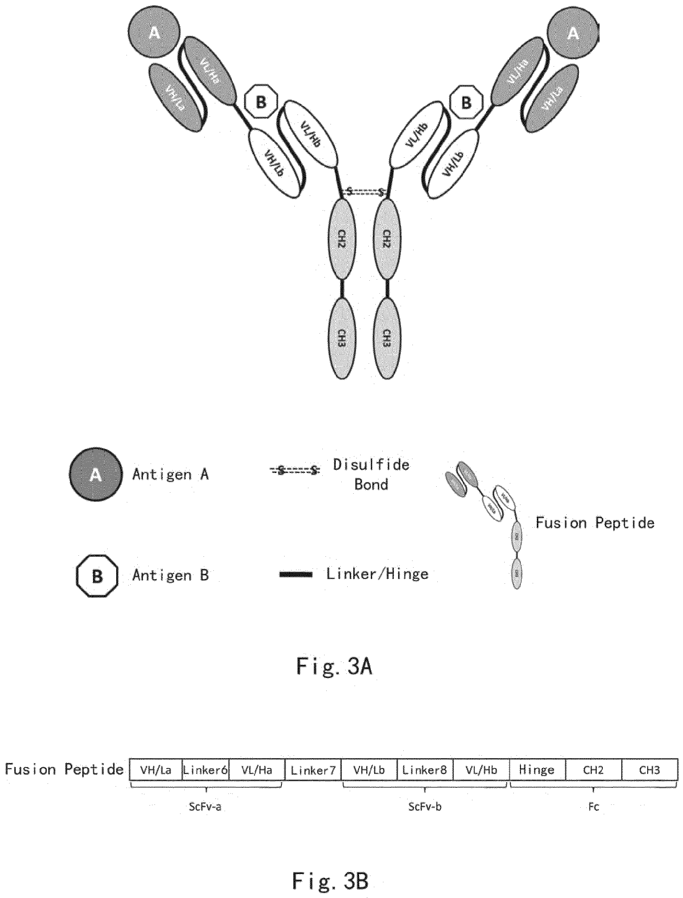

Figure 3A is a schematic diagram of the structure of bispecific antibody structure 3, and Figure 3B is a schematic diagram of the primary protein structure of each component of the antibody.

**The bispecific antibody structure 4** is DVD-IgG, which is a tetravalent symmetrical bispecific antibody, including two identical fusion light chains and two identical fusion heavy chains, wherein the two fusion heavy chains form a pair, and the two fusion light chains and the fusion heavy chains form a pair, and each pair forms one or more interchain disulfide bonds; the fusion light chain comprises a variable region 1 of antibody b and a light chain of antibody a which is linked by a linker, wherein the light chain of antibody a is located at C-terminus; the fusion heavy chain includes a variable region 2 of antibody b and a heavy chain of antibody a which is linked by a linker, wherein the heavy chain of antibody a is located at C-terminus. Wherein, (1) the variable region 1 of antibody b is a light chain variable region (VLb), the variable region 2 of antibody b is a heavy chain variable region (VHb), or (2) the variable region 1 of antibody b is a heavy chain variable region (VHb), the variable region 2 of antibody b is a light chain variable region (VLb). The VHa-VLa pair targets the antigen A, and the VHb-VLb pair targets the antigen B.

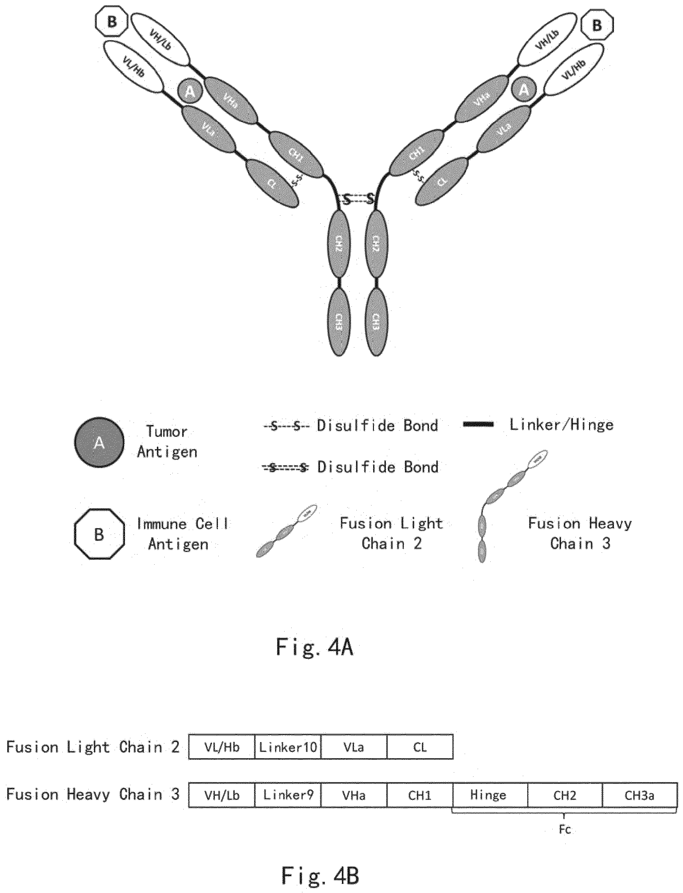

Figure 4A is a schematic diagram of the structure of bispecific antibody structure 4, and Figure 4B is a schematic diagram of the primary protein structure of each component of the antibody.

All of the above-mentioned four bispecific antibody structures are of symmetrical structure with a Fc fragment, and are all tetravalent, wherein two target the antigen A and the other two target the antigen B.

### Variable Regions of Antibody Sequences

**[Table 3] Variable Region Sequences of Anti-CD3 Antibody**

| Antibody Code (Source of Sequence) | Amino Acid Sequences of Variable Region of Anti-CD3 Antibody (Bold and underlined amino acids are CDR regions) | | | |
|---|---|---|---|---|
| | VH | SEQ ID NO. | VL | SEQ ID NO. |
| 2a5 | QVQLVESGGGVVQPGRSLRLSCAASGFTFSTYAMNWVRQAP GKGLEWVARIRSKYNNYATYYADSVKDRFTISRDDSKNTLY LQMNSLRAEDTAVYYCARHGNFGNSYVSWFAYWGQGTLV TVSS | 1 | QTVVTQEPSLTVSPGGTVTLTCRSSTGAVTTSNYAN WVQQKPGQAPRGLIGGTNKRAPGVPARFSGSLLGGK AALTLSGVQPEDEAEYYCALWYSNLWVFGGGTKVE IK | 2 |
| 2j5a | QVQLVESGGGVVQPGRSLRLSCAASGFTFSTYAMNWVRQAP GKGLEWVARIRSKYNNYATYYADSVKDRFTISRDDSKNTLY LQMNSLRAEDTAVYYCARHGNFGNSYVSWAAYWGQGTLV TVSS | 3 | QTVVTQEPSLTVSPGGTVTLTCRSSTGAVTTSNYAN WFQQKPGQAPRGLIGGTNKRAPGVPARFSGSLLGGK AALTLSGVQPEDEAEYYCALWYSNLWVFGGGTKVE IK | 4 |
| I2C (US8236308) | EVQLVESGGGLVQPGGSLKLSCAASGFTFNKYAMNWVRQAP GKGLEWVARIRSKYNNYATYYADSVKDRFTISRDDSKNTAY LQMNNLKTEDTAVYYCVRHGNFGNSYISYWAYWGQGTLVT VSS | 5 | QTVVTQEPSLTVSPGGTVTLTCGSSTGAVTSGNYPN WVQQKPGQAPRGLIGGTKFLAPGTPARFSGSLLGGK AALTLSGVQPEDEAEYYCVLWYSNRWVFGGGTKLT VL | 6 |

**[Table 4] Variable Region Sequences of Anti-B7-H3 Antibody**

| Antibody Code (Source of Sequence) | Amino Acid Sequences of Variable Region of Anti-CD38 Antibody (Bold and underlined amino acids are CDR regions) | | | |
|---|---|---|---|---|
| | VH | SEQ ID NO. | VL | SEQ ID NO. |
| 8H9 (Cancer Research 61, 4048-4054, May 15, 2001) | QVQLQQSGAELVKPGASVKLSCKASGYTFTNYDINWVRQRP EQGLEWIGWIFPGDGSTQYNEKFKGKATLTTDTSSSTAYMQ LSRLTSEDSAVYFCARQTTATWFAYWGQGTLVTVSS | 7 | DIVMTQSPATLSVTPGDRVSLSCRASQSISDYLHWYQ QKSHESPRLLIKYASQSISGIPSRFSGSGSGSDFTLSINS VEPEDVGVYYCQNGHSFPLTFGAGTKLELK | 8 |
| BRCA69D (US20120294796A1) | QVQLQQSGAELARPGASVKLSCKASGYTFTSYWMQWVKQR PGQGLEWIGTIYPGDGDTRYTQKFKGKATLTADKSSSTAYM QLSSLASEDSAVYYCARRGIPRLWYFDVWGAGTTVTVSS | 9 | DIQMTQTTSSLSASLGDRVTISCRASQDISNYLNWYQ QKPDGTVKLLIYYTSRLHSGVPSRFSGSGSGTDYSLTI DNLEQEDIATYFCQQGNTLPPTFGGGTKLEIK | 10 |

**[Table 5] Variable Region Sequences of Anti-CD38 Antibody**

| Antibody Code (Source of Sequence) | Amino Acid Sequences of Variable Region of Anti-CD38 Antibody (B old and u nderlined amino acids are CDR regions) | | | |
|---|---|---|---|---|
| | VH | SEQ ID NO. | VL | SEQ ID NO. |
| Dara (US9040050) | | 11 | | 12 |
| MOR (US8088896) | QVQLVESGGGLVQPGGSLRLSCAASGFTFSSYYMNWVRQAP GKGLEWVSGISGDPSNTYYADSVKGRFTISRDNSKNTLYLQ MNSLRAEDTAVYYCARDLPLVYTGFAYWGQGTLVTVSS | 13 | DIELTQPPSVSVAPGQTARISCSGDNLRHYYVYWYQ QKPGQAPVLVIYGDSKRPSGIPERFSGSNSGNTATLTI SGTQAEDEADYYCQTYTGGASLVFGGGTKLTVLGQ | 14 |
| 2F5 (US9040050) | QVQLVQSGAEVKKPGSSVKVSCKASGGTFSSYAFSWVRQAP GQGLEWMGRVIPFLGIANSAQKFQGRVTITADKSTSTAYMD LSSLRSEDTAVYYCARDDIAALGPFDYWGQGTLVTVSS | 15 | DIQMTQSPSSLSASVGDRVTITCRASQGISSWLAWYQ QKPEKAPKSLIYAASSLQSGVPSRFSGSGSGTDFTLTIS SLQPEDFATYYCQQYNSYPRTFGQGTKVEIK | 16 |

**[Table 6] Variable Region Sequences of Anti-EpCAM Antibody**

| Antibody Code (Source of Sequence) | Amino Acid Sequences of Variable Region of Anti-BCMA Antibody (Bold and underlined amino acids are CDR regions) | | | |
|---|---|---|---|---|
| | VH | SEQ ID NO. | VL | SEQ ID NO. |
| 3-171 (US20100310463) | QVQLVQSGAEVKKPGSSVKVSCKASGGTFSSYAISWVRQAP GQGLEWMGGIIPIFGTANYAQKFQGRVTITADESTSTAYME LSSLRSEDTAVYYCARGLLWNYWGQGTLVTVSS | 17 | EIVMTQSPATLSVSPGERATLSCRASQSVSSNLAWYQ QKPGQAPRLIIYGASTTASGIPARFSASGSGTDFTLTIS SLQSEDFAVYYCQQYNNWPPAYTFGQGTKLEIK | 18 |
| 2-6 (TW102107344) | EVQLVESGPELKKPGETVKISCKASGYTFTDYSMHWVKQAP GKGLKWMGWINTETGEPTYADDFKGRFAFSLETSASTAYLQ INNLKNEDTATYFCARTAVYWGQGTTVTVSS | 19 | DIQMTQSPSSLSASLGERVSLTC**RASQEISVSLS**WLQQ EPDGTIKRLIY**ATSTLDS**GVPKRFSGSRSGSDYSLTISS LESEDFVDYYC**LQYASYPWT**FGGGTKLEIK | 20 |

**[Table 7] Variable Region Sequences of Anti-BCMA Antibody**

| Antibody Code (Source of Sequence) | Amino Acid Sequences of Variable Region of Anti-BCMA Antibody (Bold and underlined amino acids are CDR regions) | | | |
|---|---|---|---|---|
| | VH | SEQ ID NO. | VL | SEQ ID NO. |
| B50 (US9598500) | QVQLVQSGAEVKKPGASVKVSCKASGYSFPDYYINWVRQAP GQGLEWMGWIYFASGNSEYNQKFTGRVTMTRDTSINTAYM | 21 | DIVMTQTPLSLSVTPGQPASISCKSSQSLVHSNGNTY LHWYLQKPGQSPQLLIYKVSNRFSGVPDRFSGSGSGT | 22 |
| | ELSSLTSEDTAVYFCASLYDYDWYFDVWGQGTMVTVSS | | DFTLKISRVEAEDVGIYYCSQSSIYPWTFGOGTKLEIK | |
| B140153 (WO2016090320A1) | QVQLVQSGAEVKKPGSSVKVSCKASGGTFSSYAISWVRQAP GQGLEWMGRIIPILGIANYAQKFQGRVTITADKSTSTAYMEL SSLRSEDTAVYYCARGGYYSHDMWSEDWGQGTLVTVSS | 23 | LPVLTQPPSASGTPGQRVTISCSGRSSNIGSNSVNWYR QLPGAAPKLLIYSNNQRPPGVPVRFSGSKSGTSASLAI SGLQSEDEATYYCATWDDNLNVHYVFGTGTKVTVL G | 24 |
| B69 (US2017051068A1) | QLQLQESGPGLVKPSETLSLTCTVSGGSISSGSYFWGWIRQPP GKGLEWIGSIYYSGITYYNPSLKSRVTISVDTSKNQFSLKLSS VTAADTAVYYCARHDGAVAGLFDYWGQGTLVTVSS | 25 | SYVLTQPPSVSVAPGQTARITCGGNNIGSKSVHWYQ QPPGQAPVVVVYDDSDRPSGIPERFSGNSNGNTATLT ISRVEAGDEAVYYCQVWDSSSDHVVFGGGTKLTVL | 26 |

**[Table 8] Variable Region Sequences of Anti-CTLA-4 Antibody**

| Antibody Code (Source of Sequence) | Amino Acid Sequences of Variable Region of Anti-CTLA-4 Antibody (Bold and underlined amino acids are CDR regions) | | | |
|---|---|---|---|---|
| | VH | SEQ ID NO. | VL | SEQ ID NO. |
| Yervoy(US20020086 014A1) | QVQLVESGGGVVQPGRSLRLSCAASGFTFSSYTMHWVRQAP GKGLEWVTFISYDGNNKYYADSVKGRFTISRDNSKNTLYLQ MNSLRAEDTAIYYCARTGWLGPFDYWGQGTLVTVSS | 27 | EIVLTQSPGTLSLSPGERATLSCRASQSVGSSYLAWY QQKPGQAPRLLIYGAFSRATGIPDRFSGSGSGTDFTLT ISRLEPEDFAVYYCQQYGSSPWTFGQGTKVEIK | 28 |

**[Table 9] Variable Region Sequences of Anti-TIGIT Antibody**

| Antibody Code (Source of Sequence) | Amino Acid Sequences of Variable Region of Anti-TIGIT Antibody (Bold and underlined amino acids are CDR regions) | | | |
|---|---|---|---|---|
| | VH | SEQ ID NO. | VL | SEQ ID NO. |
| 10A7 (US20090258013A1) | EVQLVESGGGLTQPGKSLKLSCEASGFTFSSFTMHWVRQSPG KGLEWVAFIRSGSGIVFYADAVRGRFTISRDNAKNLLFLQMN DLKSEDTAMYYCARRPLGHNTFDSWGQGTLVTVSS | 29 | DIVMTQSPSSLAVSPGEKVTMTCKSSQSLYYSGVKE NLLAWYQQKPGQSPKLLIYYASIRFTGVPDRFTGSGS GTDYTLTITSVQAEDMGQYFCQQGINNPLTFGDGTK LEIK | 30 |
| MAB10 (WO2017059095A1) | QVQLQESGPGLVKPSQTLSLTCTVSGGSIESGLYYWGWIRQP PGKGLEWIGSIYYSGSTYYNPSLKSRATISVDTSKNQFSLKLS SVTAADTAVYYCARDGVLALNKRSFDIWGQGTMVTVSS | 31 | EIVLTQSPGTLSLSPGERATLSCRASQSVSSSYLAWYQ QKPGQAPRLLIYGASSRATGIPDRFSGSGSGTDFTLTIS RLEPEDFAVYYCQQHTVRPPLTFGGGTKVEIK | 32 |

**[Table 10] Variable Region Sequences of Anti-LAG-3 Antibody**

| Antibody Code (Source of Sequence) | Amino Acid Sequences of Variable Region of Anti-LAG-3 Antibody (Bold and underlined amino acids are CDR regions) | | | |
|---|---|---|---|---|
| | VH | SEQ ID NO. | VL | SEQ ID NO. |
| LAG35 (US9505839B2) | QVQLQQWGAGLLKPSETLSLTCAVYGGSFSDYYWNWIRQPP GKGLEWIGEINHRGSTNSNPSLKSRVTLSLDTSKNQFSLKLRS VTAADTAVYYCAFGYSDYEYNWFDPWGQGTLVTVSS | 33 | EIVLTQSPATLSLSPGERATLSCRASQSISSYLAWYQQ KPGQAPRLLIYDASNRATGIPARFSGSGSGTDFTLTISS LEPEDFAVYYCQQRSNWPLTFGQGTNLEIK | 34 |
| L3E3(US9902772B2 ) | EVQLLESGAEVKKPGASVKVSCKASGYTFTSYYMHWVRQAP GQGLEWMGIINPSAGSTSYAQKFQGRVTMTRDTSTSTVYME LSSLRSEDTAVYYCARELMATGGFDYWGQGTLVTVSS | 35 | QSVLTQPASASGSPGQSITISCTGTSSDVGGYNYVSW YQQHPGKAPKLMIYDVSNRPSGVSNRFSGSKSGNTA SLTISGLQAEDEANYYCSSYTSSSTNVFGTGTKVTVL | 36 |

**[Table 11] Variable Region Sequences of Anti-PD-1 Antibody**

| Antibody Code (Source of Sequence) | Amino Acid Sequences of Variable Region of Anti-PD-1 Antibody (Bold and underlined amino acids are CDR regions) | | | |
|---|---|---|---|---|
| | VH | SEQ ID NO. | VL | SEQ ID NO. |
| 5C4 (WO2006121168) | | 37 | | 38 |
| H409A11 (WO2008156712A1) | | 39 | | 40 |

**[Table 12] Variable Region Sequences of Anti-PD-L1 Antibody**

| Antibody Code (Source of Sequence) | Amino Acid Sequences of Variable Region of Anti-PD-L1 Antibody (Bold and underlined amino acids are CDR regions) | | | |
|---|---|---|---|---|
| | VH | SEQ ID NO. | VL | SEQ ID NO. |
| S70 (WO2010077634A1) | | 41 | | 42 |
| Avelumab (WO2013079174A1) | EVQLLESGGGLVQPGGSLRLSCAASGFTF**SSYIMM**WVRQAPG KGLEWVS**SIYPSGGITFYADTVKG**RFTISRDNSKNTLYLQMN SLRAEDTAVYYCAR**IKLGTVTTVDY**WGQGTLVTVSS | 43 | QSALTQPASVSGSPGQSITISCTGTSSDVGGYNYVSW YQQHPGKAPKLMIYDVSNRPSGVSNRFSGSKSGNTA SLTISGLQAEDEADYYCSSYTSSSTRVFGTGTKVTVL | 44 |
| 12A4(US7943743B2 | QVQLVQSGAEVKKPGSSVKVSCKTSGDTFSTYAISWVRQAPG | 45 | EIVLTQSPATLSLSPGERATLSCRASQSVSSYLAWYQ | 46 |
| ) | QGLEWMGGIIPIFGKAHYAQKFQGRVTITADESTSTAYMELS SLRSEDTAVYFCARKFHFVSGSPFGMDVWGQGTTVTVSS | | QKPGQAPRLLIYDASNRATGIPARFSGSGSGTDFTLTI SSLEPEDFAVYYCQQRSNWPTFGQGTKVEIK | |

**[Table 13] Variable Region Sequences of Anti-CD16 Antibody**

| Antibody Code (Source of Sequence) | Amino Acid Sequences of Variable Region of Anti-CD16 Antibody (Bold and underlined amino acids are CDR regions) | | | |
|---|---|---|---|---|
| | VH | SEQ ID NO. | VL | SEQ ID NO. |
| NM3E2 | EVQLVESGGGVVRPGGSLRLSCAASGFTFDDYGMSWVRQAP GKGLEWVSGINWNGGSTGYADSVKGRFTISRDNAKNSLYLQ MNSLRAEDTAVYYCARGRSLLFDYWGQGTLVTVSR | 47 | SELTQDPAVSVALGQTVRITCQGDSLRSYYASWYQQ KPGQAPVLVIYGKNNRPSGIPDRFSGSSSGNTASLTIT GAQAEDEADYYCNSRDSSGNHVVFGGGTKLTVL | 48 |

**[Table 14] Variable Region Sequence of Anti-SLAMF7 Antibody**

| Antibody Code (Source of Sequence) | Amino Acid Sequence of Variable Region of Anti-SLAMF7 Antibody (Bold and underlined amino acids are CDR regions) | | | |
|---|---|---|---|---|
| | VH | SEQ ID NO. | VL | SEQ ID NO. |
| Elotuzumab (WO2004100898A2) | EVQLVESGGGLVQPGGSLRLSCAASGFDFSRYWMSWVRQAP GKGLEWIGEINPDSSTINYAPSLKDKFIISRDNAKNSLYLQMN SLRAEDTAVYYCARPDGNYWYFDVWGQGTLVTVSS | 49 | DIQMTQSPSSLSASVGDRVTITCKASQDVGIAVAWY QQKPGKVPKLLIYWASTRHTGVPDRFSGSGSGTDFTL TISSLQPEDVATYYCQQYSSYPYTFGQGTKVEIK | 50 |

**[Table 15] Variable Region Sequences of Anti-CEA Antibody**

| Antibody Code (Source of Sequence) | Amino Acid Sequences of Variable Region of Anti-CEA Antibody (Bold and underlined amino acids are CDR regions) | | | |
|---|---|---|---|---|
| | VH | SEQ ID NO. | VL | SEQ ID NO. |
| hPR1A3(Cancer Immunol Immunother (1999) 47: 299-306) | QVQLVQSGSELKKPGASVKVSCKASGYTFTVFGMNWVRQA PGQGLEWMGWINTKTGEATYVEEFKGRFVFSLDTSVSTAY LQISSLKADDTAVYYCARWDFYDYVEAMDYWGQGTTVTVS S | 51 | DIQMTQSPSSLSASVGDRVTITC**KASQNVGTNVA**WY QQKPGKAPKLLIY**SASYRYS**GVPSRFSGSGSGTDFTFT ISSLQPEDIATYYC**HQYYTYPLFT**FGQGTKVEIK | 52 |

**[Table 16] Variable Region Sequences of Anti-VEGF Antibody**

| Antibody Code (Source of Sequence) | Amino Acid Sequences of Variable Region of Anti-VEGF Antibody (Bold and underlined amino acids are CDR regions) | | | |
|---|---|---|---|---|
| | VH | SEQ ID NO. | VL | SEQ ID NO. |
| Avastin | | 53 | DIQMTQSPSSLSASVGDRVTITCSASQDISNYLNWYQ QKPGKAPKVLIYFTSSLHSGVPSRFSGSGSGTDFTLTI | 54 |
| | | | SSLQPEDFATYYCQQYSTVPWTFGQGTKVEIK | |
| B2041 (WO2005012359A2) | EVQLVESGGGLVQPGGSLRLSCAASGFSINGSWIFWVRQAPG KGLEWVGAIWPFGGYTHYADSVKGRFTISADTSKNTAYLQ MNSLRAEDTAVYYCARWGHSTSPWAMDYWGQGTLVTVSS | 55 | DIQMTQSPSSLSASVGDRVTITCRASQVIRRSLAWYQ QKPGKAPKLLIYAASNLASGVPSRFSGSGSGTDFTLTI SSLQPEDFATYYCQQSNTSPLTFGQGTKVEIK | 56 |
| G631 (WO2005012359A2) | EVQLVESGGGLVQPGGSLRLSCAASGFTISDYWIHWVRQAPG KGLEWVAGITPAGGYTYYADSVKGRFTISADTSKNTAYLQM NSLRAEDTAVYYCARFVFFLPYAMDYWGQGTLVTVSS | 57 | DIQMTQSPSSLSASVGDRVTITCRASQDVSTAVAWY QQKPGKAPKLLIYSASFLYSGVPSRFSGSGSGTDFTLT ISSLQPEDFATYYCQQGYGNPFTFGQGTKVEIK | 58 |

**[Table 17] Variable Region Sequences of Anti-TGF-β Antibody**

| Antibody Code (Source of Sequence) | Amino Acid Sequences of Variable Region of Anti-TGF-β Antibody (Bold and underlined amino acids are CDR regions) | | | |
|---|---|---|---|---|
| | VH | SEQ ID NO. | VL | SEQ ID NO. |
| 3G12 | QVQLVQSGAEVKKPGSSVKVSCKASGYTFSSNVISWVRQAP GQGLEWMGGVIPIVDIANYAQRFKGRVTITADESTSTTYMEL SSLRSEDTAVYYCASTLGLVLDAMDYWGQGTLVTVSS | 59 | ETVLTQSPGTLSLSPGERATLSCRASQSLGSSYLAWY QQKPGQAPRLLIYGASSRAPGIPDRFSGSGSGTDFTLT ISRLEPEDFAVYYCQQYADSPITFGQGTRLEIK | 60 |
| 4B9 | QVQLVQSGAEVKKPGSSVKVSCKASGYTFSSNVISWVRQAP GQGLEWMGGVIPIVDIANYAQRFKGRVTITADESTSTTYMEL SSLRSEDTAVYYCALPRAFVLDAMDYWGQGTLVTVSS | 61 | ETVLTQSPGTLSLSPGERATLSCRASQSLGSSYLAWY QQKPGQAPRLLIYGASSRAPGIPDRFSGSGSGTDFTLT ISRLEPEDFAVYYCQQYADSPITFGQGTRLEIK | 62 |

**[Table 18] Variable Region Sequences of Anti-IL-10 Antibody**

| Antibody Code (Source of Sequence) | Amino Acid Sequences of Variable Region of Anti-TGF-β Antibody (Bold and underlined amino acids are CDR regions) | | | |
|---|---|---|---|---|
| | VH | SEQ ID NO. | VL | SEQ ID NO. |
| B-N10 | QVQLKQSGPGLLQPSQSLSISCTVSGFSLATYGVHWVRQSPG KGLEWLGVIWRGGSTDYSAAFMSRLSITKDNSKSQVFFKMN SLQADDTAIYFCAKQAYGHYMDYWGQGTSVTVSS | 63 | DVLMTQTPLSLPVSLGDQASISCRSSQNIVHSNGNTY LEWYLQKPGQSPKLLIYKVSNRFSGVPDRFSGSGSGT DFTLKITRLEAEDLGVYYCFQGSHVPWTFGGGTKLEI K | 64 |
| BT-063 | EVQLVESGGGLVQPGGSLRLSCAASGFSFATYGVHWVRQSP GKGLEWLGVIWRGGSTDYSAAFMSRLTISKDNSKNTVYLQM NSLRAEDTAVYFCAKQAYGHYMDYWGQGTSVTVSS | 65 | DVVMTQSPLSLPVTLGQPASISCRSSQNIVHSNGNTY LEWYLQRPGQSPRLLIYKVSNRFSGVPDRFSGSGSGT DFTLKISRVEAEDVGVYYCFQGSHVPWTFGQGTKVE IK | 66 |

**[Table 19] Variable Region Sequences of Anti-luciferase Antibody**

| Antibody Code (Source of Sequence) | Amino Acid Sequences of Variable Region of Anti-luciferase Antibody (Bold and underlined amino acids are CDR regions) | | | |
|---|---|---|---|---|
| | VH | SEQ ID NO. | VL | SEQ ID NO. |
| 4420 | EVKLDETGGGLVQPGRPMKLSCVASGFTFSDYWMNWVRQS PEKGLEWVAQIRNKPYNYETYYSDSVKGRFTISRDDSKSSVY LQMNNLRVEDMGIYYCTGSYYGMDYWGQGTSVTVSS | 67 | DVVMTQTPLSLPVSLGDQASISCRSSQSLVHSNGNTY LRWYLQKPGQSPKVLIYKVSNRFSGVPDRFSGSGSGT DFTLKISRVEAEDLGVYFCSQSTHVPWTFGGGTKLEI K | 68 |

### Sequence of Other Domains

(1) Amino acid sequences of linker domain

**[Table 20] Amino acid sequences of linker**

| Domain | Code | Amino acid sequences | SEQ ID NO. |
|---|---|---|---|
| Linkers | Lin1 | SS | 69 |
| | Lin2 | AS | 70 |
| | Lin3 | GGGGS | 71 |
| | Lin4 | GGGSAAA | 72 |
| | Lin5 | GGGGSAS | 73 |
| | Lin6 | GYPGGGGS | 74 |
| | Lin7 | GGGGSGGGGS | 75 |
| | Lin8 | APAPAPAPAPAP | 76 |
| | Lin9 | AEAAAKEAAAKA | 77 |
| | Lin10 | GKSSGSGSESKS | 78 |
| | Lin11 | GSTSGSGKSSEGKG | 79 |
| | Lin12 | EPKSSDKTHTSPPS | 80 |
| | Lin13 | GGGGSDKTHTSPPS | 81 |
| | Lin14 | GGGGSGGGGSGGGGS | 82 |
| | Lin15 | GAAAGGGGSGGGGS | 83 |
| | Lin16 | EAAAKGGGGSGGGGS | 84 |
| | Lin17 | GGGGSEAAAKGGGGS | 85 |
| | Lin18 | EAAAKEAAAKGGGGS | 86 |
| | Lin19 | AGGGGSGGGGSGGGGSG | 87 |
| | Lin20 | GSTSGSGKSSEGSGSTKG | 88 |
| | Lin21 | GAAAGGGGSGGGGSGGGGS | 89 |
| | Lin22 | GGGGSGGGGSGGGGSGGGGS | 90 |

(2) Amino acid sequences of Hinge domain

**[Table 21] Amino acid sequences of hinge**

| Domain | Code | Amino acid sequences | SEQ ID NO. |
|---|---|---|---|
| Hinges | Hin1 | DKTHTCP | 91 |
| | Hin2 | ERKCCVECP | 92 |
| | Hin3 | ELKTPLGDTTHTCPRCPEPKSCDTPPPCPRCPEPKSCDTPPPCPRCPEPKSCDTPPPCPR | 93 |
| | Hin4 | ESKYGPPCPSCP | 94 |
| | Hin5 | DKTHT | 95 |
| | Hin6 | ESKYGPPCPPCP | 96 |
| | Hin7 | EPKSSDKTHTCP | 97 |
| | Hin8 | GGGGSDKTHTCP | 98 |
| | Hin9 | RGRGSDKTHTCP | 99 |
| | Hin10 | DGDGSDKTHTCP | 100 |
| | Hin11 | GRGRGSDKTHTCP | 101 |
| | Hin12 | ASTRGRGSDKTHTCP | 102 |
| | Hin13 | GQPDGDASDKTHTCP | 103 |

(3) Amino acid sequence of light chain constant region CL domain

**[Table 22] Amino acid sequences of CL**

| Domain | Code | Amino acid sequences | SEQ ID NO. |
|---|---|---|---|
| CL | Lc1 | | 104 |
| | Lc2 | GQPKANPTVTLFPPSSEELQANKATLVCLISDFYPGAVTVAWKADGSPVKAGVETTKPSKQSNNKYAASSYLSLTPEQWKSH RSYSCQVTHEGSTVEKTVAPTECS | 105 |
| | Lc3 | GQPKAAPSVTLFPPSSEELQANKATLVCLISDFYPGAVTVAWKADSSPVKAGVETTTPSKQSNNKYAASSYLSLTPEQWKSH RSYSCQVTHEGSTVEKTVAPTECS | 106 |
| | Lc4 | GQPKAAPSVTLFPPSSEELQANKATLVCLISDFYPGAVTVAWKADSSPAKAGVETTTPSKQSNNKYAASSYLSLTPEQWKSH KSYSCQVTHEGSTVEKTVAPTECS | 107 |
| | Lc5 | GQPKAAPSVTLFPPSSEELQANKATLVCLISDFYPGAVKVAWKADGSPVNTGVETTTPSKQSNNKYAASSYLSLTPEQWKSH RSYSCQVTHEGSTVEKTVAPAECS | 108 |
| | Lc6 | GQPKAAPTVTLFPPSSEELQANKATLVCLISDFYPGAVKVAWKADSSPAKAGVETTTPSKQSNNKYAASSYLSLTPEQWKSH KSYSCQVTHEGSTVEKTVAPTECS | 109 |
| | Lc7 | | 110 |

(4) Amino acid sequences of CH1 domain of heavy chain constant region

**[Table 23] Amino acid sequences of CH1**

| Domain | Code | Amino acid sequences | SEQ ID NO. |
|---|---|---|---|
| CH1 | G1CH 1 | ASTKGPSVFPLAPSSKSTSGGTAALGCLVKDYFPEPVTVSWNSGALTSGVHTFPAVLQSSGLYSLSSVV TVPSSSLGTQTYICNVNHKPSNTKVDKKVEPKSC | 111 |
| | G2CH 1 | ASTKGPSVFPLAPCSRSTSESTAALGCLVKDYFPEPVTVSWNSGALTSGVHTFPAVLQSSGLYSLSSVVT VPSSNFGTQTYTCNVDHKPSNTKVDKTV | 112 |
| | G3CH 1 | ASTKGPSVFPLAPCSRSTSGGTAALGCLVKDYFPEPVTVSWNSGALTSGVHTFPAVLQSSGLYSLSSVV TVPSSSLGTQTYTCNVNHKPSNTKVDKRV | 113 |
| | G4CH 1 | ASTKGPSVFPLAPCSRSTSESTAALGCLVKDYFPEPVTVSWNSGALTSGVHTFPAVLQSSGLYSLSSVVT VPSSSLGTKTYTCNVDHKPSNTKVDKRV | 114 |

**[Table 24] Amino acid sequences of CH2 of Fc**

| **Combination Code** | Amino acid sequences of CH2 | SEQ ID NO. |
|---|---|---|
| G1CH2 | PCPAPELLGGPSVFLFPPKPKDTLMISRTPEVTCVVVDVSHEDPEVKFNWYVDGVEVHNAKTKPREEQYNSTYRVVSVLTVLHQDW LNGKEYKCKVSNKALPAPIEKTISKAK | 115 |
| G2CH2 | PCPAPPVAGPSVFLFPPKPKDTLMISRTPEVTCVVVDVSHEDPEVQFNWYVDGVEVHNAKTKPREEQFNSTFRVVSVLTVVHQDWL NGKEYKCKVSNKGLPAPIEKTISKTK | 116 |
| G2DCH2 | PCPAPPVAGPSVFLFPPKPKDTLMISRTPEVTCVVVDVSHEAPEVQFNWYVDGVEVHNAKTKPREEQFNSTFRVVSVLTVVHQDWL NGKEYKCKVSNKGLPAPIEKTISKTK | 117 |
| G3CH2 | CPAPELLGGPSVFLFPPKPKDTLMISRTPEVTCVVVDVSHEDPEVQFKWYVDGVEVHNAKTKPREEQYNSTF RVVSVLTVLHQDWLNGKEYKCKVSNKALPAPIEKTISKTK | 118 |
| G4CH2 | APEFLGGPSVFLFPPKPKDTLMISRTPEVTCVVVDVSQEDPEVQFNWYVDGVEVHNAKTKPREEQFNSTYRVVSVLTVLHQDWLNG KEYKCKVSNKGLPSSIEKTISKAK | 119 |

**[Table 25] Amino acid sequences of CH3 of Fc forming the heterodimer**

| Combination Code | Amino acid sequences of CH3 | SEQ ID NO. |
|---|---|---|
| G1CH3 | GQPREPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGK | 120 |
| G2CH3 | GQPREPQVYTLPPSREEMTKNQVSLTCLVKGFYPSDISVEWESNGQPENNYKTTPPMLDSDGSFFLYSKLT VDKSRWQQGNVFSCSVMHEALHNHYTQKSLSLSPGK | 121 |
| G3CH3 | GQPREPQVYTLPPSREEMTKNQVSLTCLVKGFYPSDIAVEWESSGQPENNYNTTPPMLDSDGSFFLYSKLT VDKSRWQQGNIFSCSVMHEALHNRFTQKSLSLSPGK | 122 |
| G4CH3 | GQPREPQVYTLPPSQEEMTKNQVSLTCLVKGFYPSDIAVEWESNGQPENNYKTTPPVLDSDGSFFLYSRLT VDKSRWQEGNVFSCSVMHEALHNHYTQKSLSLSLGK | 123 |
| G1KCH3 | | 124 |
| G2KCH3 | GQPREPQVYTLPPSREEMTKNQVSLTCLVKGFYPSDISVEWESNGQPENNYKTTPPMLDSDGSFFLYSKLT VDKSRWQQGNVFSCSVMHEALHNHYTQKSLSLSPG | 125 |
| G3KCH3 | GQPREPQVYTLPPSREEMTKNQVSLTCLVKGFYPSDIAVEWESSGQPENNYNTTPPMLDSDGSFFLYSKLT VDKSRWQQGNIFSCSVMHEALHNRFTQKSLSLSPG | 126 |
| G4KCH3 | GQPREPQVYTLPPSQEEMTKNQVSLTCLVKGFYPSDIAVEWESNGQPENNYKTTPPVLDSDGSFFLYSRLT VDKSRWQEGNVFSCSVMHEALHNHYTQKSLSLSLG | 127 |
| G1ACH3 | GQPREPQVYTLPPSRDELTKNQVSLTCLVKGFYPSDIAVEWESNGQPENNYKTTPPVLDSDGSFFLYSKLT VDKSRWQQGNVFSCSVMHEALHNHYTQKSLSLSPGA | 128 |

| | | |
|---|---|---|
| Specific sequences of Antigens | | |

**[Table 26] Amino acid sequences of tumor antigen**

| Name of tumor antigens (Source) | Amino acid sequences | SEQ ID NO. |
|---|---|---|
| Human CD38 (Source: UniProtKB - P28907) | VPRWRQQWSGPGTTKRFPETVLARCVKYTEIHPEMRHVDCQSVWDAFKGAFISKHPCNITEEDY QPLMKLGTQTVPCNKILLWSRIKDLAHQFTQVQRDMFTLEDTLLGYLADDLTWCGEFNTSKINY QSCPDWRKDCSNNPVSVFWKTVSRRFAEAACDVVHVMLNGSRSKIFDKNSTFGSVEVHNLQPEK VQTLEAWVIHGGREDSRDLCQDPTIKELESIISKRNIQFSCKNIYRPDKFLQCVKNPEDSSCTSEI | 129 |
| Human BCMA (Source: UniProtKB - Q02223) | MLQMAGQCSQNEYFDSLLHACIPCQLRCSSNTPPLTCQRYCNASVTNSVKGTNA | 130 |
| Human CTLA-4 (Source: UniProtKB - P16410) | | 131 |
| Human LAG-3 (Source: UniProtKB - P18627) | VPVVWAQEGAPAQLPCSPTIPLQDLSLLRRAGVTWQHQPDSGPPAAAPGHPLAPGPHPAAPSSWG PRPRRYTVLSVGPGGLRSGRLPLQPRVQLDERGRQRGDFSLWLRPARRADAGEYRAAVHLRDRA LSCRLRLRLGQASMTASPPGSLRASDWVILNCSFSRPDRPASVHWFRNRGQGRVPVRESPHHHLA ESFLFLPQVSPMDSGPWGCILTYRDGFNVSIMYNLTVLGLEPPTPLTVYAGAGSRVGLPCRLPAGV GTRSFLTAKWTPPGGGPDLLVTGDNGDFTLRLEDVSQAQAGTYTCHIHLQEQQLNATVTLAIITV TPKSFGSPGSLGKLLCEVTPVSGQERFVWSSLDTPSQRSFSGPWLEAQEAQLLSQPWQCQLYQGE RLLGAAVYFTELSSPGAQRSGRAPGALPAGHL | 132 |
| Human TIGIT (Source: UniProtKB - Q495A1) | MMTGTIETTGNISAEKGGSIILQCHLSSTTAQVTQVNWEQQDQLLAICNADLGWHISPSFKDRVAP GPGLGLTLQSLTVNDTGEYFCIYHTYPDGTYTGRIFLEVLESSVAEHGARFQIP | 133 |
| Human PD-1 (Source: UniProtKB - Q15116) | | 134 |
| Human PD-L 1 (Source: UniProtKB - Q9NZQ7) | FTVTVPKDLYVVEYGSNMTIECKFPVEKQLDLAALIVYWEMEDKNIIQFVHGEEDLKVQHSSYRQ RARLLKDQLSLGNAALQITDVKLQDAGVYRCMISYGGADYKRITVKVNAPYNKINQRILVVDPV TSEHELTCQAEGYPKAEVIWTSSDHQVLSGKTTTTNSKREEKLFNVTSTLRINTTTNEIFYCTFRRL DPEENHTAELVIPELPLAHPPNER | 135 |
| Human SLAMF7 (Source: UniProtKB - Q9NQ25) | SGPVKELVGSVGGAVTFPLKSKVKQVDSIVWTFNTTPLVTIQPEGGTIIVTQNRNRERVDFPDGGY SLKLSKLKKNDSGIYYVGIYSSSLQQPSTQEYVLHVYEHLSKPKVTMGLQSNKNGTCVTNLTCCM EHGEEDVIYTWKALGQAANESHNGSILPISWRWGESDMTFICVARNPVSRNFSSPILARKLCEGAA DDPDSSM | 136 |
| Human CEA (Source: UniProtKB - P06731) | KLTIESTPFNVAEGKEVLLLVHNLPQHLFGYSWYKGERVDGNRQIIGYVIGTQQATPGPAYSGREI IYPNASLLIQNIIQNDTGFYTLHVIKSDLVNEEATGQFRVYPELPKPSISSNNSKPVEDKDAVAFTCE PETQDATYLWWVNNQSLPVSPRLQLSNGNRTLTLFNVTRNDTASYKCETQNPVSARRSDSVILNV LYGPDAPTISPLNTSYRSGENLNLSCHAASNPPAQYSWFVNGTFQQSTQELFIPNITVNNSGSYTCQ AHNSDTGLNRTTVTTITVYAEPPKPFITSNNSNPVEDEDAVALTCEPEIQNTTYLWWVNNQSLPVS PRLQLSNDNRTLTLLSVTRNDVGPYECGIQNKLSVDHSDPVILNVLYGPDDPTISPSYTYYRPGVN LSLSCHAASNPPAQYSWLIDGNIQQHTQELFISNITEKNSGLYTCQANNSASGHSRTTVKTITVSAE LPKPSISSNNSKPVEDKDAVAFTCEPEAQNTTYLWWVNGQSLPVSPRLQLSNGNRTLTLFNVTRN DARAYVCGIQNSVSANRSDPVTLDVLYGPDTPIISPPDSSYLSGANLNLSCHSASNPSPQYSWRING IPQQHTQVLFIAKITPNNNGTYACFVSNLATGRNNSIVKSITVSASGTSPGLSA | 137 |
| Human CD3ε (Source: UniProtKB - P07766) | DGNEEMGGITQTPYKVSISGTTVILTCPQYPGSEILWQHNDKNIGGDEDDKNIGSDEDHLSLKEFS ELEQSGYYVCYPRGSKPEDANFYLYLRARVCENCMEMD | 138 |
| Human CD16A (Source: UniProtKB - | GMRTEDLPKAVVFLEPQWYRVLEKDSVTLKCQGAYSPEDNSTQWFHNESLISSQASSYFIDAATV DDSGEYRCQTNLSTLSDPVQLEVHIGWLLLQAPRWVFKEEDPIHLRCHSWKNTALHKVTYLQNG | 139 |
| P08637) | KGRKYFHHNSDFYIPKATLKDSGSYFCRGLFGSKNVSSETVNITITQGLAVSTISSFFPPGYQ | |
| Human TGF-β1 (Source: UniProtKB - P01137) | ALDTNYCFSSTEKNCCVRQLYIDFRKDLGWKWIHEPKGYHANFCLGPCPYIWSLDTQYSKVLAL YNQHNPGASAAPCCVPQALEPLPIVYYVGRKPKVEQLSNMIVRSCKCS | 140 |
| Human TGF-β2 (Source: UniProtKB - P61812) | ALDAAYCFRNVQDNCCLRPLYIDFKRDLGWKWIHEPKGYNANFCAGACPYLWSSDTQHSRVLS LYNTINPEASASPCCVSQDLEPLTILYYIGKTPKIEQLSNMIVKSCKCS | 141 |
| Human TGF-β3 (Source: UniProtKB - P10600) | | 142 |
| Human VEGFA (Source: UniProtKB - P15692) | APMAEGGGQNHHEVVKFMDVYQRSYCHPIETLVDIFQEYPDEIEYIFKPSCVPLMRCGGCCNDEG LECVPTEESNITMQIMRIKPHQGQHIGEMSFLQHNKCECRPKKDRARQEKKSVRGKGKGQKRKR KKSRYKSWSVYVGARCCLMPWSLPGPHPCGPCSERRKHLFVQDPQTCKCSCKNTDSRCKARQLE LNERTCRCDKPRR | 143 |
| Human IL-10 (Source: UniProtKB - P22301) | PGQGTQSENSCTHFPGNLPNMLRDLRDAFSRVKTFFQMKDQLDNLLLKESLLEDFKGYLGCQAL SEMIQFYLEEVMPQAENQDPDIKAHVNSLGENLKTLRLRLRRCHRFLPCENKSKAVEQVKNAFN KLQEKGIYKAMSEFDIFINYIEAYMTMKIRN | 144 |
| Human IL-10R (Source: UniProtKB - Q13651) | HGTELPSPPSVWFEAEFFHHILHWTPIPNQSESTCYEVALLRYGIESWNSISNCSQTLSYDLTAVTL DLYHSNGYRARVRAVDGSRHSNWTVTNTRFSVDEVTLTVGSVNLEIHNGFILGKIQLPRPKMAPA NDTYESIFSHFREYEIAIRKVPGNFTFTHKKVKHENFSLLTSGEVGEFCVQVKPSVASRSNKGMWS KEECISLTRQYFTVTN | 145 |

### Preparation of Antibody and Detection of Antibody Activity

### Example 1. Preparation of Bispecific Antibody

### 1. Method for Constructing Plasmid

pcDNA3.1 (purchased from invitrogen) or pCHO1.0 (purchased from Gibco) is used as a vector.

### 1.1 Amplification of DNA Fragment of interest by Polymerase Chain Reaction (PCR)

| | |
|---|---|
| PCR System (µl) | 50µl |
| DNA Polymerase (2× PrimeSTAR Max Premix, TaKaRa, Item No. R405A) | 25µl |
| Forward Primer (10uM) | 1µl |
| Reverse Primer (10uM) | 1µl |
| Template DNA ^{∗} | 1µl |
| H₂O | 22µl |

| | |
|---|---|
| ^{∗}Template DNA is synthesized by Wuhan Genecreate. The DNA sequence is obtained by reverse translation based on the amino acid sequences of SEQ ID NOs. 1 to 145. | |

| PCR Program | | |
|---|---|---|
| Temperature | Time | Number of Cycles |
| 95°C | 5min | 1 |
| 98°C | 10s | 25 to 30 |
| 55°C | 5s | |
| 72°C | 12kb/min | |
| 4°C | ∞ | 1 |

| | | |
|---|---|---|
| The resulted PCR product is the fragment DNA of interest. | | |

### 1.2 Digestion of Vector Plasmid by Restriction Enzyme

| | |
|---|---|
| Vector plasmid DNA^{∗} | 10µg |
| buffer | 10µl |
| Restriction Enzyme (Such as NotI, NruI or BamHI-HF etc.) | 5µl |
| H₂O | Add to 100µl |
| Total Volume | 100µl |

Enzyme digestion is performed at the optimum temperature for 4 hours.

The enzyme-digested product is the enzyme-digested vector DNA.

^{∗}The vectors are divided into two types: a heavy chain expression vector and a light chain expression vector. The heavy chain expression vector have a signal peptide and a heavy chain constant region DNA sequence of human IgG1 (GenBank accession number MG920253.1) including a CHI, a hinge, a CH2 and a CH3; and a restriction site is located between 3'of the signal peptide and 5' end of CHI; wherein the light chain expression vector has a signal peptide and a human kappa or lambda light chain constant region DNA sequence, and a restriction site is located between the 3' of the signal peptide and the 5'end of the light chain constant region.

### 1.3 Purification of PCR products or enzyme-digested products

Tiangen's DNA purification reagents were used. As for specific steps, please refer to the instructions of the Tiangen kit. The resultant purified product was the purified fragment DNA of interest and the purified enzyme-digested vector DNA.

### (1) Recombination of fragments of interest

| | |
|---|---|
| 5XCE II Buffer | 4 µl |
| Recombinase (Exnase II, Vazyme, Item No. C112-01) | 2 µl |
| Enzyme-Digested Vector | 50-200 ng |
| Fragment 1 | 20-200 ng |
| Fragment 2 | 20-200 ng |
| H₂O | Up to 20µl |

Recombination conditions: 37°C for 30 minutes. The resultant recombinant product was placed on ice, and was ready for transformation.

The heavy chain fragment was recombined into the enzyme-digested heavy chain expression vector DNA, and the light chain fragment was recombined into the enzyme-digested light chain expression vector DNA.

### 1.4 Heat shock transformation

10µl of the recombinant product was transformed into 100µl of Trans10 competent cells by heat shock according to the conventional method. A single colony was picked, and was sequenced by Wuhan Genecreate. The plasmid obtained by recombining the heavy chain fragment into the enzyme-digested expression vector DNA is called a heavy chain expression plasmid, and the plasmid obtained by recombining the light chain fragment into the enzyme-digested expression vector DNA is called a light chain expression plasmid, and the plasmid obtained by recombining the fusion peptide fragment into the enzyme-digested expression vector DNA is called a fusion peptide expression plasmid, the plasmid obtained by recombining the fusion heavy chain fragment into the enzyme-digested expression vector DNA is called a fusion heavy chain expression plasmid, and the plasmid obtained by recombining the fusion chain fragment into the enzyme-digested expression vector is called a fusion chain expression plasmid.

The construction of specific plasmid is as follows:
1) The bispecific antibody structure 1 in Figure 1 involves the construction of two plasmids. The two plasmids are: a fusion light chain expression plasmid (pFL) and a fusion heavy chain expression plasmid (pFH).
2) The bispecific antibody structure 2 in Figure 2 involves the construction of two plasmids. The two plasmids are: a light chain expression plasmid (pL) and a fusion heavy chain 2 expression plasmid (pFH2).
3) The bispecific antibody structure 3 in Figure 3 involves the construction of one plasmid. The plasmid is: a fusion peptide expression plasmid (pFP).
4) The bispecific antibody structure 4 in Figure 4 involves the construction of two plasmids. The two plasmids are: a fusion light chain 2 expression plasmid (pFL2) and a fusion heavy chain 3 expression plasmid (pFH3).

### 1.5 Expression Method of Bispecific antibody

Two transient transfection expression systems, CHO-S and 293E, are involved and the detailed steps are as follows:
(1) CHO-S transient transfection procedure (100ml of total transfection volume as an example)
   a) Cell passage was performed one day before the transfection, for example, CD-CHO medium (purchased from Thermo Fisher) can be used for cell passage; the suspension cell density was adjusted to 1×10⁶ cells/ml and the volume was 90 ml, so as to ensure that the cells were in a logarithmic growth phase, and the cell density can reach 2×10⁶ cells/ml at the next day of transfection;
   b) the cells were cultured at 37°C, 125rpm, 5% CO₂ overnight on a shaker;
   c) on the day for transfection, FectoPRO transfection reagent (purchased from Polyplus transfection company) was preheated to room temperature and mixed gently; 50µg of plasmid DNA was diluted by 10ml serum-free medium, such as opti PRO-SFM (purchased from Thermo Fisher), mixed gently, and added into 100µl transfection reagent, mixed well, and incubated at room temperature for 10 minutes to form a transfection complex; two or three plasmid DNAs were co-transfected during transfection.
   d) the transfection complex was evenly added into the prepared 90 ml of cells in the logarithmic growth phase, shaken up immediately after addition, and placed on a shaker for culture at 37°C, 125rpm, 5% CO₂;
   e) 2 to 4 hours after transfection, 75µl of transfection enhancer Fecto PRO^{®} Booster (purchased from Polyplus transfection company) was added;
   f) 18 to 24 hours after transfection, the cells were cooled to 32°C and incubated;
   g) feeding was performed on the 3^{rd}, 5^{th}, and 7^{th} day after transfection, and the feeding volume was 3.5% of the total cell volume;
   h) cells were harvested when cell viability was less than 70%, and the expression time was 9 to 13 days.
(2) 293E transient transfection procedure (20 ml of total transfection volume as an example)
   a) Cell passage was performed one day before the transfection, for example, FreeStyle^{™} 293 (purchased from Thermo Fisher) can be used for cell passage; the suspension cell density was adjusted to 0.6×10⁶ to 0.8×10⁶ cells/ml and the volume was 20ml, so as to ensure that the cells were in a logarithmic growth phase, and the cell density can reach 1.2×10⁶ to 1.6×10⁶ cells/ml at the next day of transfection;
   b) the cells were cultured at 37°C, 125rpm, 5% CO₂ overnight on a shaker;
   c) on the day for transfection, LPEI was preheated to room temperature and mixed gently before use;
   d) 20µg of DNA was diluted with 0.67ml of serum-free medium, such as FreeStyle^{™} 293, and mixed well;
   e) 40µg of LPEI was diluted with 0.67ml of serum-free medium, such as FreeStyle^{™} 293, and mixed well;
   f) the diluted LPEI obtained in step e) was added into the diluted DNA obtained in step d), mixed well quickly, and incubated at room temperature for 15 minutes to form a complex;
   g) the transfection complex obtained in step f) was evenly added into the prepared 20ml of cells in the logarithmic growth phase, shaken up immediately after addition, and placed on a shaker for culture at 37°C, 125rpm, 5% CO₂;
   h) feeding was performed on the first and third day after transfection, and the feeding volume was 5% of the total cell volume;
   i) the cells were expressed until the sixth day and then were harvest.
(3) The specific co-transfected plasmid DNA was as follows:
   1) In order to express the bispecific antibody 1 shown in Figure 1, plasmids pFL and pFH were required to be co-transfected into CHO-S or 293E cells for expression;
   2) In order to express the bispecific antibody 2 shown in Figure 2, plasmids pL and pFH2 were required to be co-transfected into CHO-S or 293E cells for expression;
   3) In order to express the bispecific antibody 3 shown in Figure 3, plasmid pFP was required to be transfected into CHO-S or 293E cells for expression;
   4) In order to express the bispecific antibody 4 shown in Figure 4, plasmids pFL2 and pFH3 were required to be co-transfected into CHO-S or 293E cells for expression.

In general, if two plasmids were co-transfected for expression, the molar ratio of the two plasmids can be 1:1, or any other ratio.

### 2. Purification Method of bispecific antibody:

Purification method of antibody mainly includes affinity chromatography, ion exchange chromatography, hydrophobic chromatography and molecular sieve, and the steps are as follows:
(1) the culture solution of antibody-expressing cells was harvested and centrifuged at 3000×g for 10 minutes, then the supernatant was collected, filtered with a 0.22µm filter, and stored at 4°C for later use;
(2) affinity chromatography (MabSelect SuRe GE 17-5438-01, 18ml of column volume as an example)
   a) equilibration: the column was equilibrated with a binding buffer (25 mM Tris, pH 7.0 to 7.4) until the UV detector and the conductance value retain stable or at baseline, wherein at least five column volumes were used for equilibration;
   b) sample loading: the filtered supernatant obtained in step (1) was loaded at a flow rate of 5ml/min;
   c) washing and equilibration: five column volumes of binding buffer were used for washing;
   d) elution: the sample was eluted with an elution buffer (50mM citric acid, pH 3.4±0.1) at a flow rate of 5ml/min, 5 column volumes were used for elution, and the elution peak was collected;
   e) neutralization: the eluent was neutralized with 1M Tris pH8.0, and the pH of the sample was adjusted to 6.0±0.1.
(3) ion exchange chromatography (a cation exchange chromatography as an example, HiTrap SP-HP GE 17-1151-01 5ml of column volume)
   a) sample preparation: the sample of affinity chromatography was microfiltered, and then was diluted with ultrapure water to make the conductance less than 5 mS/cm, and then the pH was adjusted to 6.0±0.1;
   b) equilibration and sample loading: the column was firstly equilibrated with 5 column volumes of buffer B (25mM citric acid+1M sodium chloride, the conductance of which should be 80 ~ 90 mS/cm, pH 6.0±0.1), and then equilibrated with at least five column volumes of buffer A (25mM citric acid, the conductance of which should be less than 5 mS/cm, pH 6.0±0.1) until the conductance, pH, and UV baseline were stable, and then the sample was loaded at a flow rate of 3ml/min;
   c) washing and equilibration: the column was washed with five column volumes of buffer A at a flow rate of 5ml/min;
   d) elution: the sample was eluted by 0-30% buffer B with 20 column volumes, and then by 100% buffer B with 10 column volumes; the flow rate in the whole process was 3ml/min, and the eluent was collected in separate tubes and tested.
(4) hydrophobic chromatography (Capto phenyl ImpRes filler GE XK16/20 11.5cm/23ml)
   a) sample treatment: 5M sodium chloride was added into the sample to 1M sodium chloride, and the pH was adjusted to 6.0;
   b) equilibration and sample loading: firstly, the column was equilibrated by five column volumes of buffer A (25mM citrate + 1M sodium chloride, pH 6.0±0.1) at a flow rate of 5ml/min; and the sample was loaded at a flow rate of 3.3ml/min;
   c) washing and equilibration: five column volumes of buffer A were used for washing at a flow rate of 5ml/min; five column volumes of 10% buffer B (25mM citrate, pH 6.0±0.1) were used for washing at a flow rate of 5ml/min;
   d) elution: 90% buffer B was used for elution at a flow rate of 5ml/min, the elution peaks were collected in separate tubes and then detected;
(5) molecular sieve (HiLoad Superdex 200pg GE 28989336 26/600)
   a) equilibration and sample loading: the column was equilibrated with two column volumes of buffer (20mM histidine+0.15M sodium chloride, pH6.0±0.1), and then the sample was loaded at a flow rate of 3ml/min;
   b) elution: two column volumes of buffer were used for elution, and the elution peaks were collected in separate tubes and then detected.

### Antibody codes of some specifically expressed antibodies and corresponding amino acid sequences of antibodies are shown in the following tables:

**[Table 27] The antibody codes and amino acid sequences of some antibodies with bispecific antibody structure 1**

| Anti -body Code | Peptides | Domain | Code | Amino acid sequences (bold and underlined amino acids are CDR) | SEQ ID NO. |
|---|---|---|---|---|---|
| Y100-A1 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Linl4 | GGGGSGGGGSGGGGS | 82 |
| | | VLb | S70 | | 42 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl4 | GGGGSGGGGSGGGGS | 82 |
| | | VHb | S70 | | 41 |
| | | Linker 3 | Linl4 | GGGGSGGGGSGGGGS | 82 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A2 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Lin21 | GAAAGGGGSGGGGSGGGGS | 89 |
| | | VLb | S70 | DIQMTQSPSSLSASVGDRVTITCRASQDVSTAVAWYQQKPGKAPKLLIYSASFLYSGVPSRFSGSGSGTDFTLTISSLQP EDFATYYCOOYLYHPATFGOGTKVEIK | 42 |
| | Fusion | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Heavy Chain | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl5 | GAAAGGGGSGGGGS | 83 |
| | | VHb | S70 | | 41 |
| | | Linker 3 Hinge | Lin6 Hin1 | GYPGGGGS DKTHTCP | 74 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A3 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Linl7 | GGGGSEAAAKGGGGS | 85 |
| | | VLb | S70 | | 42 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | S70 | | 41 |
| | | Linker 3 Hinge | Lin6 Hin1 | GYPGGGGS DKTHTCP | 74 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A4 | Fusion | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | Light | CL | Lc1 | | 104 |
| | | Linker 1 | Lin21 | GAAAGGGGSGGGGSGGGGS | 89 |
| | Chain | VLb | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl5 | GAAAGGGGSGGGGS | 83 |
| | | VHb | 12A4 | QVQLVQSGAEVKKPGSSVKVSCKTSGDTFSTYAISWVRQAPGQGLEWMGGIIPIFGKAHYAQKFQGRVTITADESTST AYMELSSLRSEDTAVYFCARKFHFVSGSPFGMDVWGQGTTVTVSS | 45 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A5 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL Linker 1 | Lc1 Lin17 | | 104 |
| | | VLb | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 85 46 |
| | | | | GGGGSEAAAKGGGGS | |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl7 | GGGGSEAAAKGGGGS | 85 |
| | | VHb | 12A4 | QVQLVQSGAEVKKPGSSVKVSCKTSGDTFSTYAISWVRQAPGQGLEWMGGIIPIFGKAHYAQKFQGRVTITADESTST AYMELSSLRSEDTAVYFCARKFHFVSGSPFGMDVWGQGTTVTVSS | 45 |
| | | Linker 3 Hinge | Lin6 Hin1 | GYPGGGGS DKTHTCP | 74 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A6 | Fusion Light Chain | VLa | S70 | | 42 |
| | | CL | Lc1 | | 104 |
| | | Linker 1 | Lin21 | GAAAGGGGSGGGGSGGGGS | 89 |
| | | VLb | G631 | DIQMTQSPSSLSASVGDRVTITC**RASQDVSTAVA**WYQQKPGKAPKLLIY**SASFLYS**GVPSRFSGSGSGTDFTLTISSLQP EDFATYYC**QQGYGNPFT**FGQGTKVEIK | 58 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl5 | GAAAGGGGSGGGGS | 83 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A7 | Fusion Light Chain | VLa | S70 | | 42 |
| | | CL | Lc1 | RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC GGGGSEAAAKGGGGS | 104 |
| | | Linker 1 | Linl7 | GGGGSEAAAKGGGGS | 85 |
| | | VLb | G631 | DIQMTQSPSSLSASVGDRVTITC**RASQDVSTAVA**WYQQKPGKAPKLLIY**SASFLYS**GVPSRFSGSGSGTDFTLTISSLQP EDFATYYC**QQGYGNPFT**FGQGTKVEIK | 58 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl7 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A8 | Fusion Light | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | Chain | | | | |
| | | Linker 1 | Lin21 | GAAAGGGGSGGGGSGGGGS | 89 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl5 | GAAAGGGGSGGGGS | 83 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Linl7 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| Y100-A9 | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl7 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A10 | Fusion Light Chain | VLa | S70 | | 42 |
| | | CL | Lc1 | | 104 |
| | | Linker 1 | Linl7 | GGGGSEAAAKGGGGS | 85 |
| | | VLb | Avastin | | 54 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl7 | GGGGSEAAAKGGGGS | 85 |
| | | VHb | Avastin | | 53 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-A11 | Fusion Light Chain | VLa | S70 | | 42 |
| | | CL | Lc1 | | 104 |
| | | Linker 1 | Linl7 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Linl7 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-AC1 | Fusion Light Chain | VLa | S70 | | 42 |
| | | CL | Lc1 | | 104 |
| | | Linker 1 | Lin21 | GAAAGGGGSGGGGSGGGGS | 89 |
| | | VLb | 4420 | DVVMTQTPLSLPVSLGDQASISCRSSQSLVHSNGNTYLRWYLQKPGQSPKVLIYKVSNRFSGVPDRFSGSGSGTDFTL KISRVEAEDLGVYFCSQSTHVPWTFGGGTKLEIK | 68 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | 4420 | EVKLDETGGGLVQPGRPMKLSCVASGFTFSDYWMNWVRQSPEKGLEWVAQIRNKPYNYETYYSDSVKGRFTISRD DSKSSVYLQMNNLRVEDMGIYYCTGSYYGMDYWGQGTSVTVSS | 67 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-AC2 | Fusion Light Chain | VLa | 4420 | DVVMTQTPLSLPVSLGDQASISCRSSQSLVHSNGNTYLRWYLQKPGQSPKVLIYKVSNRFSGVPDRFSGSGSGTDFTL KISRVEAEDLGVYFCSQSTHVPWTFGGGTKLEIK | 68 |
| | | CL | Lc1 | | 104 |
| | | Linker 1 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | Fusion Heavy Chain | VHa | 4420 | EVKLDETGGGLVQPGRPMKLSCVASGFTFSDYWMNWVRQSPEKGLEWVAQIRNKPYNYETYYSDSVKGRFTISRD DSKSSVYLQMNNLRVEDMGIYYCTGSYYGMDYWGQGTSVTVSS | 67 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-AC3 | Fusion Light Chain | VLa | 4420 | DVVMTQTPLSLPVSLGDQASISCRSSQSLVHSNGNTYLRWYLQKPGQSPKVLIYKVSNRFSGVPDRFSGSGSGTDFTL KISRVEAEDLGVYFCSQSTHVPWTFGGGTKLEIK | 68 |
| | | CL | Lc1 | | 104 |
| | | Linker 1 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | Fusion Heavy Chain | VHa | 4420 | EVKLDETGGGLVQPGRPMKLSCVASGFTFSDYWMNWVRQSPEKGLEWVAQIRNKPYNYETYYSDSVKGRFTISRD DSKSSVYLQMNNLRVEDMGIYYCTGSYYGMDYWGQGTSVTVSS | 67 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-A1 | Fusion Light Chain | VLa | S70 | | 42 |
| | | CL | Lc1 | | 104 |
| | | Linker 1 | Lin21 | GAAAGGGGSGGGGSGGGGS | 89 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | .Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-A2 | Fusion Light Chain | VLa | S70 | | 42 |
| | | CL | Lc1 | | 104 |
| | | Linker 1 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-A3 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin21 | GAAAGGGGSGGGGSGGGGS | 89 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-A4 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-A5 | Fusion Light Chain | VLa | S70 | | 42 |
| | | CL | Lc1 | | 104 |
| | | Linker 1 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ | 111 |
| | Chain | | | TYICNVNHKPSNTKVDKKVEPKSC | |
| | | Linker 2 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | S70 | | 42 |
| | | CL | Lc1 | | 104 |
| | | Linker 1 | Lin21 | GAAAGGGGSGGGGSGGGGS | 89 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| Y103-A1 | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y103-A2 | Fusion Light Chain | VLa | S70 | | 42 |
| | | CL | Lc1 | | 104 |
| | | Linker 1 | Lin17 | GGGGSEAAAKGGGGS | 85 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y103-A3 | Fusion Light Chain | VLa | S70 | | 42 |
| | | CL | Lc1 | | 104 |
| | | Linker 1 | Lin17 | GGGGSEAAAKGGGGS | 85 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y104-A1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y104-A2 | Fusion Light Chain | VLa | 5C4 | | 38 |
| | | CL | Lc1 | | 104 |
| | | Linker 1 | Lin17 | GGGGSEAAAKGGGGS | 85 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y104-A3 | Fusion Light | VLa | 5C4 | | 38 |
| | | CL | Lc1 | | 104 |
| | Chain | Linker 1 | Lin17 | GGGGSEAAAKGGGGS | 85 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC GGGGSEAAAKGGGGS | 111 |
| | | Linker 2 | Lin17 | GGGGSEAAAKGGGGS | 85 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y105-A1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | 5C4 | | 38 |
| | | CL | Lc1 | | 104 |
| | | Linker 1 | Lin17 | GGGGSEAAAKGGGGS | 85 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| Y105-A2 | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | 5C4 | | 38 |
| | | CL | Lc1 | | 104 |
| | | Linker 1 | Lin17 | GGGGSEAAAKGGGGS | 85 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| Y105-A3 | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-A1 | | VLa | 5C4 | | 38 |
| | Fusion | CL | Lc1 | | 104 |
| | Light | Linker 1 | Lin21 | GAAAGGGGSGGGGSGGGGS | 89 |
| | Chain | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | Fusion Heavy | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Chain | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-A2 | | VLa | 5C4 | | 38 |
| | Fusion Light | CL | Lc1 | | 104 |
| | Chain | Linker 1 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | VHa | 5C4 | | 37 |
| | Fusion | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | Heavy Chain | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-A3 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | [?]VVHQDWLNGKEYKCKVSNKALPAPIEKTISKAK GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-A4 | Fusion Light Chain | VLa | 5C4 | | 38 |
| | | CL | Lc1 | | 104 |
| | | Linker 1 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | 5C4 | | 38 |
| | | CL | Lc1 | | 104 |
| | | Linker 1 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| Y106-A5 | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-A1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | | Linker 3 | Lin6 | GYPGGGGS | 74 |
| | | Hinge | Hin1 | DKTHTCP | 91 |
| | | CH2 | G2DCH2 | DKTHTCP PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | 5C4 | | 38 |
| | | CL | Lc1 | | 104 |
| | | Linker 1 | Lin17 | GGGGSEAAAKGGGGS | 85 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| Y110-A2 | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-A3 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin21 | GAAAGGGGSGGGGSGGGGS | 89 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | Fusion Heavy | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ | 111 |
| | Chain | Linker 2 | Lin15 | TYICNVNHKPSNTKVDKKVEPKSC GAAAGGGGSGGGGS | 83 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-A4 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin17 | GGGGSEAAAKGGGGS | 85 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | | Linker 3 Hinge | Lin6 Hin1 | GYPGGGGS DKTHTCP | 74 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y116-A1 | Fusion Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | | Linker 1 | Lin21 | GAAAGGGGSGGGGSGGGGS | 89 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | Fusion Heavy Chain | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y116-A2 | Fusion Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | | Linker 1 | Lin17 | GGGGSEAAAKGGGGS | 85 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | Fusion Heavy Chain | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y116-A3 | Fusion Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | | Linker 1 | Lin17 | GGGGSEAAAKGGGGS | 85 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | Fusion Heavy | Linker 2 VHb | Lin17 NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 85 |
| | | | | GGGGSEAAAKGGGGS | 47 |
| | Chain | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B1 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Lin14 | GGGGSGGGGSGGGGS | 82 |
| | | VHb | S70 | | 41 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin14 | GGGGSGGGGSGGGGS | 82 |
| | | VLb | S70 | | 42 |
| | | Linker 3 | Lin14 | GGGGSGGGGSGGGGS | 82 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B2 | Fusion Light | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | Chain | Linker 1 | Lin21 | GAAAGGGGSGGGGSGGGGS | 89 |
| | | VHb | S70 | EVQLVESGGGLVQPGGSLRLSCAASGFTFSDSWIHWVRQAPGKGLEWVAWISPYGGSTYYADSVKGRFTISADTSK NTAYLQMNSLRAEDTAVYYCARRHWPGGFDYWGOGTLVTVSS | 41 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | S70 | | 42 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B3 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Lin17 | GGGGSEAAAKGGGGS | 85 |
| | | VHb | S70 | | 41 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | S70 | | 42 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B4 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Lin21 | GAAAGGGGSGGGGSGGGGS | 89 |
| | | VHb | 12A4 | QVQLVQSGAEVKKPGSSVKVSCKTSGDTFSTYAISWVRQAPGQGLEWMGGIIPIFGKAHYAQKFQGRVTITADESTST AYMELSSLRSEDTAVYFCARKFHFVSGSPFGMDVWGQGTTVTVSS | 45 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B5 | Fusion Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | | Linker 1 | Lin17 | GGGGSEAAAKGGGGS | 85 |
| | | VHb | 12A4 | QVQLVQSGAEVKKPGSSVKVSCKTSGDTFSTYAISWVRQAPGQGLEWMGGIIPIFGKAHYAQKFQGRVTITADESTST AYMELSSLRSEDTAVYFCARKFHFVSGSPFGMDVWGQGTTVTVSS | 45 |
| | Fusion Heavy Chain | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK [?]HRGGGGS | 46 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B6 | Fusion Light Chain | VLa | S70 | | 42 |
| | | CL | Lc1 | | 104 |
| | | Linker | 1 Lin21 | GAAAGGGGSGGGGSGGGGS | 89 |
| | | VHb | G631 | [?] EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker | 2 Lin15 | GAAAGGGGSGGGGS | 83 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker | 3 Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Fusion Light Chain | VLa | S70 | | 42 |
| | | CL | Lc1 | | 104 |
| | | Linker | 1 Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| Y100-B7 | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker | 2 Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | 3 Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B8 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin21 | GAAAGGGGSGGGGSGGGGS | 89 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | 12A4 | QVQLVQSGAEVKKPGSSVKVSCKTSGDTFS**TYAIS**WVRQAPGQGLEWMGGI**IPIFGKAHYAQKFQG**RVTITADESTS TAYMELSSLRSEDTAVYFCAR**KFHFVSGSPFGMDV**WGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | [?] PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B9 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B10 | Fusion Light Chain | VLa | S70 | | 42 |
| | | CL | Lc1 | | 104 |
| | | Linker 1 | Lin17 | GGGGSEAAAKGGGGS | 85 |
| | | VHb | Avastin | | 53 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | Avastin | | 54 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B11 | Fusion Light Chain | VLa | S70 | | 42 |
| | | CL | Lc1 | | 104 |
| | | Linker 1 | Lin16 | EAAAKGGGGSGGGGS | 84 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin16 | EAAAKGGGGSGGGGS | 84 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B12 | Fusion Light Chain | VLa | S70 | | 42 |
| | | CL | Lc1 | | 104 |
| | | Linker 1 | Lin18 | EAAAKEAAAKGGGGS | 86 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin18 | EAAAKEAAAKGGGGS | 86 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-B13 | Fusion Light Chain | VLa | S70 | | 42 |
| | | CL | Lc1 | | 104 |
| | | Linker 1 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ | 111 |
| | Chain | Linker 2 | Lin17 | TYICNVNHKPSNTKVDKKVEPKSC GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-BC1 | Fusion Light Chain | VLa | S70 | | 42 |
| | | CL | Lc1 | | 104 |
| | | Linker 1 | Lin17 | GGGGSEAAAKGGGGS | 85 |
| | | VHb | 4420 | EVKLDETGGGLVQPGRPMKLSCVASGFTFSDYWMNWVRQSPEKGLEWVAQIRNKPYNYETYYSDSVKGRFTISRD DSKSSVYLQMNNLRVEDMGIYYCTGSYYGMDYWGQGTSVTVSS | 67 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 4420 | DVVMTQTPLSLPVSLGDQASISCRSSQSLVHSNGNTYLRWYLQKPGQSPKVLIYKVSNRFSGVPDRFSGSGSGTDFTL KISRVEAEDLGVYFCSQSTHVPWTFGGGTKLEIK | 68 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-BC2 | Fusion Light Chain | VLa | 4420 | DVVMTQTPLSLPVSLGDQASISCRSSQSLVHSNGNTYLRWYLQKPGQSPKVLIYKVSNRFSGVPDRFSGSGSGTDFTL KISRVEAEDLGVYFCSQSTHVPWTFGGGTKLEIK | 68 |
| | | CL | Lc1 | | 104 |
| | | Linker 1 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLOMNSLRAEDTAVYYCARFVFFLPYAMDYWGQGTLVTVSS | 57 |
| | Fusion Heavy Chain | VHa | 4420 | EVKLDETGGGLVQPGRPMKLSCVASGFTFSDYWMNWVRQSPEKGLEWVAQIRNKPYNYETYYSDSVKGRFTISRD DSKSSVYLQMNNLRVEDMGIYYCTGSYYGMDYWGQGTSVTVSS | 67 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y100-BC3 | Fusion Light Chain | VLa | 4420 | DVVMTQTPLSLPVSLGDQASISCRSSQSLVHSNGNTYLRWYLQKPGQSPKVLIYKVSNRFSGVPDRFSGSGSGTDFTL KISRVEAEDLGVYFCSQSTHVPWTFGGGTKLEIK | 68 |
| | | CL | Lc1 | | 104 |
| | | Linker 1 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | 4420 | EVKLDETGGGLVQPGRPMKLSCVASGFTFSDYWMNWVRQSPEKGLEWVAQIRNKPYNYETYYSDSVKGRFTISRD DSKSSVYLQMNNLRVEDMGIYYCTGSYYGMDYWGQGTSVTVSS | 67 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-B1 | Fusion Light Chain | VLa | S70 | | 42 |
| | | CL | Lc1 | | 104 |
| | | Linker 1 | Lin21 | GAAAGGGGSGGGGSGGGGS | 89 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-B2 | Fusion Light Chain | VLa | S70 | | 42 |
| | | CL | Lc1 | | 104 |
| | | Linker 1 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-B3 | Fusion Light | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | Chain | | | | |
| | | Linker 1 | Lin21 | GAAAGGGGSGGGGSGGGGS | 89 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-B4 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge CH2 | Hin1 G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK DKTHTCP | 91 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-B5 | Fusion Light Chain | VLa | S70 | | 42 |
| | | CL | Lc1 | | 104 |
| | | Linker 1 | Lin16 | EAAAKGGGGSGGGGS | 84 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin16 | EAAAKGGGGSGGGGS | 84 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y101-B6 | Fusion Light Chain | VLa | S70 | | 42 |
| | | CL | Lc1 | | 104 |
| | | Linker 1 | Lin18 | EAAAKEAAAKGGGGS | 86 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin18 | EAAAKEAAAKGGGGS | 86 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y103-B1 | Fusion Light Chain | VLa | S70 | | 42 |
| | | CL | Lc1 | | 104 |
| | | Linker 1 | Lin21 | GAAAGGGGSGGGGSGGGGS | 89 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y103-B2 | Fusion Light Chain | VLa | S70 | | 42 |
| | | CL | Lc1 | | 104 |
| | | Linker 1 | Lin18 | EAAAKEAAAKGGGGS | 86 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin18 | EAAAKEAAAKGGGGS | 86 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y103-B3 | Fusion Light Chain | VLa | S70 | | 42 |
| | | CL | Lc1 | | 104 |
| | | Linker 1 | Lin18 | EAAAKEAAAKGGGGS | 86 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | Fusion Heavy Chain | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin18 | EAAAKEAAAKGGGGS | 86 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y104-B1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y104-B2 | Fusion Light Chain | VLa | 5C4 | | 38 |
| | | CL | Lc1 | RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC GGGGSEAAAKGGGGS | 104 |
| | | Linker 1 | Lin17 | GGGGSEAAAKGGGGS | 85 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y104-B3 | Fusion Light Chain | VLa | 5C4 | | 38 |
| | | CL | Lc1 | | 104 |
| | | Linker 1 | Lin17 | GGGGSEAAAKGGGGS | 85 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y105-B1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y105-B2 | Fusion Light Chain | VLa | 5C4 | | 38 |
| | | CL | Lc1 | | 104 |
| | | Linker 1 | Lin17 | GGGGSEAAAKGGGGS | 85 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | Fusion Heavy | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ | 111 |
| | Chain | | | TYICNVNHKPSNTKVDKKVEPKSC | |
| | | Linker 2 | Lin17 | GGGGSEAAAKGGGGS | 85 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y105-B3 | Fusion Light Chain | VLa | 5C4 | | 38 |
| | | CL | Lc1 | | 104 |
| | | Linker 1 | Lin17 | GGGGSEAAAKGGGGS | 85 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-B1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-B2 | Fusion Light Chain | VLa | 5C4 | | 38 |
| | | CL | Lc1 | | 104 |
| | | Linker 1 | Lin17 | GGGGSEAAAKGGGGS | 85 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 3 | Lin6 | GYPGGGGS | 74 |
| | | Hinge | Hin1 | DKTHTCP | 91 |
| | | CH2 | G2DCH2 | [?] PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-B3 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-B4 | Fusion Light Chain | VLa | 5C4 | | 38 |
| | | CL | Lc1 | | 104 |
| | | Linker 1 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 3G12 | GGGGSEAAAKGGGGS ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y106-B5 | Fusion Light | VLa | 5C4 | | 38 |
| | | CL | Lc1 | | 104 |
| | Chain | | | | |
| | | Linker 1 | Lin17 | GGGGSEAAAKGGGGS | 85 |
| | | VHb | 3G12 | QVQLVQSGAEVKKPGSSVKVSCKASGYTFSSNVISWVRQAPGQGLEWMGGVIPIVDIANYAQRFKGRVTITADESTST TYMELSSLRSEDTAVYYCASTLGLVLDAMDYWGQGTLVTVSS | 59 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLEP EDFAVYYCQQYADSPITFGQGTRLEIK | 60 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-B1 | Fusion Light Chain | VLa | 5C4 | | 38 |
| | | CL | Lc1 | | 104 |
| | | Linker 1 | Lin21 | GAAAGGGGSGGGGSGGGGS | 89 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-B2 | Fusion Light Chain | VLa | 5C4 | | 38 |
| | | CL | Lc1 | | 104 |
| | | Linker 1 | Lin17 | GGGGSEAAAKGGGGS | 85 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | Fusion Heavy Chain | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | VLTVLHQDWLNGKEYKCKVSNKALPAPIEKTISKAK GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-B3 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin21 | GAAAGGGGSGGGGSGGGGS | 89 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | | | | |
| | | Linker 3 Hinge | Lin6 Hin1 | GYPGGGGS DKTHTCP | 74 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-B4 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin17 | GGGGSEAAAKGGGGS | 85 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y110-B5 | Fusion Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | | Linker 1 | Lin17 | GGGGSEAAAKGGGGS | 85 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | Fusion Heavy Chain | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y116-B1 | Fusion Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | | Linker 1 | Lin21 | GAAAGGGGSGGGGSGGGGS | 89 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | Fusion Heavy Chain | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin15 | GAAAGGGGSGGGGS | 83 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Linker 3 | Lin6 | GYPGGGGS | 74 |
| | | Hinge CH2 | Hin1 G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK DKTHTCP | 91 117 |
| | | CH3 | G1CH3 | [?] GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y116-B2 | Fusion Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | | Linker 1 | Lin17 | GGGGSEAAAKGGGGS | 85 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | Fusion Heavy Chain | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Linker 3 | Lin6 | GYPGGGGS | 74 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y116-B3 | Fusion Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | | Linker 1 | Lin17 | GGGGSEAAAKGGGGS | 85 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | Fusion Heavy Chain | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTVPSSSLGTQ TYICNVNHKPSNTKVDKKVEPKSC | 111 |
| | | Linker 2 | Lin17 | GGGGSEAAAKGGGGS | 85 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Linker 3 | Lin6 | GYPGGGGS | 74 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |

**[Table 28] The antibody codes and amino acid sequences of some antibodies with bispecific antibody structure 2 for comparison**

| Anti -body code | Polypeptides | Domain | Code | Amino acid sequences (bold and underlined amino acids are CDR) | SEQ ID NO. |
|---|---|---|---|---|---|
| Y200-A1 | Light Chain | VLa | S70 | | 42 |
| | | CL | Lc1 | | 104 |
| | Fusion | VHa | S70 | EVQLVESGGGLVQPGGSLRLSCAASGFTFSDSWIHWVRQAPGKGLEWVAWISPYGGSTYYADSVKGRFTISADTSK | 41 |
| | Heavy Chain2 | CH1 | G1CH1 | NTAYLQMNSLRAEDTAVYYCARRHWPGGFDYWGQGTLVTVSS ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC DKTHTCP | 111 |
| | | Hinge | Hin1 | | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 5 | Lin14 | GGGGSGGGGSGGGGS | 82 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| Y200-A2 | Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | Fusion Heavy Chain2 | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | Avastin | | 53 |
| | | Linker 5 | Lin14 | GGGGSGGGGSGGGGS | 82 |
| | | VLb | Avastin | | 54 |
| Y200- | Light | VLa | G631 | DIQMTQSPSSLSASVGDRVTITCRASQDVSTAVAWYQQKPGKAPKLLIYSASFLYSGVPSRFSGSGSGTDFTLTISSLQP | 58 |
| A3 | Chain | | | EDFATYYCQQGYGNPFTFGQGTKVEIK | |
| | | CL | Lc1 | | 104 |
| | Fusion Heavy Chain2 | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | S70 | | 41 |
| | | Linker 5 | Lin14 | GGGGSGGGGSGGGGS | 82 |
| | | VLb | S70 | | 42 |
| Y201-A1 | Light Chain | VLa | S70 | | 42 |
| | | CL | Lc1 | | 104 |
| | Fusion Heavy Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | 3G12 | QVQLVQSGAEVKKPGSSVKVSCKASGYTFSSNVISWVRQAPGQGLEWMGGVIPIVDIANYAQRFKGRVTITADESTS TTYMELSSLRSEDTAVYYCASTLGLVLDAMDYWGQGTLVTVSS | 59 |
| | | Linker | Lin14 | GGGGSGGGGSGGGGS | 82 |
| | | 5 VLb | 3G12 | ETVLTQSPGTLSLSPGERATLSCRASQSLGSSYLAWYQQKPGQAPRLLIYGASSRAPGIPDRFSGSGSGTDFTLTISRLE PEDFAVYYCQQYADSPITFGQGTRLEIK | 60 |
| Y201-A2 | Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | Fusion Heavy Chain2 | VHa | 12A4 | QVQLVQSGAEVKKPGSSVKVSCKTSGDTFS**TYAIS**WVRQAPGQGLEWMGGI**IPIFGKAHYAQKFQG**RVTITADESTS TAYMELSSLRSEDTAVYFCAR**KFHFVSGSPFGMDV**WGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | 3G12 | QVQLVQSGAEVKKPGSSVKVSCKASGYTFSSNVISWVRQAPGQGLEWMGGVIPIVDIANYAQRFKGRVTITADESTS TTYMELSSLRSEDTAVYYCASTLGLVLDAMDYWGQGTLVTVSS | 59 |
| | | Linker 5 | Lin14 | GGGGSGGGGSGGGGS | 82 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLE PEDFAVYYCQQYADSPITFGQGTRLEIK | 60 |
| Y203-A1 | Light Chain | VLa | S70 | | 42 |
| | | CL | Lc1 | EDFATYYCQQYETHPTTFGQGTKVEIK RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC | 104 |
| | Fusion Heavy Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | | | | |
| | | Linker 4 | Lin19 | AGGGGSGGGGSGGGGSG | 87 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | | Linker 5 | Lin14 | GGGGSGGGGSGGGGS | 82 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| Y204-A1 | Light Chain | VLa | S70 | | 42 |
| | | CL | Lc1 | | 104 |
| | Fusion Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Heavy Linker | Lin19 | AGGGGSGGGGSGGGGSG | 87 |
| | | 4 VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Linker 5 | Lin14 | GGGGSGGGGSGGGGS | 82 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| Y205-A1 | Light Chain | VLa | S70 | | 42 |
| | | CL | Lc1 | | 104 |
| | Fusion | VHa | S70 | | 41 |
| | Heavy Chain2 | | |  [?] | |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| | | Linker 5 | Lin14 | GGGGSGGGGSGGGGS | 82 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| Y205-A2 | Light Chain | VLa | 5C4 | | 38 |
| | | CL | Lc1 | | 104 |
| | Fusion Heavy Chain2 | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS [?] | 29 |
| | | Linker 5 | Lin14 | GGGGSGGGGSGGGGS | 82 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| Y206-A1 | Light Chain | VLa | 5C4 | | 38 |
| | | CL | Lc1 | | 104 |
| | Fusion Heavy Chain2 | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 5 | Lin14 | GGGGSGGGGSGGGGS | 82 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| Y206-A2 | Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | Fusion Heavy Chain2 | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | 5C4 | | 37 |
| | | Linker 5 | Lin14 | GGGGSGGGGSGGGGS | 82 |
| | | VLb | 5C4 | | 38 |
| Y210-A1 | Light Chain | VLa | 5C4 | | 38 |
| | | CL | Lc1 | [?]QQSSNWPRTFGQGTKVEIK RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC | 104 |
| | Fusion Heavy Chain2 | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | | Linker 5 | Lin14 | GGGGSGGGGSGGGGS | 82 |
| | | VLb | BN-10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| Y216-A1 | Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | Fusion | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | Heavy Chain2 | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Linker 5 | Lin14 | GGGGSGGGGSGGGGS | 82 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| Y200-B1 | Light Chain | VLa | S70 | | 42 |
| | | CL | Lc1 | | 104 |
| | Fusion Heavy Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 5 | Lin14 | GGGGSGGGGSGGGGS | 82 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| Y200- | Light | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| B2 | Chain | CL | Lc1 | | 104 |
| | Fusion Heavy Chain2 | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker | Lin19 | AGGGGSGGGGSGGGGSG | 87 |
| | | 4 VLb | Avastin | | 54 |
| | | Linker 5 | Lin14 | GGGGSGGGGSGGGGS | 82 |
| | | VHb | Avastin | | 53 |
| Y200-B3 | Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | Fusion Heavy Chain2 | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | S70 | | 42 |
| | | Linker 5 | Lin14 | GGGGSGGGGSGGGGS | 82 |
| | | VHb | S70 | | 41 |
| Y201-B1 | Light Chain | VLa | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | Fusion Heavy Chain2 | VHa | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLE PEDFAVYYCQQYADSPITFGQGTRLEIK | 60 |
| | | Linker 5 | Lin14 | GGGGSGGGGSGGGGS | 82 |
| | | VHb | 3G12 | QVQLVQSGAEVKKPGSSVKVSCKASGYTFSSNVISWVRQAPGQGLEWMGGVIPIVDIANYAQRFKGRVTITADESTS TTYMELSSLRSEDTAVYYCASTLGLVLDAMDYWGQGTLVTVSS | 59 |
| Y201-B2 | Light Chain | VLa | S70 | | 42 |
| | | CL | Lc1 | | 104 |
| | Fusion Heavy Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | 3G12 | ETVLTQSPGTLSLSPGERATLSCRASQSLGSSYLAWYQQKPGQAPRLLIYGASSRAPGIPDRFSGSGSGTDFTLTISRLE PEDFAVYYCQQYADSPITFGQGTRLEIK | 60 |
| | | Linker 5 | Lin14 | GGGGSGGGGSGGGGS | 82 |
| | | VHb | 3G12 | QVQLVQSGAEVKKPGSSVKVSCKASGYTFSSNVISWVRQAPGQGLEWMGGVIPIVDIANYAQRFKGRVTITADESTS TTYMELSSLRSEDTAVYYCASTLGLVLDAMDYWGQGTLVTVSS | 59 |
| Y203-B1 | Light Chain | VLa | S70 | | 42 |
| | | CL | Lc1 | | 104 |
| | Fusion Heavy Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK GGGGSGGGGSGGGGS | 34 |
| | | Linker 5 | Lin14 | GGGGSGGGGSGGGGS | 82 |
| | | VHb | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| Y204-B1 | Light Chain | VLa | S70 | | 42 |
| | | CL | Lc1 | | 104 |
| | Fusion | VHa | S70 | | 41 |
| | Heavy Chain2 | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Linker 5 | Lin14 | GGGGSGGGGSGGGGS | 82 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| Y205-B1 | Light Chain | VLa | S70 | | 42 |
| | | CL | Lc1 | EDFATYYCQQYETHRTTFGQGTKVEIK RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC | 104 |
| | Fusion Heavy Chain2 | VHa | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | [?] PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | | Linker 5 | Lin14 | GGGGSGGGGSGGGGS | 82 |
| | | VHb | 10A7 | EVQLVESGGGLTQPGKSLKLSCEASGFTFSSFTMHWVRQSPGKGLEWVAFIRSGSGIVFYADAVRGRFTISRDNAKN | 29 |
| | | | | LLFLQMNDLKSEDTAMYYCARRPLGHNTFDSWGQGTLVTVSS | |
| Y205-B2 | Light Chain | VLa | 5C4 | | 38 |
| | | CL | Lc1 | | 104 |
| | Fusion Chain2 | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Heavy Linker 4 | Lin19 | AGGGGSGGGGSGGGGSG | 87 |
| | | VLb | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | | Linker 5 | Lin14 | GGGGSGGGGSGGGGS | 82 |
| | | VHb | 10A7 | EVQLVESGGGLTQPGKSLKLSCEASGFTFSSFTMHWVRQSPGKGLEWVAFIRSGSGIVFYADAVRGRFTISRDNAKN LLFLQMNDLKSEDTAMYYCARRPLGHNTFDSWGQGTLVTVSS | 29 |
| Y206-B1 | Light Chain | VLa | 5C4 | | 38 |
| | | CL | Lc1 | | 104 |
| | Fusion Heavy Chain2 | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPG AGGGGSGGGGSGGGGSG | 124 |
| | | Linker | Lin19 | AGGGGSGGGGSGGGGSG | 87 |
| | | 4 VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 5 | Lin14 | GGGGSGGGGSGGGGS | 82 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| Y206-B2 | Light Chain | VLa | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | EDFATYYCQQGYGNPFTFGQGTKVEIK RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC | 104 |
| | Fusion Heavy Chain2 | VHa | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | 5C4 | | 38 |
| | | Linker 5 | Lin14 | GGGGSGGGGSGGGGS | 82 |
| | | VHb | 5C4 | | 37 |
| Y210-B1 | Light Chain | VLa | 5C4 | | 38 |
| | | CL | Lc1 | | 104 |
| | Fusion Heavy Chain2 | VHa | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1KCH3 | | 124 |
| | | Linker 4 | Lin19 | AGGGGSGGGGSGGGGSG | 87 |
| | | VLb | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | Linker 5 | Lin14 | GGGGSGGGGSGGGGS | 82 |
| | | VHb | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| Y216-B1 | Light Chain | VLa | Elotuzumab | DIQMTQSPSSLSASVGDRVTITCKASQDVGIAVAWYQQKPGKVPKLLIYWASTRHTGVPDRFSGSGSGTDFTLTISSL QPEDVATYYCQQYSSYPYTFGQGTKVEIK | 50 |
| | | CL | Lc1 | | 104 |
| | Fusion Chain2 | VHa | Elotuzumab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 Heavy | G1KCH3 | | 124 |
| | | Linker 4 | Lin19 | AGGGGSGGGGSGGGGSG | 87 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Linker | Lin14 | GGGGSGGGGSGGGGS | 82 |
| | | 5 VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |

**[Table 29] The antibody codes and amino acid sequences of some antibodies with bispecific antibody structure 3 for comparison**

| Antibody code | Polypeptides | Domain | Code | Amino acid sequences (bold and underlined amino acids are CDR) | SE Q ID NO. |
|---|---|---|---|---|---|
| Y300-A1 | Fusion Peptide | VHa | S70 | | 41 |
| | | Linker 6 | Lin14 | GGGGSGGGGSGGGGS | 82 |
| | | VLa | S70 | | 42 |
| | | Linker 7 | Lin5 | GGGGSAS | 73 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 8 | Lin14 | GGGGSGGGGSGGGGS | 82 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK DKTHTCP | 58 |
| | | Hinge | Hin1 | EDFATYYCQQGYGNPFT FGQGTKVEIK DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y304-A1 | Fusion Peptide | VHa | 5C4 | | 37 |
| | | Linker 6 | Lin14 | GGGGSGGGGSGGGGS | 82 |
| | | VLa | 5C4 | | 38 |
| | | Linker 7 | Lin5 | GGGGSAS | 73 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Linker 8 | Lin14 | GGGGSGGGGSGGGGS | 82 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | TVVHQDWLNGKEYKCKVSNKGLPAPIEKTISKTK GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y316-A1 | Fusion Peptide | VHa | Elotuzu mab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | Linker 6 | Lin14 | GGGGSGGGGSGGGGS | 82 |
| | | VLa | Elotuzu mab | DIQMTQSPSSLSASVGDRVTITCKASQDVGIAVAWYQQKPGKVPKLLIYWASTRHTGVPDRFSGSGSGTDFTLTISSLQ PEDVATYYCQQYSSYPYTFGQGTKVEIK | 50 |
| | | Linker 7 | Lin5 | GGGGSAS | 73 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Linker 8 | Lin14 | GGGGSGGGGSGGGGS | 82 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y300-B1 | Fusion Peptide | VHa | S70 | | 41 |
| | | Linker 6 | Lin14 | GGGGSGGGGSGGGGS | 82 |
| | | VLa | S70 | | 42 |
| | | Linker 7 | Lin5 | GGGGSAS | 73 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK GGGGSGGGGSGGGGS | 58 |
| | | Linker 8 | Lin14 | GGGGSGGGGSGGGGS | 82 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 120 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | |
| Y304-B1 | Fusion Peptide | VHa | 5C4 | | 37 |
| | | Linker 6 | Lin14 | GGGGSGGGGSGGGGS | 82 |
| | | VLa | 5C4 | | 38 |
| | | Linker 7 | Lin5 | GGGGSAS | 73 |
| | | VLb | Yervoy | GGGGSAS EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Linker 8 | Lin14 | GGGGSGGGGSGGGGS | 82 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y316-B1 | Fusion Peptide | VHa | Elotuzu mab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | Linker 6 | Lin14 | GGGGSGGGGSGGGGS | 82 |
| | | VLa | Elotuzu mab | DIQMTQSPSSLSASVGDRVTITCKASQDVGIAVAWYQQKPGKVPKLLIYWASTRHTGVPDRFSGSGSGTDFTLTISSLQ PEDVATYYCQQYSSYPYTFGQGTKVEIK | 50 |
| | | Linker 7 | Lin5 | GGGGSAS | 73 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Linker 8 | Lin14 | GGGGSGGGGSGGGGS | 82 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y300-C1 | Fusion Peptide | VLa | S70 | DIQMTQSPSSLSASVGDRVTITCRASQDVSTAVAWYQQKPGKAPKLLIYSASFLYSGVPSRFSGSGSGTDFTLTISSLQP EDFATYYCQQYLYHPATFGQGTKVEIK GGGGSGGGGSGGGGS | 42 |
| | | Linker 6 | Lin14 | GGGGSGGGGSGGGGS | 82 |
| | | VHa | S70 | | 41 |
| | | Linker 7 | Lin5 | GGGGSAS | 73 |
| | | VHb | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Linker 8 | Lin14 | GGGGSGGGGSGGGGS | 82 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y304-C1 | Fusion Peptide | VLa | 5C4 | | 38 |
| | | Linker 6 | Lin14 | GGGGSGGGGSGGGGS | 82 |
| | | VHa | 5C4 | | 37 |
| | | Linker 7 | Lin5 | GGGGSAS | 73 |
| | | VHb | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS [?] | 27 |
| | | Linker 8 | Lin14 | GGGGSGGGGSGGGGS | 82 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y316-C1 | Fusion Peptide | VLa | Elotuzu mab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | Linker 6 | Lin14 | GGGGSGGGGSGGGGS | 82 |
| | | VHa | Elotuzu mab | DIQMTQSPSSLSASVGDRVTITCKASQDVGIAVAWYQQKPGKVPKLLIYWASTRHTGVPDRFSGSGSGTDFTLTISSLQ PEDVATYYCQQYSSYPYTFGQGTKVEIK | 50 |
| | | Linker 7 | Lin5 | GGGGSAS | 73 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Linker 8 | Lin14 | GGGGSGGGGSGGGGS | 82 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y300-D1 | Fusion Peptide | VLa | S70 | | 42 |
| | | Linker 6 | Lin14 | GGGGSGGGGSGGGGS | 82 |
| | | VHa | S70 | | 41 |
| | | Linker 7 | Lin5 | GGGGSAS | 73 |
| | | VLb | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | Linker 8 | Lin14 | GGGGSGGGGSGGGGS | 82 |
| | | VHb | G631 | GGGGSGGGGSGGGGS EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y304-D1 | Fusion Peptide | VLa | 5C4 | | 38 |
| | | Linker 6 | Lin14 | GGGGSGGGGSGGGGS | 82 |
| | | VHa | 5C4 | | 37 |
| | | Linker 7 | Lin5 | GGGGSAS | 73 |
| | | VLb | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | Linker 8 | Lin14 | GGGGSGGGGSGGGGS | 82 |
| | | VHb | Yervoy | [?] QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Y316-D2 | Fusion Peptide | VLa | Elotuzu mab | DIQMTQSPSSLSASVGDRVTITCKASQDVGIAVAWYQQKPGKVPKLLIYWASTRHTGVPDRFSGSGSGTDFTLTISSLQ PEDVATYYCQQYSSYPYTFGQGTKVEIK | 50 |
| | | Linker 6 | Lin14 | GGGGSGGGGSGGGGS | 82 |
| | | VHa | Elotuzu mab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | Linker 7 | Lin5 | GGGGSAS | 73 |
| | | VLb | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | Linker 8 | Lin14 | GGGGSGGGGSGGGGS | 82 |
| | | VHb | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNSTFRVVSVL TVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |

**[Table 30] The antibody codes and amino acid sequences of some antibodies with bispecific antibody structure 4 for comparison**

| Antibody code | Polypeptide | Domain | Code | Amino acid sequences (bold and underlined amino acids are CDR) | SE Q ID NO. |
|---|---|---|---|---|---|
| Y400-A1 | Fusion Light Chain | VLb | S70 | | 42 |
| | | Linker 10 | Lin14 | GGGGSGGGGSGGGGS | 82 |
| | | VLa | G631 | DIQMTQSPSSLSASVGDRVTITCRASQDVSTAVAWYQQKPGKAPKLLIYSASFLYSGVPSRFSGSGSGTDFTLTISSLQPE DFATYYCQQGYGNPFTFGQGTKVEIK | 58 |
| | | CL | Lc1 | | 104 |
| | Fusion Heavy | VHb | S70 | | 41 |
| | Chain | Linker 9 | Lin14 | GGGGSGGGGSGGGGS | 82 |
| | | VHa | G631 | EVQLVESGGGLVQPGGSLRLSCAASGFTISDYWIHWVRQAPGKGLEWVAGITPAGGYTYYADSVKGRFTISADTSKN TAYLQMNSLRAEDTAVYYCARFVFFLPYAMDYWGQGTLVTVSS | 57 |
| | | CH1 | G1C H1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G2D CH2 | PCPAPPVAGPSVFLFPPKPKDTLMISRTPEVTCVVVDVSHEAPEVQFNWYVDGVEVHNAKTKPREEQFNSTFRVVSVLT VVHQDWLNGKEYKCKVSNKGLPAPIEKTISKTK | 117 |
| | | CH3 | G1C H3 | GQPREPQVYTLPPSRDEL TKNQVSL TCL VKGFYPSDIA VEWESNGQPENNYKTTPPVLDSDGSFFL YSKL TVDKSRWQQ GNVFSCSVMHEALHNHYTQKSLSLSPGK | 120 |
| | Fusion Light Chain | VLb | 5C4 | | 38 |
| | | Linker 10 | Lin14 | GGGGSGGGGSGGGGS | 82 |
| | | VLa | Yervo y | EIVLTQSPGTLSLSPGERATLSCRASQSVGSSYLAWYQQKPGQAPRLLIYGAFSRATGIPDRFSGSGSGTDFTLTISRLEPE DFAVYYCQQYGSSPWTFGQGTKVEIK | 28 |
| | | CL | Lc1 | | 104 |
| Y404-A1 | Fusion Heavy Chain | VHb | 5C4 | | 37 |
| | | Linker 9 | Lin14 | GGGGSGGGGSGGGGS | 82 |
| | | VHa | Yervo y | QVQLVESGGGVVQPGRSLRLSCAASGFTFSSYTMHWVRQAPGKGLEWVTFISYDGNNKYYADSVKGRFTISRDNSKN TLYLQMNSLRAEDTAIYYCARTGWLGPFDYWGQGTLVTVSS | 27 |
| | | CH1 | G1C H1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G2D CH2 | PCPAPPVAGPSVFLFPPKPKDTLMISRTPEVTCVVVDVSHEAPEVQFNWYVDGVEVHNAKTKPREEQFNSTFRVVSVLT VVHQDWLNGKEYKCKVSNKGLPAPIEKTISKTK | 117 |
| | | CH3 | G1C H3 | GQPREPQVYTLPPSRDELTKNQVSLTCLVKGFYPSDIAVEWESNGQPENNYKTTPPVLDSDGSFFLYSKLTVDKSRWQQ GNVFSCSVMHEALHNHYTQKSLSLSPGK | 120 |
| Y416-A1 | Fusion Light Chain | VLb | Elotuz umab | DIQMTQSPSSLSASVGDRVTITCKASQDVGIAVAWYQQKPGKVPKLLIYWASTRHTGVPDRFSGSGSGTDFTLTISSLQP EDVATYYCQQYSSYPYTFGQGTKVEIK | 50 |
| | | Linker | Lin14 | GGGGSGGGGSGGGGS | 82 |
| | | 10 VLa CL | NM3E | SELTQDPAVSVALGQTVRITCQGDSLRSYYASWYQQKPGQAPVLVIYGKNNRPSGIPDRFSGSSSGNTASLTITGAQAE DEADYYCNSRDSSGNHVVFGGGTKLTVL | 48 |
| | | | 2 Lc1 | | 104 |
| | Fusion Heavy Chain | VHb | Elotuz umab | EVQLVESGGGLVQPGGSLRLSCAASGFDFSRYWMSWVRQAPGKGLEWIGEINPDSSTINYAPSLKDKFIISRDNAKNSL YLQMNSLRAEDTAVYYCARPDGNYWYFDVWGQGTLVTVSS | 49 |
| | | Linker | Lin14 | GGGGSGGGGSGGGGS | 82 |
| | | 9 VHa | NM3E 2 | EVQLVESGGGVVRPGGSLRLSCAASGFTFDDYGMSWVRQAPGKGLEWVSGINWNGGSTGYADSVKGRFTISRDNAK NSLYLQMNSLRAEDTAVYYCARGRSLLFDYWGQGTLVTVSR | 47 |
| | | CH1 | G1C H1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G2D CH2 | PCPAPPVAGPSVFLFPPKPKDTLMISRTPEVTCVVVDVSHEAPEVQFNWYVDGVEVHNAKTKPREEQFNSTFRVVSVLT VVHQDWLNGKEYKCKVSNKGLPAPIEKTISKTK | 117 |
| | | CH3 | G1C H3 | GQPREPQVYTLPPSRDELTKNQVSLTCLVKGFYPSDIAVEWESNGQPENNYKTTPPVLDSDGSFFLYSKLTVDKSRWQQ GNVFSCSVMHEALHNHYTQKSLSLSPGK | 120 |
| | Fusion Light Chain | VHb | S70 | | 41 |
| | | Linker 10 | Lin14 | GGGGSGGGGSGGGGS | 82 |
| | | VLa | G631 | DIQMTQSPSSLSASVGDRVTITCRASQDVSTAVAWYQQKPGKAPKLLIYSASFLYSGVPSRFSGSGSGTDFTLTISSLQPE DFATYYCQQGYGNPFTFGQGTKVEIK | 58 |
| | | CL | Lc1 | | 104 |
| Y400-B1 | Fusion Heavy Chain | VLb | S70 | | 42 |
| | | Linker 9 | Lin14 | GGGGSGGGGSGGGGS | 82 |
| | | VHa | G631 | EVQLVESGGGLVQPGGSLRLSCAASGFTISDYWIHWVRQAPGKGLEWVAGITPAGGYTYYADSVKGRFTISADTSKN TAYLQMNSLRAEDTAVYYCARFVFFLPYAMDYWGQGTLVTVSS | 57 |
| | | CH1 | G1C H1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G2D CH2 | PCPAPPVAGPSVFLFPPKPKDTLMISRTPEVTCVVVDVSHEAPEVQFNWYVDGVEVHNAKTKPREEQFNSTFRVVSVLT VVHQDWLNGKEYKCKVSNKGLPAPIEKTISKTK | 117 |
| | | CH3 | G1C H3 | GQPREPQVYTLPPSRDELTKNQVSLTCLVKGFYPSDIAVEWESNGQPENNYKTTPPVLDSDGSFFLYSKLTVDKSRWQQ GNVFSCSVMHEALHNHYTQKSLSLSPGK | 120 |
| | Fusion Light Chain | VHb | 5C4 | | 37 |
| | | Linker 10 | Lin14 | GGGGSGGGGSGGGGS | 82 |
| | | VLa | Yervo y | EIVLTQSPGTLSLSPGERATLSCRASQSVGSSYLAWYQQKPGQAPRLLIYGAFSRATGIPDRFSGSGSGTDFTLTISRLEPE DFAVYYCQQYGSSPWTFGQGTKVEIK | 28 |
| | | CL | Lc1 | | 104 |
| Y404-B1 | Fusion Heavy Fusion Heavy Chain | VLb | 5C4 | | 38 |
| | | Linker 9 | Lin14 | GGGGSGGGGSGGGGS | 82 |
| | | VHa | Yervo y | QVQLVESGGGVVQPGRSLRLSCAASGFTFSSYTMHWVRQAPGKGLEWVTFISYDGNNKYYADSVKGRFTISRDNSKN TLYLQMNSLRAEDTAIYYCARTGWLGPFDYWGQGTLVTVSS | 27 |
| | | CH1 | G1C H1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G2D CH2 | PCPAPPVAGPSVFLFPPKPKDTLMISRTPEVTCVVVDVSHEAPEVQFNWYVDGVEVHNAKTKPREEQFNSTFRVVSVLT VVHQDWLNGKEYKCKVSNKGLPAPIEKTISKTK | 117 |
| | | CH3 | G1C H3 | GQPREPQVYTLPPSRDELTKNQVSLTCLVKGFYPSDIAVEWESNGQPENNYKTTPPVLDSDGSFFLYSKLTVDKSRWQQ GNVFSCSVMHEALHNHYTQKSLSLSPGK | 120 |
| Y416-B1 | Fusion Light Chain | VHb | Elotuz umab | EVQLVESGGGLVQPGGSLRLSCAASGFDFSRYWMSWVRQAPGKGLEWIGEINPDSSTINYAPSLKDKFIISRDNAKNSL YLQMNSLRAEDTAVYYCARPDGNYWYFDVWGQGTLVTVSS | 49 |
| | | Linker 10 | Lin14 | GGGGSGGGGSGGGGS | 82 |
| | | VLa | NM3E 2 | SELTQDPAVSVALGQTVRITCQGDSLRSYYASWYQQKPGQAPVLVIYGKNNRPSGIPDRFSGSSSGNTASLTITGAQAE DEADYYCNSRDSSGNHVVFGGGTKLTVL | 48 |
| | | CL | Lc1 | | 104 |
| | Fusion | VLb | Elotuz umab | DIQMTQSPSSLSASVGDRVTITCKASQDVGIAVAWYQQKPGKVPKLLIYWASTRHTGVPDRFSGSGSGTDFTLTISSLQP EDVATYYCQQYSSYPYTFGQGTKVEIK | 50 |
| | Heavy Chain | Linker 9 | Lin14 | GGGGSGGGGSGGGGS | 82 |
| | | VHa | NM3E 2 | EVQLVESGGGVVRPGGSLRLSCAASGFTFDDYGMSWVRQAPGKGLEWVSGINWNGGSTGYADSVKGRFTISRDNAK NSLYLQMNSLRAEDTAVYYCARGRSLLFDYWGQGTLVTVSR | 47 |
| | | CH1 | G1C H1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G2D CH2 | PCPAPPVAGPSVFLFPPKPKDTLMISRTPEVTCVVVDVSHEAPEVQFNWYVDGVEVHNAKTKPREEQFNSTFRVVSVLT VVHQDWLNGKEYKCKVSNKGLPAPIEKTISKTK | 117 |
| | | CH3 | G1C H3 | GQPREPQVYTLPPSRDELTKNQVSLTCLVKGFYPSDIAVEWESNGQPENNYKTTPPVLDSDGSFFLYSKLTVDKSRWQQ GNVFSCSVMHEALHNHYTQKSLSLSPGK | 120 |

**[Table 31] Antibody codes and amino acid sequences of monoclonal antibodies**

| Anti -body code | Polypeptides | Domain | Code | Amino acid sequences (bold and underlined amino acids are CDR) | SE Q ID NO. |
|---|---|---|---|---|---|
| | Light Chain | VL | S70 | | 42 |
| | | CL | Lc1 | EDFATYYCQQYETHPTTFGQGTKVEIK RTVAAPSVFIFPPSDEQLKSGTASVVCLLNNFYPREAKVQWKVDNALQSGNSQESVTEQDSKDSTYSLSSTLTLSKAD YEKHKVYACEVTHQGLSSPVTKSFNRGEC | 104 |
| S70mAb | Heavy Chain | VH | S70 | | 41 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| 12A4mA b | Light Chain | VL | 12A4 | EIVLTQSPATLSLSPGERATLSCRASQSVSSYLAWYQQKPGQAPRLLIYDASNRATGIPARFSGSGSGTDFTLTISSLEPE DFAVYYCQQRSNWPTFGQGTKVEIK | 46 |
| | | CL | Lc1 | | 104 |
| | Heavy Chain | VH | 12A4 | QVQLVQSGAEVKKPGSSVKVSCKTSGDTFSTYAISWVRQAPGQGLEWMGGIIPIFGKAHYAQKFQGRVTITADESTS TAYMELSSLRSEDTAVYFCARKFHFVSGSPFGMDVWGQGTTVTVSS | 45 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| | Light Chain | VL | 3G12 | ETVLTQSPGTLSLSPGERATLSCRASQSLGSSYLAWYQQKPGQAPRLLIYGASSRAPGIPDRFSGSGSGTDFTLTISRLE PEDFAVYYCQQYADSPITFGQGTRLEIK | 60 |
| | | CL | Lc1 | | 104 |
| 3G12mA b | Heavy Chain | VH | 3G12 | QVQLVQSGAEVKKPGSSVKVSCKASGYTFSSNVISWVRQAPGQGLEWMGGVIPIVDIANYAQRFKGRVTITADESTS TTYMELSSLRSEDTAVYYCASTLGLVLDAMDYWGQGTLVTVSS | 59 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 91 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| TAG35m Ab | Light Chain | VL | LAG35 | EIVLTQSPATLSLSPGERATLSCRASQSISSYLAWYQQKPGQAPRLLIYDASNRATGIPARFSGSGSGTDFTLTISSLEPE DFAVYYCQQRSNWPLTFGQGTNLEIK | 34 |
| | | CL | Lc1 | | 104 |
| | Heavy Chain | VH | LAG35 | QVQLQQWGAGLLKPSETLSLTCAVYGGSFSDYYWNWIRQPPGKGLEWIGEINHRGSTNSNPSLKSRVTLSLDTSKNQ FSLKLRSVTAADTAVYYCAFGYSDYEYNWFDPWGQGTLVTVSS | 33 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| 5C4mAb | Light Chain | VL | 5C4 | | 38 |
| | | CL | Lc1 | | 104 |
| | Heavy Chain | VH | 5C4 | | 37 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | [?]T[?]T[?]T[?] PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| 10A7mA b | Light Chain | VL | 10A7 | DIVMTQSPSSLAVSPGEKVTMTCKSSQSLYYSGVKENLLAWYQQKPGQSPKLLIYYASIRFTGVPDRFTGSGSGTDY TLTITSVQAEDMGQYFCQQGINNPLTFGDGTKLEIK | 30 |
| | | CL | Lc1 | | 104 |
| | Heavy Chain | VH | | | |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| BN10mA b | Light Chain | VL | B-N10 | DVLMTQTPLSLPVSLGDQASISCRSSQNIVHSNGNTYLEWYLQKPGQSPKLLIYKVSNRFSGVPDRFSGSGSGTDFTL KITRLEAEDLGVYYCFQGSHVPWTFGGGTKLEIK | 64 |
| | | CL | Lc1 | | 104 |
| | Heavy Chain | VH | B-N10 | QVQLKQSGPGLLQPSQSLSISCTVSGFSLATYGVHWVRQSPGKGLEWLGVIWRGGSTDYSAAFMSRLSITKDNSKSQ VFFKMNSLQADDTAIYFCAKQAYGHYMDYWGQGTSVTVSS | 63 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK [?] | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Elotuzum | Light Chain | VL | Elotuzu mab | DIQMTQSPSSLSASVGDRVTITCKASQDVGIAVAWYQQKPGKVPKLLIYWASTRHTGVPDRFSGSGSGTDFTLTISSLQ PEDVATYYCQQYSSYPYTFGQGTKVEIK | 50 |
| Ab | | CL | Lc1 | | 104 |
| | Heavy Chain | VH | Elotuzu mab | EVQLVESGGGLVQPGGSLRLSCAASGFDFSRYWMSWVRQAPGKGLEWIGEINPDSSTINYAPSLKDKFIISRDNAKNS LYLQMNSLRAEDTAVYYCARPDGNYWYFDVWGQGTLVTVSS | 49 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| G631mA b | Light Chain | VL | G631 | DIQMTQSPSSLSASVGDRVTITCRASQDVSTAVAWYQQKPGKAPKLLIYSASFLYSGVPSRFSGSGSGTDFTLTISSLQP EDFATYYCQQGYGNPFTFGQGTKVEIK | 58 |
| | | CL | Lc1 | | 104 |
| | Heavy Chain | VH | G631 | EVQLVESGGGLVQPGGSLRLSCAASGFTISDYWIHWVRQAPGKGLEWVAGITPAGGYTYYADSVKGRFTISADTSK NTAYLQMNSLRAEDTAVYYCARFVFFLPYAMDYWGQGTLVTVSS | 57 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| Yervoym Ab | Light Chain | VL | Yervoy | EIVLTQSPGTLSLSPGERATLSCRASQSVGSSYLAWYQQKPGQAPRLLIYGAFSRATGIPDRFSGSGSGTDFTLTISRLE PEDFAVYYCQQYGSSPWTFGQGTKVEIK | 28 |
| | | CL | Lc1 | | 104 |
| | Heavy Chain | VH | Yervoy | QVQLVESGGGVVQPGRSLRLSCAASGFTFSSYTMHWVRQAPGKGLEWVTFISYDGNNKYYADSVKGRFTISRDNSK NTLYLQMNSLRAEDTAIYYCARTGWLGPFDYWGQGTLVTVSS | 27 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |
| NM3E2 mAb | Light Chain | VL | NM3E2 | SELTQDPAVSVALGQTVRITCQGDSLRSYYASWYQQKPGQAPVLVIYGKNNRPSGIPDRFSGSSSGNTASLTITGAQA EDEADYYCNSRDSSGNHVVFGGGTKLTVL | 48 |
| | | CL | Lc1 | | 104 |
| | Heavy Chain | VH | NM3E2 | EVQLVESGGGVVRPGGSLRLSCAASGFTFDDYGMSWVRQAPGKGLEWVSGINWNGGSTGYADSVKGRFTISRDNA KNSLYLQMNSLRAEDTAVYYCARGRSLLFDYWGQGTLVTVSR | 47 |
| | | CH1 | G1CH1 | ASTKGPSVFPLAPSSKSTSGGTAALGCLVKDYFPEPVTVSWNSGALTSGVHTFPAVLQSSGLYSLSSVVTV PSSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNSTYRVVSV LTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTVDKSRWQ QGNVFSCSVMHEALHNHYTQKSLSLSPGK | 120 |

The expression levels of some antibodies with bispecific antibody structure 1 of the present disclosure are shown in Figures 5A-D. Figure 5A shows the transient transfection expression levels of Y100-A series and Y100-B series antibodies in CHO cells. Figure 5B shows the transient transfection expression levels of Y101/Y103/Y104/Y105-A series and Y101/Y103/Y104/Y105-B series antibodies in CHO cells. Figure 5C shows the transient transfection expression levels of Y106/Y110/Y116-A series and Y106/Y110/Y116-B series antibodies in CHO cells. Figure 5D shows the purity detected by HPLC-SEC after proteinA affinity chromatography for Y100/Y101/Y103/Y104/Y105/Y106/Y110/Y116-A and B series antibodies that are expressed at a level of greater than 5 mg/L.

Based on Figures 5A-C, it can be seen that the transient transfection expression level of the two series of bispecific antibody structure 1 (that is, A series and B series) are significantly different, wherein the expression level of the A series rarely exceeds 10 mg/L, and the expression level of the B series is higher. In addition, it can be seen from Figure 5D that the purity of the B series of the bispecific antibody structure 1 is more than 70%, and the purity of the A series of the bispecific antibody structure 1 is substantially the same as that of the B series. The purity of antibodies whose expression levels are less than 5 mg/L is not detected.

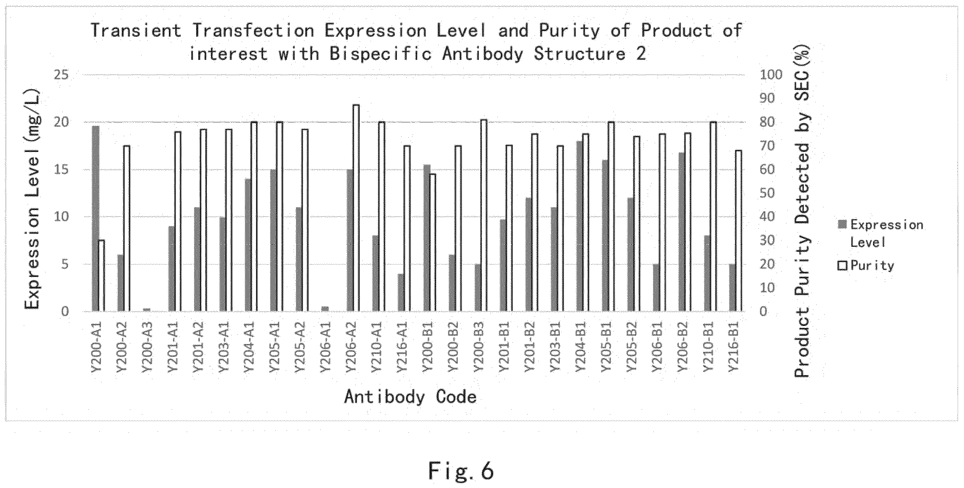

Figure 6 shows the expression level of some antibodies with bispecific antibody structure 2 for comparison. Specially, Figure 6 shows the transient transfection expression levels of Y200/Y201/Y203/Y204/Y205/Y206/Y210/Y216-A series and B series antibodies in CHO cells, and the purity detected by HPLC-SEC after proteinA affinity chromatography.

It can be seen from Figure 6 that the two series of bispecific antibody structure 2 (that is, A series and B series) have no significant difference in transient expression level, and the expression level does not exceed 20mg/L. In addition, the purity of the bispecific antibody structure 2 is detected by HPLC-SEC after proteinA affinity chromatography, and the result shows that the purity of different molecules varies greatly, ranging from 30% to 90%. The expression level of some antibodies is too low to detect the purity.

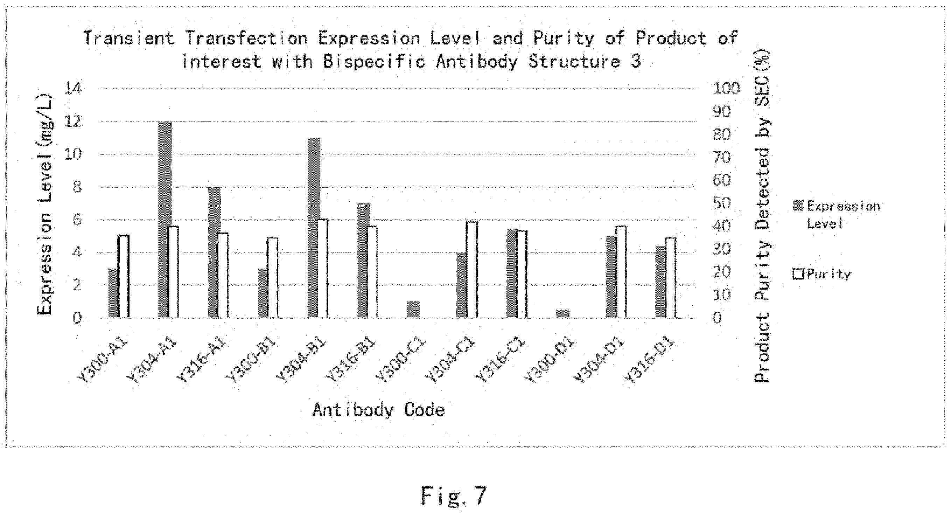

Figure 7 shows the expression level of some antibodies with bispecific antibody structure 3 for comparison. Specially, Figure 7 shows the transient transfection expression levels of Y300/Y304/Y316-A series, B series, C series and D series antibodies in CHO cells and the purity detected by HPLC-SEC after proteinA affinity chromatography.

It can be seen from Figure 7 that the transient transfection expression levels of the four series of bispecific antibody structure 3 (that is, A series, B series, C series and D series) are not high, and the expression levels do not exceed 12 mg/L. In addition, the purity of bispecific antibody structure 3 is detected by HPLC-SEC after proteinA affinity chromatography, and the result shows that the purity is low and is no more than 50%. The expression level of some antibodies is too low to detect the purity.

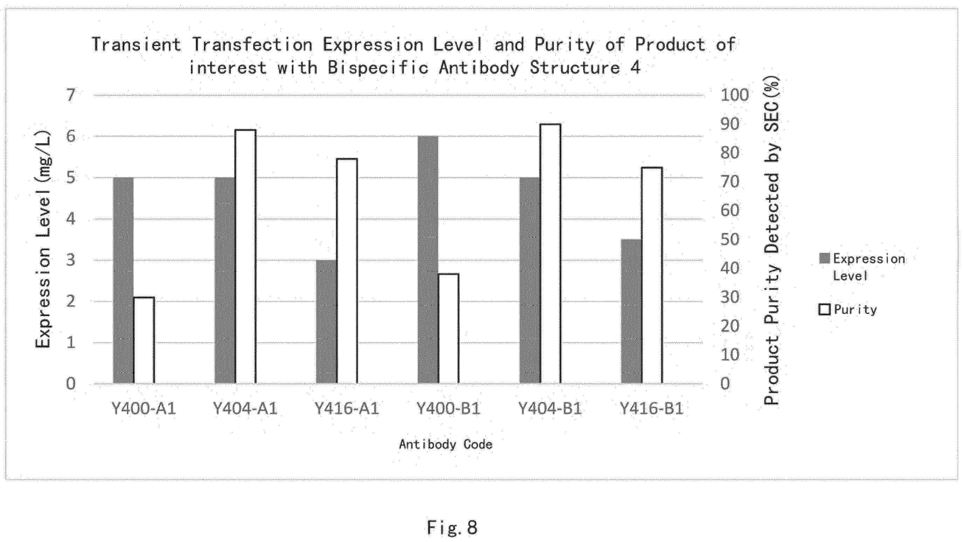

Figure 8 shows the expression level of some antibodies with bispecific antibody structure 4 for comparison. Specially, Figure 8 shows the transient transfection expression level of Y400/Y404/Y416-A series and B series antibodies in CHO cells and the purity detected by HPLC-SEC after proteinA affinity chromatography.

It can be seen from Figure 8 that the expression level of two series of bispecific antibody structure 4 (that is, A series and B series) is not high, and the expression level does not exceed 6mg/L. In addition, the purity of the bispecific antibody structure 4 is detected by HPLC-SEC after proteinA affinity chromatography, and the result shows that the purity varies greatly, ranging from 30% to 90%. The expression level of some antibodies is too low to detect the purity.

The expression level and purity between the antibodies of bispecific antibody structure 1-4 with the same target and antibody variable region sequence are compared, and the results are shown in Figure 9A-I. Figure 9A shows the expression level and purity of the antibodies of bispecific antibody structures 1 to 4 with S70 antibody variable region sequences (SEQ ID NOs: 41 and 42) and G631 antibody variable region sequences (SEQ ID NOs: 57 and 58). Figure 9B shows the expression level and purity of the antibodies of bispecific antibody structures 1 and 2 with S70 antibody variable region sequences (SEQ ID NOs: 41 and 42) and 3G12 antibody variable region sequences (SEQ ID NOs: 59 and 60). Figure 9C shows the expression level and purity of the antibodies of bispecific antibody structures 1 and 2 with 12A4 antibody variable region sequences (SEQ ID NOs: 45 and 46) and 3G12 antibody variable region sequences (SEQ ID NOs: 59 and 60). Figure 9D shows the expression level and purity of the antibodies of bispecific antibody structures 1 and 2 with S70 antibody variable region sequences (SEQ ID NOs: 41 and 42) and LAG35 antibody variable region sequences (SEQ ID NOs: 33 and 34). Figure 9E shows the expression level and purity of the antibodies of bispecific antibody structures 1, 3, and 4 with 5C4 antibody variable region sequences (SEQ ID NOs: 37 and 38) and Yervoy antibody variable region sequences (SEQ ID NOs: 27 and 28). Figure 9F shows the expression level and purity of the antibodies of bispecific antibody structures 1 and 2 with 5C4 antibody variable region sequences (SEQ ID NOs: 37 and 38) and 10A7 antibody variable region sequences (SEQ ID NOs: 29 and 30). Figure 9G shows the expression level and purity of the antibodies of bispecific antibody structures 1 and 2 with 5C4 antibody variable region sequences (SEQ ID NOs: 37 and 38) and G631 antibody variable region sequences (SEQ ID NOs: 57 and 58). Figure 9H shows the expression level and purity of the antibodies of bispecific antibody structures 1 and 2 with 5C4 antibody variable region sequences (SEQ ID NOs: 37 and 38) and B-N10 antibody variable region sequences (SEQ ID NOs: 63 and 64). Figure 9I shows the expression level and purity of the antibodies of bispecific antibody structures 1-4 with Elotuzumab antibody variable region sequences (SEQ ID NOs: 49 and 50) and NM3E2 antibody variable region sequences (SEQ ID NOs: 47 and 48).

It can be seen from Figure 9A that the antibody of bispecific antibody structure 1 has a higher expression level and purity than other structures, wherein Y100-B6 and Y100-B7 have a better expression level and/or purity than antibodies of other structures, and wherein Y100-B7 has the best expression level and purity; Figure 9B shows that the antibodies of bispecific antibody structure 1 have a higher expression level and purity than other structures, wherein Y101-B1 and Y101-B2 have a better expression level and purity than antibodies of other structures, and wherein Y101-B2 has the best expression level and purity; Figure 9C shows that the antibodies of bispecific antibody structure 1 have a higher expression level and/or antibody purity than other structures, wherein Y101-B3 and Y101- B4 have a better expression level and purity than the antibodies of bispecific antibody structure 2, and wherein Y101-B4 has the best expression level and purity; Figure 9D shows that the antibodies of bispecific antibody structure 1 have a better expression level and purity than other structures, wherein Y103-B1 and Y103-B2 have a better expression level and purity than the antibodies of bispecific antibody structure 2, and wherein Y103-B2 has the best expression level and purity; Figure 9E shows that the antibodies of bispecific antibody structure 1 have a better expression level and/or antibody purity than other structures, wherein Y104-B1 and Y104-B2 have a better expression level and purity than the antibodies of bispecific antibody structure 3 and structure 4, and wherein Y104-B2 has the best expression level and purity; Figure 9F shows that the antibodies of bispecific antibody structure 1 have a better expression level and/or antibody purity than other structures, wherein Y105-B1 And Y105-B2 have a better expression level and purity than the antibodies of bispecific antibody structure 2, and wherein Y105-B2 has the best expression level and purity; it can be seen from Figure 9G that the antibodies of bispecific antibody structure 1 have a better expression level and antibody purity than other structures, wherein Y106-B1 and Y106-B2 have a better expression level and/or purity than the antibodies of bispecific antibody structure 2, and wherein Y106-B2 has the best expression level and purity; it can be seen from Figure 9H that the antibodies of bispecific antibody structure 1 have a higher expression level and purity than other structures, wherein Y110-B1 and Y110-B2 have a better expression level and purity than antibodies of bispecific antibody structure 2, and wherein Y110-B2 has the best expression level and purity; Figure 9I shows that the antibodies of bispecific antibody structure 1 have a higher expression level and/or antibody purity than other structures, wherein Y116-B1 and Y116-B2 have a better expression level and purity than antibodies of other bispecific antibody structures, and wherein Y116-B2 has the best expression level and purity.

If the hinge region, CH2 domain and CH3 domain of each of the above antibodies were substituted with corresponding sequences of IgG2 (GenBank accession number MH025834.2), IgG3 (GenBank accession number AJ390278) or IgG4 (GenBank accession number KJ901516) and the sequence of antibody variable region and the linker were remained the same to obtain a substituted antibody, there was no significant difference in the expression level and purity between the resultant substituted antibody and the original antibody. Based on the above data, it can be seen that bispecific antibody structure 1 has significant advantages over other bispecific antibody structures in terms of expression level and purity.

In addition, antibodies such as Y100-B7, Y101-B2 and Y101-B4 are expressed in a CHO cell stable expression pool with an expression level of more than 700 mg/L and a purity of more than 85%. The antibody was subject to proteinA affinity chromatography, and then monoclonal antibody purification processes such as conventional anion/cation exchange chromatography, to obtain a final product of interest with a purity of more than 95% and a total recovery rate of 40%, which is consistent with that of a monoclonal antibody in expression and purification processes.

### Example 2. Detection of Biological Activity of Bispecific Antibodies

1. Antigen affinity
1) Antigen preparation: for antigen proteins VEGFA, TGF-β1, IL-10, PD-1, CTLA-4, TIGIT, SLAMF7, LAG-3 and CD16a (see Table 26 for the sequence respectively) and the like, an expression plasmid pcDNA3.1 (purchased from invitrogen) with a His tag was constructed, wherein extracellular domains of the five proteins PD-1, CTLA-4, TIGIT, SLAMF7 and CD16a were selected for construction, and then transiently transfected into 293E cells for expression and purification; the method for purification was divided into two steps, i.e. nickel column purification and molecular sieve purification, and finally the purity of the antigen protein detected by SDS-PAGE was not less than 95%; the concentration of each antigen protein was adjusted to 2µg/ml, and an microtiter plate was coated with the antigen protein at 100µl/well, 4°C overnight; the supernatant was discarded, 250µl of blocking solution (3% BSA in PBS) was added into each well;
2) addition of antibody: according to the experimental design, operation was performed at room temperature, and the antibody was diluted in gradient with 1% BSA in PBS. For example, the initial concentration of antibody for dilution was 3000 nM, and the antibody was diluted by 3-fold with 11 gradients. The diluted antibody was added into wells of microtiter plate at 200µl/well, incubated at room temperature for 2h, and then the supernatant was discarded;
3) washing: the plate was washed by 200µl/well PBST (PBS containing 0.1% Tween20) for 3 times;
4) incubation of secondary antibody: a diluted secondary antibody HRP-labeled anti-human IgG (Sigma, A8792) was added with a volume of 100 µl/well and incubated at room temperature for 1h, wherein the secondary antibody was diluted at 1:40,000 and the diluent was 1% BSA in PBS, and the wells coated with only antigen and secondary antibody were used as a control;
5) washing: the plate was washed with 200µl/well PBST (PBS containing 0.1% Tween20) for 5 times;
6) color-developing: TMB color-developing solution (prepared from A and B color-developing solutions purchased from Wuhan Boster Company, and mixed according to A:B = 1:1, ready to use) was added at 100µl/well, and the color-developing was performed at 37°C for 5min;
7) 2M HCl stopping solution was added at 100µl/well, and then the microplate reader should necessarily be read at 450nm within 30 minutes, and the data were plotted by Graphpad Prism 5 with the antibody concentration (nM) as an abscissa and the average fluorescence intensity as an ordinate; the EC50 value which indicated the affinity of the antibody to the corresponding target antigen was calculated by One site - Specific binding method. The results are shown in Table 32. The results show that for the antibodies with the same variable region sequences, the affinity of the bispecific antibody of the present disclosure (that is, bispecific antibody structure 1) to the corresponding target antigen is significantly higher than that of the bispecific antibody structures 2 to 4. In addition, the bispecific antibodies of the present disclosure (bispecific antibody structure 1) have the strongest antigen-binding activity to any target.

2. Cell affinity
1) Cell preparation: PD-L1 positive H358 cells (purchased from the Cell Resource Center of Shanghai Institutes for Biological Sciences, Chinese Academy of Sciences) were used to detect the binding activity of bispecific antibody molecules against PD-L1 end cells; a sufficient amount of cells were centrifuged at 300×g for 5 min, the supernatant was discarded; the cells were resuspended in 1% FBS-PBS, adjusted to a density of 4×10⁶/ml; 50 µl of cells were taken for each well, and plated at 2×10⁵ per well; wherein the centrifugation was performed at 300×g at 4°C for 5 min, the supernatant was discarded, and the cell plating was operated on ice;
2) Addition of the antibody: according to the experimental design, the antibodies were diluted in a gradient, and the dilution of antibodies was operated on ice; for example, the initial concentration of antibody for dilution was 3000 nM, and the antibody was diluted by 3 times with 11 concentration gradients; the diluted antibody was added into a well at 50µl per well, mixed well by gentle blow, and incubated at 4°C, 1100rpm/min shaking for 2h;
3) washing: the cells were resuspended with 150µl of 1% FBS-PBS, and centrifuged at 300×g at 4°C for 5min; the supernatant was discarded; and the cells were washed repeatedly once;
4) incubation of secondary antibody: a diluted secondary antibody PE anti-human IgG FC (Biolegend, 409304) was added with a volume of 50µl/well, wherein the final concentration of the secondary antibody was 8 µg/ml, mixed well by gentle blow, and incubated at 4°C in dark with 1100rpm/min shaking for 1h; at the same time, a well added with only cells and secondary antibody was used as a control;
5) washing: the cells were resuspended with 150µl of 1% FBS-PBS, and centrifuged at 300g at 4°C for 5min, the supernatant was discarded; the cells were washed repeatedly once;
6) fixation: 2% paraformaldehyde was added at 200µl/well to resuspend and fix the cells at room temperature for 20min, and then the cells were centrifuged at 300×g for 5min; the supernatant was discarded;
7) cell resuspension: the cells were resuspended with 200µl of 1% FBS-PBS, centrifuged at 300×g for 5 min, and the supernatant was discarded;
8) flow cytometry loading: the cells were resuspended with 150µl of 1% FBS-PBS, and detected on a flow cytometer;
9) data analysis: the data were analyzed with a flow analysis software FlowJo 7.6 to obtain the average fluorescence intensity at specific antibody concentration, and plotted by Graphpad Prism 5 with the antibody concentration (nM) as an abscissa and the average fluorescence intensity as an ordinate; One site - specific binding method was used to calculate the EC50 value, which indicated the cell affinity of the antibody to corresponding target antigens; the results are shown in Table 32.

**[Table 32] Binding activity of some antibodies with bispecific antibody structures 1 to 4**

| Antibody code | Binding activity to H358 cell EC50(pM ) | Binding activity to VEGF antigen EC50(pM ) | Binding activity to TGF-β1 antigen EC50 (pM) | Binding activity to LAG-3 antigen EC50 (pM) | Binding activity to PD-1 antigen EC50 (pM) | Binding activity to CTLA-4 antigen EC50 (pM) | Binding activity to TIGIT antigen EC50 (pM) | Binding activity to IL-10 antigen EC50 (pM) | Binding activity to SLAMF7 antigen EC50 (pM) | Binding activity to CD16a antigen EC50 (pM) |
|---|---|---|---|---|---|---|---|---|---|---|
| Y100-B6 | 170.60 | 34.09 | NA | NA | NA | NA | NA | NA | NA | NA |
| Y100-B7 | 169.60 | 35.19 | NA | NA | NA | NA | NA | NA | NA | NA |
| Y200-A1 | 156.24 | 237.93 | NA | NA | NA | NA | NA | NA | NA | NA |
| Y200-B1 | 173.00 | 188.41 | NA | NA | NA | NA | NA | NA | NA | NA |
| Y200-B3 | 207.15 | 47.33 | NA | NA | NA | NA | NA | NA | NA | NA |
| Y300-A1 | 180.95 | 307.96 | NA | NA | NA | NA | NA | NA | NA | NA |
| Y400-B1 | 254.02 | 268.35 | NA | NA | NA | NA | NA | NA | NA | NA |
| Y101-B1 | 179.42 | NA | 5.97 | NA | NA | NA | NA | NA | NA | NA |
| Y101-B2 | 191.23 | NA | 3.09 | NA | NA | NA | NA | NA | NA | NA |
| Y201-A1 | 173.80 | NA | 25.05 | NA | NA | NA | NA | NA | NA | NA |
| Y201-B1 | 192.18 | NA | 28.46 | NA | NA | NA | NA | NA | NA | NA |
| Y101-B3 | 169.40 | NA | 5.46 | NA | NA | NA | NA | NA | NA | NA |
| Y101-B4 | 119.07 | NA | 1.86 | NA | NA | NA | NA | NA | NA | NA |
| Y201-A2 | 143.63 | NA | 31.18 | NA | NA | NA | NA | NA | NA | NA |
| Y201-B2 | 124.05 | NA | 28.01 | NA | NA | NA | NA | NA | NA | NA |
| Y103-B1 | 181.84 | NA | NA | 16.61 | NA | NA | NA | NA | NA | NA |
| Y103-B2 | 169.09 | NA | NA | 12.68 | NA | NA | NA | NA | NA | NA |
| Y203-A1 | 143.09 | NA | NA | 65.70 | NA | NA | NA | NA | NA | NA |
| Y203-B1 | 187.96 | NA | NA | 89.44 | NA | NA | NA | NA | NA | NA |
| Y104-B1 | NA | NA | NA | NA | 127.65 | 56.24 | NA | NA | NA | NA |
| Y104-B2 | NA | NA | NA | NA | 93.78 | 69.68 | NA | NA | NA | NA |
| Y304-A1 | NA | NA | NA | NA | 464.65 | 468.46 | NA | NA | NA | NA |
| Y404-B1 | NA | NA | NA | NA | 388.51 | 330.93 | NA | NA | NA | NA |
| Y105-B1 | NA | NA | NA | NA | 114.13 | NA | 90.17 | NA | NA | NA |
| Y105-B2 | NA | NA | NA | NA | 114.70 | NA | 86.97 | NA | NA | NA |
| Y205-A2 | NA | NA | NA | NA | 306.23 | NA | 484.29 | NA | NA | NA |
| Y205-B2 | NA | NA | NA | NA | 155.11 | NA | 409.16 | NA | NA | NA |
| Y106-B1 | NA | 66.33 | NA | NA | 102.46 | NA | NA | NA | NA | NA |
| Y106-B2 | NA | 53.91 | NA | NA | 112.44 | NA | NA | NA | NA | NA |
| Y206-A2 | NA | 63.03 | NA | NA | 449.79 | NA | NA | NA | NA | NA |
| Y206-B1 | NA | 212.78 | NA | NA | 164.52 | NA | NA | NA | NA | NA |
| Y206-B2 | NA | 59.64 | NA | NA | 311.52 | NA | NA | NA | NA | NA |
| Y110-B1 | NA | NA | NA | NA | 129.81 | NA | NA | 58.79 | NA | NA |
| Y110-B2 | NA | NA | NA | NA | 113.27 | NA | NA | 66.62 | NA | NA |
| Y210-A1 | NA | NA | NA | NA | 148.67 | NA | NA | 409.05 | NA | NA |
| Y210-B1 | NA | NA | NA | NA | 198.43 | NA | NA | 234.03 | NA | NA |
| Y116-B1 | NA | NA | NA | NA | NA | NA | NA | NA | 636.49 | 564.05 |
| Y116-B2 | NA | NA | NA | NA | NA | NA | NA | NA | 648.23 | 436.73 |
| Y216-A1 | NA | NA | NA | NA | NA | NA | NA | NA | 714.64 | 3257.47 |
| Y216-B1 | NA | NA | NA | NA | NA | NA | NA | NA | 499.21 | 3220.97 |
| Y316-A1 | NA | NA | NA | NA | NA | NA | NA | NA | 9176.07 | 3842.87 |
| Y416-B1 | NA | NA | NA | NA | NA | NA | NA | NA | 5590.07 | 3557.75 |
| S70mAb | 175.64 | NA | NA | NA | NA | NA | NA | NA | NA | NA |
| 12A4mAb | 125.69 | NA | NA | NA | NA | NA | NA | NA | NA | NA |
| G631mAb | NA | 30.00 | NA | NA | NA | NA | NA | NA | NA | NA |
| 3G12mAb | NA | NA | 2.60 | NA | NA | NA | NA | NA | NA | NA |
| LAG35mAb | NA | NA | NA | 10.41 | NA | NA | NA | NA | NA | NA |
| 5C4mAb | NA | NA | NA | NA | 111.42 | NA | NA | NA | NA | NA |
| YervoymAb | NA | NA | NA | NA | NA | 59.27 | NA | NA | NA | NA |
| 10A7mAb | NA | NA | NA | NA | NA | NA | 83.14 | NA | NA | NA |
| BN10mAb | NA | NA | NA | NA | NA | NA | NA | 62.96 | NA | NA |
| ElotuzumAb | NA | NA | NA | NA | NA | NA | NA | NA | 628.00 | NA |
| NM3E2mAb | NA | NA | NA | NA | NA | NA | NA | NA | NA | 520.10 |

From the data in Table 32, it can be seen that when the variable region sequences of different antibodies are identical, the binding activity of such antibodies with different bispecific antibody structures to a target-antigen is significantly different, wherein for any targets, the antibodies with bispecific antibody structure 1 have the strongest antigen-binding activity. If the hinge region, CH2 domain and CH3 domain of each of the above antibodies are substituted with the corresponding sequence of IgG2, IgG3 or IgG4 (supra) and the antibody variable region and linker sequence are remained the same to obtain a substituted antibody, there is no significant difference in antigen-binding activity between the resultant substituted antibody and the original antibody.

### Example 3. Stability test of the antibodies

Experimental operation:
1. The specific steps of 40°C accelerated thermal stability test are:
   1) the sample was displaced into a specific buffer, the component of the buffer is 20mM citric acid, pH5.5, and the concentration of the sample was adjusted to 1mg/mL;
   2) each sample was divided into 500µL per tube (6 tubes in total) and the tubes were placed in a 40°C water bath after being sealed. On day 0, day 3, day 5, day 7, day 10, and day 14, samples were taken for HPLC-SEC. The water bath time was 14 days in total.
2. Acid resistance test, also known as low-pH stability test, is a test to detect whether the antibody molecule can maintain its original state after being treated in an acidic environment for a period of time and then being neutralized to a physiological condition. The specific steps are:
   when antibody molecules were subjected to protein A affinity chromatography, the eluted antibody solution was not neutralized in the acid elution step (citrate buffer at pH 3.5 was used); after being kept in said buffer for a period of time, samples were taken at 30 min and 60 min, and 1/10 volume of 1M Tris-HCl (pH 8.0) was added for neutralization, and the samples were detected by HPLC-SEC.
3.1 Stability test of bispecific antibody structure 1
   (1) 40°C accelerated thermal stability test of different bispecific antibody structures

The test results are shown in Figures 10A-E. Figure 10A shows the purity of Y100-B6, Y100-B7, Y200-A1, Y200-B1, Y200-B3, Y300-A1 and Y400-B1 detected by HPLC-SEC on day 0, day 7 and day 14 after being treated at 40°C; Figure 10B shows the purity of Y101-B1, Y101-B2, Y201-A1, Y201-B1, Y101-B3, Y101-B4, Y201-A2 and Y201-B2 detected by HPLC-SEC at day 0, day 7 and day 14 after being treated at 40°C; Figure 10C shows purity of Y103-B1, Y103-B2, Y203-A1, Y203-B1, Y104-B1, Y104-B2, Y304-A1 and Y404-B1 detected by HPLC -SEC at day 0, day 7 and day 14 after being treated at 40°C; Figure 10D shows purity of Y105-B1, Y105-B2, Y205-A2, Y205-B2, Y106-B1, Y106-B2, Y206-A2, Y206-B1 and Y206-B2 detected by HPLC-SEC at day 0, day 7 and day 14 after being treated at 40°C; Figure 10E shows purity of Y110-B1, Y110-B2, Y210-A1, Y210-B1, Y116-B1, Y116-B2, Y216-A1, Y216-B1, Y316-A1, Y416-B1 detected by HPLC-SEC at day 0, day 7 and day 14 after being treated at 40°C .

It can be seen from Figure 10 that the purity of different antibodies with bispecific antibody structure 1 was maintained at more than 90% after being treated at 40°C for 14 days, without a large amount of aggregation or degradation, which indicates that the bispecific antibody structure 1 has a good thermal stability. The purity of other bispecific antibody structures decreased significantly after being treated at 40°C for 14 days.

### (2) Acid resistance test of different bispecific antibody structures

The test results are shown in Figures 11A-E. Figure 11A shows the purity of Y100-B6, Y100-B7, Y200-A1, Y200-B1, Y200-B3, Y300-A1 and Y400-B1 detected by HPLC-SEC after being treated under low pH conditions for 0 minutes, 30 minutes and 60 minutes; Figure 11B shows the purity of Y101-B1, Y101-B2, Y201-A1, Y201-B1, Y101-B3, Y101-B4, Y201-A2 and Y201-B2 detected by HPLC-SEC after being treated under low pH conditions for 0 minutes, 30 minutes and 60 minutes; Figure 11C shows the purity of Y103-B1, Y103-B2, Y203-A1, Y203-B1, Y104-B1, Y104-B2, Y304-A1 and Y404-B1 detected by HPLC-SEC after being treated under low pH conditions for 0 minutes, 30 minutes and 60 minutes; Figure 11D shows purity of Y105-B1, Y105-B2, Y205-A2, Y205-B2, Y106-B1, Y106-B2, Y206-A2, Y206-B1 and Y206-B2 detected by HPLC-SEC after being treated under low pH conditions for 0 minutes, 30 minutes and 60 minutes; Figure 11E shows purity of Y110-B1, Y110-B2, Y210-A1, Y210-B1, Y116-B1, Y116-B2, Y216-A1, Y216-B1, Y316-A1 and Y416-B1 detected by HPLC-SEC after being treated under low pH conditions for 0 minutes, 30 minutes and 60 minutes.

It can be seen from Figures 11A-E that if the antibodies of the bispecific antibody structure 1 were treated for 60 minutes under low pH conditions, the antibody purity did not change significantly, which indicates that the bispecific antibody structure 1 has a good acid resistance. For other bispecific antibody structures, the purity of some antibodies decreased significantly after 60 minutes of treatment under low pH conditions.

Based on Figures 10 and 11, it can be seen that as compared to other bispecific antibody structures, the antibody of bispecific antibody structure 1 has a better thermal stability and acid resistance.

If the hinge region, CH2 domain and CH3 domain of each of the above antibodies are substituted with corresponding sequence of IgG2, IgG3 or IgG4 (supra) and the antibody variable region and linker sequence are remained the same to obtain a substituted antibody, there is no significant difference in stability between the resultant substituted antibody and the original antibody.

### Example 4. In vivo pharmacodynamics experiment 1 of bispecific antibodies

1). Experimental materials:
   Cells: MC38 (mouse colon cancer cell line, purchased from ATCC);
   Mouse: C57BL/6 mouse, female, purchased from Beijing Vital River;
   Method of inoculation: MC38 cells were cultured and collected, and the cell concentration was adjusted, and then the cells were subcutaneously inoculated on the back at 1^{∗}10⁶ cells/mouse (0.1ml/mouse). When the tumor was grown to 100 to 200mm³, the mice were administered in group with 8 mice in each group;
   Test drug: Y100-B7;
   Negative control: physiological saline;
   Positive control: Tecentriq (PD-L1 monoclonal antibody, Roche);
   Administration method: different dosages (13.3mg/kg and 4mg/kg) were set for Y100-B7, and the administration dosage of Tecentriq was 10mg/kg; the drug was administered intraperitoneally, starting on day 0, 3 times per week for one week.
   Tumor volume: the length and width of the tumor were measured every 2 to 3 days. When the tumor volume of a group approached 2000mm³ or the tumor volume of an individual mouse reached 3000mm³, the group will end.
2). Experimental results
   The results of in vivo pharmacodynamics test for the Y100-B7 are shown in Figure 12. Figure 12 shows the in vivo pharmacodynamics of the bispecific antibody Y100-B7 in a mouse tumor model and the tumor volume. The results show that the Y100-B7 high-dosage group (13.3 mg/kg) has a significant anti-tumor effect and the effect is better than Tecentriq .

### Example 5. In vivo pharmacodynamics experiment 2 of bispecific antibodies

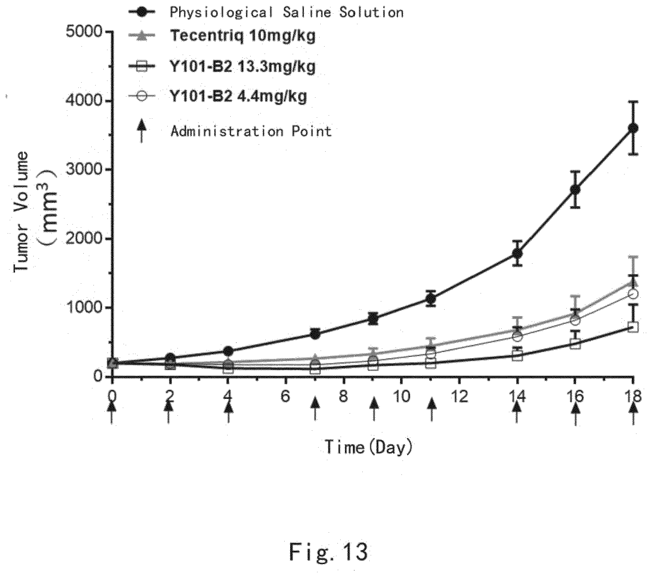

1). Experimental materials:
   Cells: MC38 (mouse colon cancer cell line, purchased from ATCC);
   Mouse: C57BL/6 mouse, female, purchased from Beijing Vital River;
   Method of inoculation: MC38 cells were cultured, collected and the cell concentration was adjusted, and the cells were subcutaneously inoculated on the back at 1^{∗}10⁶ cells/mouse (0.1ml/mouse). When the tumor was grown to 100 to 200 mm³, the mice were administered in group with 8 mice in each group;
   Test drug: Y101-B2;
   Negative control: physiological saline;
   Positive control: Tecentriq (PD-L1 monoclonal antibody, Roche);
   Administration method: different dosages (13.3mg/kg and 4mg/kg) were set for Y101-B2, and the administration dosage of Tecentriq was 10mg/kg; the drug was administered intraperitoneally, starting on day 0, 3 times per week for 3 weeks.
   Tumor volume: the length and width of the tumor were measured 3 times per week. When the tumor volume of a group approached 2000mm³ or the tumor volume of an individual mouse reached 3000mm³, the group will end.
2). Experimental results
   The results of in vivo pharmacodynamics test for the Y101-B2 are shown in Figure 13. Figure 13 shows the in vivo pharmacodynamics of the bispecific antibody Y101-B2 in a mouse tumor model and the tumor volume. The results show that the Y101-B2 high-dosage group (13.3 mg/kg) has a significant tumor-inhibiting effect and the effect is better than Tecentriq.

### Example 6: Preparation and biological activity detection of biparatopic antibody

Biparatopic antibody is a kind of bispecific antibody and can bind to two different epitopes of the same antigen.

The construction of expression plasmid, the transient transfection of 293E cells and the method of preparing antibody were the same as the above-mentioned methods.

The antigen involved was the extracellular domain of human Her2 (sequence source: UniProtKB-P04626).

The involved antibody variable region sequences were shown in the table below:

**[Table 33] variable region sequences of anti-Her2 antibody**

| Antibody code (Source of Sequence) | amino acid sequences of anti-Her2 antibody variable region (Bold and underlined amino acids are CDR regions) | | | |
|---|---|---|---|---|
| | VH | SEQ ID NO. | VL | SEQ ID NO. |
| Herceptin | | 146 | | 147 |
| Perjeta | | 148 | | 149 |

The sequences of the constructed antibodies are shown in the following tables:

**[Table 34] The antibody codes and amino acid sequences of some antibodies with bispecific antibody structure 1**

| Antibody code | Peptide | Domain | Code | Amino acid sequences (Bold and underlined amino acids are CDR regions) | SEQ ID NO. |
|---|---|---|---|---|---|
| Herceptin mAb | Heavy Chain | VH | Herceptin | | 146 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| | Light Chain | VL | Herceptin | | 147 |
| | | CL | Lc1 | | 104 |
| Perjeta mAb | Heavy Chain | VH | Perjeta | | 148 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Hinge | Hinl | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| | Light Chain | VL | Perjeta | | 149 |
| | | CL | Lc1 | | 104 |
| Y140-A1 | Fusion Light Chain | VLa | Herceptin | | 147 |
| | | CL | Lc1 | | 104 |
| | | Linker1 | Lin17 | GGGGSEAAAKGGGGS | 85 |
| | | VLb | Perjeta | | 149 |
| | Fusion Heavy Chain | VHa | Herceptin | | 146 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VHb | Perjeta | | 148 |
| | | Linker3 | Lin14 | GGGGSGGGGSGGGGS | 82 |
| | | Hinge CH2 | Hin1 G2DCH2 | [?] PCPAPPVAGPSVFLFPPKPKDTLMISRTPEVTCVVVDVSHEAPEVQFNWYVDGVEVHNAKTKPREEQFNST FRVVSVLTVVHQDWLNGKEYKCKVSNKGLPAPIEKTISKTK | 91 |
| | | | | DKTHTCP | 117 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| Y140-A2 | Fusion Light Chain | VLa | Perjeta | | 149 |
| | | CL | Lc1 | | 104 |
| | | Linker1 | Lin17 | GGGGSEAAAKGGGGS | 85 |
| | | VLb | Herceptin | | 147 |
| | Fusion Heavy Chain | VHa | Perjeta | | 148 |
| | | CH1 Linker2 | G1CH1 Lin17 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | | | GGGGSEAAAKGGGGS | 85 |
| | | VHb | Herceptin | | 146 |
| | | Linker3 | Lin14 | GGGGSGGGGSGGGGS | 82 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNST FRVVSVLTVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| | Fusion Light Chain | VLa | Herceptin | | 147 |
| | | CL | Lc1 | | 104 |
| | | Linker1 | Lin17 | GGGGSEAAAKGGGGS | 85 |
| | | VLb | Perjeta | | 149 |
| Y140-A3 | Fusion Heavy Chain | VHa | Herceptin | | 146 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VHb | Perjeta | | 148 |
| | | Linker3 | Lin14 | GGGGSGGGGSGGGGS | 82 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| Y140-A4 | Fusion Light Chain | VLa | Perjeta | | 149 |
| | | CL | Lc1 | | 104 |
| | | Linker1 | Lin17 | GGGGSEAAAKGGGGS | 85 |
| | | VLb | Herceptin | | 147 |
| | Fusion Heavy Chain | VHa | Perjeta | | 148 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VHb | Herceptin | | 146 |
| | | Linker3 | Linl4 | GGGGSGGGGSGGGGS | 82 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| Y140-B1 | Fusion Light Chain | VLa | Herceptin | | 147 |
| | | CL | Lc1 | | 104 |
| | | Linker1 | Lin17 | GGGGSEAAAKGGGGS | 85 |
| | | VHb | Perjeta | | 148 |
| | Fusion Heavy Chain | VHa | Herceptin | | 146 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VLb | Perjeta | | 149 |
| | | Linker3 | Linl4 | GGGGSGGGGSGGGGS | 82 |
| | | Hinge | Hin1 | DKTHTCP | 91 |
| | | CH2 | G2DCH2 | PCPAPPVAGPSVFLFPPKPKDTLMISRTPEVTCVVVDVSHEAPEVQFNWYVDGVEVHNAKTKPREEQFNST FRVVSVLTVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| Y140-B2 | Fusion Light Chain | VLa | Perjeta | | 149 |
| | | CL | Lc1 | | 104 |
| | | Linker1 | Lin17 | GGGGSEAAAKGGGGS | 85 |
| | | VHb | Herceptin | | 146 |
| | Fusion Heavy Chain | VHa | Perjeta | | 148 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VLb | Herceptin | | 147 |
| | | Linker3 | Linl4 | GGGGSGGGGSGGGGS | 82 |
| | | Hinge | Hin1 | DKTHTCP | 91 |
| | | CH2 | G2DCH2 | DKTHTCP PCPAPPVAGPSVFLFPPKPKDTLMISRTPEVTCVVVDVSHEAPEVQFNWYVDGVEVHNAKTKPREEQFNST FRVVSVLTVVHQDWLNGKEYKCKVSNKGLPAPIEKTISKTK | 117 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| Y140-B3 | Fusion Light Chain | VLa | Herceptin | | 147 |
| | | CL | Lcl | | 104 |
| | | Linker1 | Lin17 | GGGGSEAAAKGGGGS | 85 |
| | | VHb | Perjeta | | 148 |
| | Fusion Heavy Chain | VHa | Herceptin | | 146 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VLb | Peijeta | GGGGSEAAAKGGGGS DIQMTQSPSSLSASVGDRVTITCKASQDVSIGVAWYQQKPGKAPKLLIYSASYRYTGVPSRFSGSGSGTDFT LTISSLQPEDFATYYCQQYYIYPYTFGQGTKVEIK | 149 |
| | | Linker3 | Linl4 | GGGGSGGGGSGGGGS | 82 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAK | 115 |
| | | CH3 | G1CH3 | GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |
| Y140-B4 | Fusion Light Chain | VLa | Perjeta | | 149 |
| | | CL | Lcl | | 104 |
| | | Linker1 | Lin17 | GGGGSEAAAKGGGGS | 85 |
| | | VHb | Herceptin | | 146 |
| | Fusion Heavy Chain | VHa | Perjeta | | 148 |
| | | CH1 | G1CH1 | ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC | 111 |
| | | Linker2 | Lin17 | GGGGSEAAAKGGGGS | 85 |
| | | VLb | Herceptin | | 147 |
| | | Linker3 | Linl4 | GGGGSGGGGSGGGGS | 82 |
| | | Hinge | Hin1 | DKTHTCP | 91 |
| | | CH2 | G1CH2 | PCPAPELLGGPSVFLFPPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAK | 115 |
| | | CH3 | G1CH3 | [?] GQPREPQVYTLPPSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK | 120 |

The transient transfection expression level of both of Herceptin mAb and Perjeta mAb is 30 mg/L; the transient transfection expression level of bispecific antibody Y140-B1/B2/B3/B4 is not less than 25mg/L; the expression level of bispecific antibody Y140-A1/A2/A3/A4 is 7 mg/L to 20mg/L, and the purity of all antibodies detected by SEC is not less than 80%.

### 2. Detection of biological activity

### Cell affinity

1) Cell preparation: Her2-positive BT-474 cells (purchased from the Cell Bank of the Type Culture Collection Committee of the Chinese Academy of Sciences) were used for a Her2-end affinity detection of bispecific antibody molecule; sufficient cells were centrifuged at 300×g for 5 min, the supernatant was discarded; the cells were resuspended in 1% FBS-PBS, adjusted to a density of 2×10⁶/ml, and 50 µl of cells were taken per well, and then plated at 1×10⁵ per well; wherein, the cells were centrifuged at 300×g at 4°C for 5 min, the supernatant was discarded, and the cell plating was operated on ice;
2) Addition of antibody: according to the experimental design, the antibody in Table 34 was diluted in gradient, and the dilution of antibody was operated on ice; for example, the initial concentration of antibody for dilution was 3000 nM, and the antibody was diluted by 3-fold with 11 concentration gradients. The diluted antibody was added into a well at 50µl per well, mixed well by gentle blow, and incubated at 4°Cwith 1 100rpm/min shaking for 2h;
3) washing: the cells were resuspended with 150µl 1% FBS-PBS, centrifuged at 300×g at 4°C for 5min, and the supernatant was discarded; the cells were washed repeatedly once;
4) incubation of secondary antibody: a diluted secondary antibody PE anti-human IgG FC (Biolegend, 409304) was added with a volume of 50 µl/well, wherein the final concentration of the secondary antibody was 8 µg/ml, mixed well by gentle blow, and incubated at 4°C in dark with 1100 rpm/min shaking for 1h; at the same time, a well added with only cells and secondary antibody was used as a control;
5) washing: the cells were resuspended with 150µl of 1% FBS-PBS, and centrifuged at 300g at 4°C for 5min, the supernatant was discarded; the cells were washed repeatedly once;
6) fixation: 2% paraformaldehyde was added at 200 µl/well to resuspend and fix the cells at room temperature for 20min, and then the cells were centrifuged at 300×g for 5min; the supernatant was discarded;
7) cell resuspension: the cells were resuspended with 200 µl of 1% FBS-PBS, centrifuged at 300×g for 5 min, and the supernatant was discarded;
8) flow cytometry loading: the cells were resuspended with 150 µl of 1% FBS-PBS, and detected on a flow cytometer;
9) data analysis: the data were analyzed with a flow analysis software FlowJo 7.6 to obtain the average fluorescence intensity at specific antibody concentration, and plotted by Graphpad Prism 5 with the antibody concentration (nM) as an abscissa and the average fluorescence intensity as an ordinate; One site - specific binding method was used to calculate the EC50 value, which indicated the cell affinity of the antibody to corresponding target antigens.

**[Table 35] Binding activity of some bispecific antibodies**

| Antibody code | Binding activity to BT-474 cells EC50 (pM) |
|---|---|
| Herceptin mAb | 2567.33 |
| Perjeta mAb | 5456.55 |
| Y140-A1 | 1931.84 |
| Y140-A2 | 1313.78 |
| Y140-A3 | 1889.42 |
| Y140-A4 | 1537.68 |
| Y140-B1 | 838.577 |
| Y140-B2 | 813.730 |
| Y140-B3 | 526.554 |
| Y140-B4 | 420.308 |

It can be seen from the above table that the affinity of bispecific antibodies assembled with two antibodies that target the same antigen but different epitopes is significantly improved as compared with that of two monoclonal antibodies, and the affinity of the B series bispecific antibodies is significantly stronger than that of the A series antibodies.

## Claims

1. A bispecific antibody, which comprises two identical fusion heavy chains and two identical fusion light chains, wherein the two fusion heavy chains form a pair, and the fusion light chain and the fusion heavy chain form a pair; wherein the fusion heavy chain comprises a heavy chain variable region of antibody a (VHa), a first constant region CH1, a variable region 1 of antibody b and a Fc fragment, the variable region 1 of antibody b is linked to the CH1 by a linker 2 or a peptide bond and to the Fc by a linker 3 or a peptide bond; the fusion light chain of the bispecific antibody comprises a light chain variable region of antibody a (VLa), a light chain constant region CL and a variable region 2 of antibody b, wherein the variable region 2 of antibody b is linked to a C-terminus of the CL through a linker 1 or a peptide bond, wherein,
(1) the variable region 1 of antibody b is a heavy chain variable region of antibody b (VHb), and the variable region 2 of antibody b is a light chain variable region of antibody b (VLb);
or
(2) the variable region 1 of antibody b is a light chain variable region of antibody b (VLb), and the variable region 2 of antibody b is a heavy chain variable region of antibody b (VHb),
wherein, the VHa-VLa pair targets antigen A, and the VHb-VLb pair targets antigen B,
preferably, the bispecific antibody is a tetravalent symmetrical bispecific antibody with a structure of F(ab)₂-(Fv)₂-Fc, wherein F(ab)₂ includes two VHa, two CH1, two VLa and two CL; (Fv)₂ includes two VH/VLb and two VL/VHb, wherein positions of the VH/VLb and the VL/VHb are interchangeable; and the Fc fragment includes a hinge region, a second constant region CH2 and a third constant region CH3.

2. The bispecific antibody of claim 1, wherein the VHa-VLa pair forms one or more interchain disulfide bonds, and the VHb-VLb pair forms one or more interchain disulfide bonds.

3. The bispecific antibody of claim 1 or 2, wherein the variable region 1 of antibody b is the light chain variable region of antibody b (VLb), and the variable region 2 of antibody b is the heavy chain variable region of antibody b (VHb).

4. The bispecific antibody of any one of claims 1 to 3, wherein the linkers 1 to 3 may be the same or different, and the linkers 1 to 3 are each independently selected from a group consisting of SEQ ID NOs: 69 to 90, preferably selected from SEQ ID NOs: 71, 72, 73, 74, 75, 76, 77, 78, 79, 80, 82, 83, 85, 86, 87, 88, 89, or 90.

5. The bispecific antibody of any one of claims 1 to 4, wherein sequence of the CL is selected from a group consisting of SEQ ID NOs: 104 to 110.

6. The bispecific antibody of any one of claims 1 to 5, wherein sequence of the CH1 is selected from a group consisting of SEQ ID NOs: 111 to 114.

7. The bispecific antibody of any one of claims 1 to 6, wherein sequence of the CH2 is selected from a group consisting of SEQ ID NOs: 115 to 119.

8. The bispecific antibody of any one of claims 1 to 7, wherein sequence of the CH3 is selected from a group consisting of SEQ ID NOs: 120 to 128.

9. The bispecific antibody of any one of claims 1 to 8, wherein the antigen A and the antigen B may be the same or different, preferably, the antigen A and the antigen B are each independently selected from a group consisting of: immune cell surface antigens, tumor antigens, viruses, bacteria, endotoxins, cytokines, or a combination thereof, more preferably, the antigen A or the antigen B are different or represent different epitopes on the same antigen.

10. The bispecific antibody of any one of claims 1 to 9, wherein the antigen A and/or the antigen B is selected from PD-L1, PD-1, VEGFA, IL-10, IL-10R, BCMA, VEGF, TGF-β, CTLA-4, LAG-3, TIGIT, CEA, CD38, SLAMF7, B7-H3, Her2, EpCAM, CD19, CD20, CD30, CD33, CD47, CD52, CD133, EGFR, GD2, GD3, GM2, RANKL, CD3 and/or CD16a, preferably, wherein the antigen A and/or the antigen B is selected from SEQ ID NOs: 129 to 145.

11. The bispecific antibody of any one of claims 1 to 10, wherein the antigen A and the antigen B are selected from a group consisting of:
PD-L1 and VEGF,
PD-1 and VEGF,
PD-L1 and TGF-β,
PD-1 and TGF-β,
PD-1 and CTLA-4,
PD-1 and LAG-3,
PD-1 and TIGIT,
PD-1 and IL-10,
SLAMF7 and CD16a, and
Her2 and Her2.

12. The bispecific antibody of any one of claims 1 to 10, which is selected from a group consisting of:
(1) the fusion light chain includes SEQ ID NO: 58, SEQ ID NO: 104, SEQ ID NO: 82 and SEQ ID NO: 42; the fusion heavy chain includes SEQ ID NO: 57, SEQ ID NO: 111, SEQ ID NO: 82, SEQ ID NO: 41, SEQ ID NO: 82, SEQ ID NO: 91, SEQ ID NO: 117 and SEQ ID NO: 120;
(2) the fusion light chain includes SEQ ID NO: 58, SEQ ID NO: 104, SEQ ID NO: 89 and SEQ ID NO: 42; the fusion heavy chain includes SEQ ID NO: 57, SEQ ID NO: 111, SEQ ID NO: 83, SEQ ID NO: 41, SEQ ID NO: 74, SEQ ID NO: 91, SEQ ID NO: 117 and SEQ ID NO: 120;
(3) the fusion light chain includes SEQ ID NO: 58, SEQ ID NO: 104, SEQ ID NO: 85 and SEQ ID NO: 42; the fusion heavy chain includes SEQ ID NO: 57, SEQ ID NO: 111, SEQ ID NO: 85, SEQ ID NO: 41, SEQ ID NO: 74, SEQ ID NO: 91, SEQ ID NO: 117 and SEQ ID NO: 120;
(4) the fusion light chain includes SEQ ID NO: 58, SEQ ID NO: 104, SEQ ID NO: 89 and SEQ ID NO: 46; the fusion heavy chain includes SEQ ID NO: 57, SEQ ID NO: 111, SEQ ID NO: 83, SEQ ID NO: 45, SEQ ID NO: 74, SEQ ID NO: 91, SEQ ID NO: 117 and SEQ ID NO: 120;
(5) the fusion light chain includes SEQ ID NO: 58, SEQ ID NO: 104, SEQ ID NO: 85 and SEQ ID NO: 46; the fusion heavy chain includes SEQ ID NO: 57, SEQ ID NO: 111, SEQ ID NO: 85, SEQ ID NO: 45, SEQ ID NO: 74, SEQ ID NO: 91, SEQ ID NO: 117 and SEQ ID NO: 120;
(6) the fusion light chain includes SEQ ID NO: 42, SEQ ID NO: 104, SEQ ID NO: 89 and SEQ ID NO: 58; the fusion heavy chain includes SEQ ID NO: 41, SEQ ID NO: 111, SEQ ID NO: 83, SEQ ID NO: 57, SEQ ID NO: 74, SEQ ID NO: 91, SEQ ID NO: 117 and SEQ ID NO: 120;
(7) the fusion light chain includes SEQ ID NO: 42, SEQ ID NO: 104, SEQ ID NO: 85 and SEQ ID NO: 58; the fusion heavy chain includes SEQ ID NO: 41, SEQ ID NO: 111, SEQ ID NO: 85, SEQ ID NO: 57, SEQ ID NO: 74, SEQ ID NO: 91, SEQ ID NO: 117 and SEQ ID NO: 120;
(8) the fusion light chain includes SEQ ID NO: 46, SEQ ID NO: 104, SEQ ID NO: 89 and SEQ ID NO: 58; the fusion heavy chain includes SEQ ID NO: 45, SEQ ID NO: 111, SEQ ID NO: 83, SEQ ID NO: 57, SEQ ID NO: 74, SEQ ID NO: 91, SEQ ID NO: 117 and SEQ ID NO: 120;
(9) the fusion light chain includes SEQ ID NO: 46, SEQ ID NO: 104, SEQ ID NO: 85 and SEQ ID NO: 58; the fusion heavy chain includes SEQ ID NO: 45, SEQ ID NO: 111, SEQ ID NO: 85, SEQ ID NO: 57, SEQ ID NO: 74, SEQ ID NO: 91, SEQ ID NO: 117 and SEQ ID NO: 120;
(10) the fusion light chain includes SEQ ID NO: 42, SEQ ID NO: 104, SEQ ID NO: 85 and SEQ ID NO: 54; the fusion heavy chain includes SEQ ID NO: 41, SEQ ID NO: 111, SEQ ID NO: 85, SEQ ID NO: 53, SEQ ID NO: 74, SEQ ID NO: 91, SEQ ID NO: 117 and SEQ ID NO: 120;
(11) the fusion light chain includes SEQ ID NO: 42, SEQ ID NO: 104, SEQ ID NO: 85 and SEQ ID NO: 58; the fusion heavy chain includes SEQ ID NO: 41, SEQ ID NO: 111, SEQ ID NO: 85, SEQ ID NO: 57, SEQ ID NO: 74, SEQ ID NO: 91, SEQ ID NO: 115 and SEQ ID NO: 120;
(12) the fusion light chain includes SEQ ID NO: 42, SEQ ID NO: 104, SEQ ID NO: 89 and SEQ ID NO: 68; the fusion heavy chain includes SEQ ID NO: 41, SEQ ID NO: 111, SEQ ID NO: 83, SEQ ID NO: 67, SEQ ID NO: 74, SEQ ID NO: 91, SEQ ID NO: 117 and SEQ ID NO: 120;
(13) the fusion light chain includes SEQ ID NO: 68, SEQ ID NO: 104, SEQ ID NO: 85 and SEQ ID NO: 58; the fusion heavy chain includes SEQ ID NO: 67, SEQ ID NO: 111, SEQ ID NO: 85, SEQ ID NO: 57, SEQ ID NO: 74, SEQ ID NO: 91, SEQ ID NO: 117 and SEQ ID NO: 120;
(14) the fusion light chain includes SEQ ID NO: 42, SEQ ID NO: 104, SEQ ID NO: 89 and SEQ ID NO: 60; the fusion heavy chain includes SEQ ID NO: 41, SEQ ID NO: 111, SEQ ID NO: 83, SEQ ID NO: 59, SEQ ID NO: 74, SEQ ID NO: 91, SEQ ID NO: 117 and SEQ ID NO: 120;
(15) the fusion light chain includes SEQ ID NO: 42, SEQ ID NO: 104, SEQ ID NO: 85 and SEQ ID NO: 60; the fusion heavy chain includes SEQ ID NO: 41, SEQ ID NO: 111, SEQ ID NO: 85, SEQ ID NO: 59, SEQ ID NO: 74, SEQ ID NO: 91, SEQ ID NO: 117 and SEQ ID NO: 120;
(16) the fusion light chain includes SEQ ID NO: 46, SEQ ID NO: 104, SEQ ID NO: 89 and SEQ ID NO: 60; the fusion heavy chain includes SEQ ID NO: 45, SEQ ID NO: 111, SEQ ID NO: 83, SEQ ID NO: 59, SEQ ID NO: 74, SEQ ID NO: 91, SEQ ID NO: 117 and SEQ ID NO: 120;
(17) the fusion light chain includes SEQ ID NO: 46, SEQ ID NO: 104, SEQ ID NO: 85 and SEQ ID NO: 60; the fusion heavy chain includes SEQ ID NO: 45, SEQ ID NO: 111, SEQ ID NO: 85, SEQ ID NO: 59, SEQ ID NO: 74, SEQ ID NO: 91, SEQ ID NO: 117 and SEQ ID NO: 120;
(18) the fusion light chain includes SEQ ID NO: 42, SEQ ID NO: 104, SEQ ID NO: 85 and SEQ ID NO: 60; the fusion heavy chain includes SEQ ID NO: 41, SEQ ID NO: 111, SEQ ID NO: 85, SEQ ID NO: 59, SEQ ID NO: 74, SEQ ID NO: 91, SEQ ID NO: 115 and SEQ ID NO: 120;
(19) the fusion light chain includes SEQ ID NO: 42, SEQ ID NO: 104, SEQ ID NO: 89 and SEQ ID NO: 34; the fusion heavy chain includes SEQ ID NO: 41, SEQ ID NO: 111, SEQ ID NO: 83, SEQ ID NO: 33, SEQ ID NO: 74, SEQ ID NO: 91, SEQ ID NO: 117 and SEQ ID NO: 120;
(20) the fusion light chain includes SEQ ID NO: 42, SEQ ID NO: 104, SEQ ID NO: 85 and SEQ ID NO: 34; the fusion heavy chain includes SEQ ID NO: 41, SEQ ID NO: 111, SEQ ID NO: 85, SEQ ID NO: 33, SEQ ID NO: 74, SEQ ID NO: 91, SEQ ID NO: 117 and SEQ ID NO: 120;
(21) the fusion light chain includes SEQ ID NO: 42, SEQ ID NO: 104, SEQ ID NO: 85 and SEQ ID NO: 34; the fusion heavy chain includes SEQ ID NO: 41, SEQ ID NO: 111, SEQ ID NO: 85, SEQ ID NO: 33, SEQ ID NO: 74, SEQ ID NO: 91, SEQ ID NO: 115 and SEQ ID NO: 120;
(22) the fusion light chain includes SEQ ID NO: 38, SEQ ID NO: 104, SEQ ID NO: 89 and SEQ ID NO: 28; the fusion heavy chain includes SEQ ID NO: 37, SEQ ID NO: 111, SEQ ID NO: 83, SEQ ID NO: 27, SEQ ID NO: 74, SEQ ID NO: 91, SEQ ID NO: 117 and SEQ ID NO: 120;
(23) the fusion light chain includes SEQ ID NO: 38, SEQ ID NO: 104, SEQ ID NO: 85 and SEQ ID NO: 28; the fusion heavy chain includes SEQ ID NO: 37, SEQ ID NO: 111, SEQ ID NO: 85, SEQ ID NO: 27, SEQ ID NO: 74, SEQ ID NO: 91, SEQ ID NO: 117 and SEQ ID NO: 120;
(24) the fusion light chain includes SEQ ID NO: 38, SEQ ID NO: 104, SEQ ID NO: 85 and SEQ ID NO: 28; the fusion heavy chain includes SEQ ID NO: 37, SEQ ID NO: 111, SEQ ID NO: 85, SEQ ID NO: 27, SEQ ID NO: 74, SEQ ID NO: 91, SEQ ID NO: 115 and SEQ ID NO: 120;
(25) the fusion light chain includes SEQ ID NO: 38, SEQ ID NO: 104, SEQ ID NO: 89 and SEQ ID NO: 30; the fusion heavy chain includes SEQ ID NO: 37, SEQ ID NO: 111, SEQ ID NO: 83, SEQ ID NO: 29, SEQ ID NO: 74, SEQ ID NO: 91, SEQ ID NO: 117 and SEQ ID NO: 120;
(26) the fusion light chain includes SEQ ID NO: 38, SEQ ID NO: 104, SEQ ID NO: 85 and SEQ ID NO: 30; the fusion heavy chain includes SEQ ID NO: 37, SEQ ID NO: 111, SEQ ID NO: 85, SEQ ID NO: 29, SEQ ID NO: 74, SEQ ID NO: 91, SEQ ID NO: 117 and SEQ ID NO: 120;
(27) the fusion light chain includes SEQ ID NO: 38, SEQ ID NO: 104, SEQ ID NO: 85 and SEQ ID NO: 30; the fusion heavy chain includes SEQ ID NO: 37, SEQ ID NO: 111, SEQ ID NO: 85, SEQ ID NO: 29, SEQ ID NO: 74, SEQ ID NO: 91, SEQ ID NO: 115 and SEQ ID NO: 120;
(28) the fusion light chain includes SEQ ID NO: 38, SEQ ID NO: 104, SEQ ID NO: 89 and SEQ ID NO: 58; the fusion heavy chain includes SEQ ID NO: 37, SEQ ID NO: 111, SEQ ID NO: 83, SEQ ID NO: 57, SEQ ID NO: 74, SEQ ID NO: 91, SEQ ID NO: 117 and SEQ ID NO: 120;
(29) the fusion light chain includes SEQ ID NO: 38, SEQ ID NO: 104, SEQ ID NO: 85 and SEQ ID NO: 58; the fusion heavy chain includes SEQ ID NO: 37, SEQ ID NO: 111, SEQ ID NO: 85, SEQ ID NO: 57, SEQ ID NO: 74, SEQ ID NO: 91, SEQ ID NO: 117 and SEQ ID NO: 120;
(30) the fusion light chain includes SEQ ID NO: 38, SEQ ID NO: 104, SEQ ID NO: 89 and SEQ ID NO: 60; the fusion heavy chain includes SEQ ID NO: 37, SEQ ID NO: 111, SEQ ID NO: 83, SEQ ID NO: 59, SEQ ID NO: 74, SEQ ID NO: 91, SEQ ID NO: 117 and SEQ ID NO: 120;
(31) the fusion light chain includes SEQ ID NO: 38, SEQ ID NO: 104, SEQ ID NO: 85 and SEQ ID NO: 60; the fusion heavy chain includes SEQ ID NO: 37, SEQ ID NO: 111, SEQ ID NO: 85, SEQ ID NO: 59, SEQ ID NO: 74, SEQ ID NO: 91, SEQ ID NO: 117 and SEQ ID NO: 120;
(32) the fusion light chain includes SEQ ID NO: 38, SEQ ID NO: 104, SEQ ID NO: 85 and SEQ ID NO: 60; the fusion heavy chain includes SEQ ID NO: 37, SEQ ID NO: 111, SEQ ID NO: 85, SEQ ID NO: 59, SEQ ID NO: 74, SEQ ID NO: 91, SEQ ID NO: 115 and SEQ ID NO: 120;
(33) the fusion light chain includes SEQ ID NO: 38, SEQ ID NO: 104, SEQ ID NO: 89 and SEQ ID NO: 64; the fusion heavy chain includes SEQ ID NO: 37, SEQ ID NO: 111, SEQ ID NO: 83, SEQ ID NO: 63, SEQ ID NO: 74, SEQ ID NO: 91, SEQ ID NO: 117 and SEQ ID NO: 120;
(34) the fusion light chain includes SEQ ID NO: 38, SEQ ID NO: 104, SEQ ID NO: 85 and SEQ ID NO: 64; the fusion heavy chain includes SEQ ID NO: 37, SEQ ID NO: 111, SEQ ID NO: 85, SEQ ID NO: 63, SEQ ID NO: 74, SEQ ID NO: 91, SEQ ID NO: 117 and SEQ ID NO: 120;
(35) the fusion light chain includes SEQ ID NO: 46, SEQ ID NO: 104, SEQ ID NO: 89 and SEQ ID NO: 64; the fusion heavy chain includes SEQ ID NO: 45, SEQ ID NO: 111, SEQ ID NO: 83, SEQ ID NO: 63, SEQ ID NO: 74, SEQ ID NO: 91, SEQ ID NO: 117 and SEQ ID NO: 120;
(36) the fusion light chain includes SEQ ID NO: 46, SEQ ID NO: 104, SEQ ID NO: 85 and SEQ ID NO: 64; the fusion heavy chain includes SEQ ID NO: 45, SEQ ID NO: 111, SEQ ID NO: 85, SEQ ID NO: 63, SEQ ID NO: 74, SEQ ID NO: 91, SEQ ID NO: 115 and SEQ ID NO: 120;
(37) the fusion light chain includes SEQ ID NO: 50, SEQ ID NO: 104, SEQ ID NO: 89 and SEQ ID NO: 48; the fusion heavy chain includes SEQ ID NO: 49, SEQ ID NO: 111, SEQ ID NO: 83, SEQ ID NO: 47, SEQ ID NO: 74, SEQ ID NO: 91, SEQ ID NO: 117 and SEQ ID NO: 120;
(38) the fusion light chain includes SEQ ID NO: 50, SEQ ID NO: 104, SEQ ID NO: 85 and SEQ ID NO: 48; the fusion heavy chain includes SEQ ID NO: 49, SEQ ID NO: 111, SEQ ID NO: 85, SEQ ID NO: 47, SEQ ID NO: 74, SEQ ID NO: 91, SEQ ID NO: 117 and SEQ ID NO: 120;
(39) the fusion light chain includes SEQ ID NO: 50, SEQ ID NO: 104, SEQ ID NO: 85 and SEQ ID NO: 48; the fusion heavy chain includes SEQ ID NO: 49, SEQ ID NO: 111, SEQ ID NO: 85, SEQ ID NO: 47, SEQ ID NO: 74, SEQ ID NO: 91, SEQ ID NO: 115 and SEQ ID NO: 120;
(40) the fusion light chain includes SEQ ID NO: 58, SEQ ID NO: 104, SEQ ID NO: 82 and SEQ ID NO: 41; the fusion heavy chain includes SEQ ID NO: 57, SEQ ID NO: 111, SEQ ID NO: 82, SEQ ID NO: 42, SEQ ID NO: 82, SEQ ID NO: 91, SEQ ID NO: 117 and SEQ ID NO: 120;
(41) the fusion light chain includes SEQ ID NO: 58, SEQ ID NO: 104, SEQ ID NO: 89 and SEQ ID NO: 41; the fusion heavy chain includes SEQ ID NO: 57, SEQ ID NO: 111, SEQ ID NO: 83, SEQ ID NO: 42, SEQ ID NO: 74, SEQ ID NO: 91, SEQ ID NO: 117 and SEQ ID NO: 120;
(42) the fusion light chain includes SEQ ID NO: 58, SEQ ID NO: 104, SEQ ID NO: 85 and SEQ ID NO: 41; the fusion heavy chain includes SEQ ID NO: 57, SEQ ID NO: 111, SEQ ID NO: 85, SEQ ID NO: 42, SEQ ID NO: 74, SEQ ID NO: 91, SEQ ID NO: 117 and SEQ ID NO: 120;
(43) the fusion light chain includes SEQ ID NO: 58, SEQ ID NO: 104, SEQ ID NO: 89 and SEQ ID NO: 45; the fusion heavy chain includes SEQ ID NO: 57, SEQ ID NO: 111, SEQ ID NO: 83, SEQ ID NO: 46, SEQ ID NO: 74, SEQ ID NO: 91, SEQ ID NO: 117 and SEQ ID NO: 120;
(44) the fusion light chain includes SEQ ID NO: 58, SEQ ID NO: 104, SEQ ID NO: 85 and SEQ ID NO: 45; the fusion heavy chain includes SEQ ID NO: 57, SEQ ID NO: 111, SEQ ID NO: 85, SEQ ID NO: 46, SEQ ID NO: 74, SEQ ID NO: 91, SEQ ID NO: 117 and SEQ ID NO: 120;
(45) the fusion light chain includes SEQ ID NO: 42, SEQ ID NO: 104, SEQ ID NO: 89 and SEQ ID NO: 57; the fusion heavy chain includes SEQ ID NO: 41, SEQ ID NO: 111, SEQ ID NO: 83, SEQ ID NO: 58, SEQ ID NO: 74, SEQ ID NO: 91, SEQ ID NO: 117 and SEQ ID NO: 120;
(46) the fusion light chain includes SEQ ID NO: 42, SEQ ID NO: 104, SEQ ID NO: 85 and SEQ ID NO: 57; the fusion heavy chain includes SEQ ID NO: 41, SEQ ID NO: 111, SEQ ID NO: 85, SEQ ID NO: 58, SEQ ID NO: 74, SEQ ID NO: 91, SEQ ID NO: 117 and SEQ ID NO: 120;
(47) the fusion light chain includes SEQ ID NO: 46, SEQ ID NO: 104, SEQ ID NO: 89 and SEQ ID NO: 57; the fusion heavy chain includes SEQ ID NO: 45, SEQ ID NO: 111, SEQ ID NO: 83, SEQ ID NO: 58, SEQ ID NO: 74, SEQ ID NO: 91, SEQ ID NO: 117 and SEQ ID NO: 120;
(48) the fusion light chain includes SEQ ID NO: 46, SEQ ID NO: 104, SEQ ID NO: 85 and SEQ ID NO: 57; the fusion heavy chain includes SEQ ID NO: 45, SEQ ID NO: 111, SEQ ID NO: 85, SEQ ID NO: 58, SEQ ID NO: 74, SEQ ID NO: 91, SEQ ID NO: 117 and SEQ ID NO: 120;
(49) the fusion light chain includes SEQ ID NO: 42, SEQ ID NO: 104, SEQ ID NO: 85 and SEQ ID NO: 53; the fusion heavy chain includes SEQ ID NO: 41, SEQ ID NO: 111, SEQ ID NO: 85, SEQ ID NO:54, SEQ ID NO:74, SEQ ID NO:91, SEQ ID NO:117 and SEQ ID NO:120;
(50) the fusion light chain includes SEQ ID NO: 42, SEQ ID NO: 104, SEQ ID NO: 84 and SEQ ID NO: 57; the fusion heavy chain includes SEQ ID NO: 41, SEQ ID NO: 111, SEQ ID NO: 84, SEQ ID NO: 58, SEQ ID NO: 74, SEQ ID NO: 91, SEQ ID NO: 117 and SEQ ID NO: 120;
(51) the fusion light chain includes SEQ ID NO: 42, SEQ ID NO: 104, SEQ ID NO: 86 and SEQ ID NO: 57; the fusion heavy chain includes SEQ ID NO: 41, SEQ ID NO: 111, SEQ ID NO: 86, SEQ ID NO: 58, SEQ ID NO: 74, SEQ ID NO: 91, SEQ ID NO: 117 and SEQ ID NO: 120;
(52) the fusion light chain includes SEQ ID NO: 42, SEQ ID NO: 104, SEQ ID NO: 85 and SEQ ID NO: 57; the fusion heavy chain includes SEQ ID NO: 41, SEQ ID NO: 111, SEQ ID NO: 85, SEQ ID NO: 58, SEQ ID NO: 74, SEQ ID NO: 91, SEQ ID NO: 115 and SEQ ID NO: 120;
(53) the fusion light chain includes SEQ ID NO: 42, SEQ ID NO: 104, SEQ ID NO: 85 and SEQ ID NO: 67; the fusion heavy chain includes SEQ ID NO: 41, SEQ ID NO: 111, SEQ ID NO: 85, SEQ ID NO: 68, SEQ ID NO: 74, SEQ ID NO: 91, SEQ ID NO: 117 and SEQ ID NO: 120;
(54) the fusion light chain includes SEQ ID NO: 68, SEQ ID NO: 104, SEQ ID NO: 85 and SEQ ID NO: 57; the fusion heavy chain includes SEQ ID NO: 67, SEQ ID NO: 111, SEQ ID NO: 85, SEQ ID NO: 58, SEQ ID NO: 74, SEQ ID NO: 91, SEQ ID NO: 117 and SEQ ID NO: 120;
(55) the fusion light chain includes SEQ ID NO: 68, SEQ ID NO: 104, SEQ ID NO: 85 and SEQ ID NO: 57; the fusion heavy chain includes SEQ ID NO: 67, SEQ ID NO: 111, SEQ ID NO: 85, SEQ ID NO: 58, SEQ ID NO: 74, SEQ ID NO: 91, SEQ ID NO: 115 and SEQ ID NO: 120;
(56) the fusion light chain includes SEQ ID NO: 42, SEQ ID NO: 104, SEQ ID NO: 89 and SEQ ID NO: 59; the fusion heavy chain includes SEQ ID NO: 41, SEQ ID NO: 111, SEQ ID NO: 83, SEQ ID NO: 60, SEQ ID NO: 74, SEQ ID NO: 91, SEQ ID NO: 117 and SEQ ID NO: 120;
(57) the fusion light chain includes SEQ ID NO: 42, SEQ ID NO: 104, SEQ ID NO: 85 and SEQ ID NO: 59; the fusion heavy chain includes SEQ ID NO: 41, SEQ ID NO: 111, SEQ ID NO: 85, SEQ ID NO: 60, SEQ ID NO: 74, SEQ ID NO: 91, SEQ ID NO: 117 and SEQ ID NO: 120;
(58) the fusion light chain includes SEQ ID NO: 46, SEQ ID NO: 104, SEQ ID NO: 89 and SEQ ID NO: 59; the fusion heavy chain includes SEQ ID NO: 45, SEQ ID NO: 111, SEQ ID NO: 83, SEQ ID NO: 60, SEQ ID NO: 74, SEQ ID NO: 91, SEQ ID NO: 117 and SEQ ID NO: 120;
(59) the fusion light chain includes SEQ ID NO: 46, SEQ ID NO: 104, SEQ ID NO: 85 and SEQ ID NO: 59; the fusion heavy chain includes SEQ ID NO: 45, SEQ ID NO: 111, SEQ ID NO: 85, SEQ ID NO: 60, SEQ ID NO: 74, SEQ ID NO: 91, SEQ ID NO: 117 and SEQ ID NO: 120;
(60) the fusion light chain includes SEQ ID NO: 42, SEQ ID NO: 104, SEQ ID NO: 84 and SEQ ID NO: 59; the fusion heavy chain includes SEQ ID NO: 41, SEQ ID NO: 111, SEQ ID NO: 84, SEQ ID NO: 60, SEQ ID NO: 74, SEQ ID NO: 91, SEQ ID NO: 117 and SEQ ID NO: 120;
(61) the fusion light chain includes SEQ ID NO: 42, SEQ ID NO: 104, SEQ ID NO: 86 and SEQ ID NO: 59; the fusion heavy chain includes SEQ ID NO: 41, SEQ ID NO: 111, SEQ ID NO: 86, SEQ ID NO: 60, SEQ ID NO: 74, SEQ ID NO: 91, SEQ ID NO: 117 and SEQ ID NO: 120;
(62) the fusion light chain includes SEQ ID NO: 42, SEQ ID NO: 104, SEQ ID NO: 89 and SEQ ID NO: 33; the fusion heavy chain includes SEQ ID NO: 41, SEQ ID NO: 111, SEQ ID NO: 83, SEQ ID NO: 34, SEQ ID NO: 74, SEQ ID NO: 91, SEQ ID NO: 117 and SEQ ID NO: 120;
(63) the fusion light chain includes SEQ ID NO: 42, SEQ ID NO: 104, SEQ ID NO: 86 and SEQ ID NO: 33; the fusion heavy chain includes SEQ ID NO: 41, SEQ ID NO: 111, SEQ ID NO: 86, SEQ ID NO: 34, SEQ ID NO: 74, SEQ ID NO: 91, SEQ ID NO: 117 and SEQ ID NO: 120;
(64) the fusion light chain includes SEQ ID NO: 42, SEQ ID NO: 104, SEQ ID NO: 86 and SEQ ID NO: 33; the fusion heavy chain includes SEQ ID NO: 41, SEQ ID NO: 111, SEQ ID NO: 86, SEQ ID NO: 34, SEQ ID NO: 74, SEQ ID NO: 91, SEQ ID NO: 115 and SEQ ID NO: 120;
(65) the fusion light chain includes SEQ ID NO: 38, SEQ ID NO: 104, SEQ ID NO: 89 and SEQ ID NO: 27; the fusion heavy chain includes SEQ ID NO: 37, SEQ ID NO: 111, SEQ ID NO: 83, SEQ ID NO: 28, SEQ ID NO: 74, SEQ ID NO: 91, SEQ ID NO: 117 and SEQ ID NO: 120;
(66) the fusion light chain includes SEQ ID NO: 38, SEQ ID NO: 104, SEQ ID NO: 85 and SEQ ID NO: 27; the fusion heavy chain includes SEQ ID NO: 37, SEQ ID NO: 111, SEQ ID NO: 85, SEQ ID NO: 28, SEQ ID NO: 74, SEQ ID NO: 91, SEQ ID NO: 117 and SEQ ID NO: 120;
(67) the fusion light chain includes SEQ ID NO: 38, SEQ ID NO: 104, SEQ ID NO: 85 and SEQ ID NO: 27; the fusion heavy chain includes SEQ ID NO: 37, SEQ ID NO: 111, SEQ ID NO: 85, SEQ ID NO: 28, SEQ ID NO: 74, SEQ ID NO: 91, SEQ ID NO: 115 and SEQ ID NO: 120;
(68) the fusion light chain includes SEQ ID NO: 38, SEQ ID NO: 104, SEQ ID NO: 89 and SEQ ID NO: 29; the fusion heavy chain includes SEQ ID NO: 37, SEQ ID NO: 111, SEQ ID NO: 83, SEQ ID NO: 30, SEQ ID NO: 74, SEQ ID NO: 91, SEQ ID NO: 117 and SEQ ID NO: 120;
(69) the fusion light chain includes SEQ ID NO: 38, SEQ ID NO: 104, SEQ ID NO: 85 and SEQ ID NO: 29; the fusion heavy chain includes SEQ ID NO: 37, SEQ ID NO: 111, SEQ ID NO: 85, SEQ ID NO: 30, SEQ ID NO: 74, SEQ ID NO: 91, SEQ ID NO: 117 and SEQ ID NO: 120;
(70) the fusion light chain includes SEQ ID NO: 38, SEQ ID NO: 104, SEQ ID NO: 85 and SEQ ID NO: 29; the fusion heavy chain includes SEQ ID NO: 37, SEQ ID NO: 111, SEQ ID NO: 85, SEQ ID NO: 30, SEQ ID NO: 74, SEQ ID NO: 91, SEQ ID NO: 115 and SEQ ID NO: 120;
(71) the fusion light chain includes SEQ ID NO: 38, SEQ ID NO: 104, SEQ ID NO: 89 and SEQ ID NO: 57; the fusion heavy chain includes SEQ ID NO: 37, SEQ ID NO: 111, SEQ ID NO: 83, SEQ ID NO: 58, SEQ ID NO: 74, SEQ ID NO: 91, SEQ ID NO: 117 and SEQ ID NO: 120;
(72) the fusion light chain includes SEQ ID NO: 38, SEQ ID NO: 104, SEQ ID NO: 85 and SEQ ID NO: 57; the fusion heavy chain includes SEQ ID NO: 37, SEQ ID NO: 111, SEQ ID NO: 85, SEQ ID NO: 58, SEQ ID NO: 74, SEQ ID NO: 91, SEQ ID NO: 117 and SEQ ID NO: 120;
(73) the fusion light chain includes SEQ ID NO: 38, SEQ ID NO: 104, SEQ ID NO: 89 and SEQ ID NO: 59; the fusion heavy chain includes SEQ ID NO: 37, SEQ ID NO: 111, SEQ ID NO: 83, SEQ ID NO: 60, SEQ ID NO: 74, SEQ ID NO: 91, SEQ ID NO: 117 and SEQ ID NO: 120;
(74) the fusion light chain includes SEQ ID NO: 38, SEQ ID NO: 104, SEQ ID NO: 85 and SEQ ID NO: 59; the fusion heavy chain includes SEQ ID NO: 37, SEQ ID NO: 111, SEQ ID NO: 85, SEQ ID NO: 60, SEQ ID NO: 74, SEQ ID NO: 91, SEQ ID NO: 117 and SEQ ID NO: 120;
(75) the fusion light chain includes SEQ ID NO: 38, SEQ ID NO: 104, SEQ ID NO: 85 and SEQ ID NO: 59; the fusion heavy chain includes SEQ ID NO: 37, SEQ ID NO: 111, SEQ ID NO: 85, SEQ ID NO: 60, SEQ ID NO: 74, SEQ ID NO: 91, SEQ ID NO: 115 and SEQ ID NO: 120;
(76) the fusion light chain includes SEQ ID NO: 38, SEQ ID NO: 104, SEQ ID NO: 89 and SEQ ID NO: 63; the fusion heavy chain includes SEQ ID NO: 37, SEQ ID NO: 111, SEQ ID NO: 83, SEQ ID NO: 64, SEQ ID NO: 74, SEQ ID NO: 91, SEQ ID NO: 117 and SEQ ID NO: 120;
(77) the fusion light chain includes SEQ ID NO: 38, SEQ ID NO: 104, SEQ ID NO: 85 and SEQ ID NO: 63; the fusion heavy chain includes SEQ ID NO: 37, SEQ ID NO: 111, SEQ ID NO: 85, SEQ ID NO: 64, SEQ ID NO: 74, SEQ ID NO: 91, SEQ ID NO: 115 and SEQ ID NO: 120;
(78) the fusion light chain includes SEQ ID NO: 46, SEQ ID NO: 104, SEQ ID NO: 89 and SEQ ID NO: 63; the fusion heavy chain includes SEQ ID NO: 45, SEQ ID NO: 111, SEQ ID NO: 83, SEQ ID NO: 64, SEQ ID NO: 74, SEQ ID NO: 91, SEQ ID NO: 117 and SEQ ID NO: 120;
(79) the fusion light chain includes SEQ ID NO: 46, SEQ ID NO: 104, SEQ ID NO: 85 and SEQ ID NO: 63; the fusion heavy chain includes SEQ ID NO: 45, SEQ ID NO: 111, SEQ ID NO: 85, SEQ ID NO: 64, SEQ ID NO: 74, SEQ ID NO: 91, SEQ ID NO: 117 and SEQ ID NO: 120;
(80) the fusion light chain includes SEQ ID NO: 46, SEQ ID NO: 104, SEQ ID NO: 85 and SEQ ID NO: 63; the fusion heavy chain includes SEQ ID NO: 45, SEQ ID NO: 111, SEQ ID NO: 85, SEQ ID NO: 64, SEQ ID NO: 74, SEQ ID NO: 91, SEQ ID NO: 115 and SEQ ID NO: 120;
(80) the fusion light chain includes SEQ ID NO: 50, SEQ ID NO: 104, SEQ ID NO: 89 and SEQ ID NO: 47; the fusion heavy chain includes SEQ ID NO: 49, SEQ ID NO: 111, SEQ ID NO: 83, SEQ ID NO: 48, SEQ ID NO: 74, SEQ ID NO: 91, SEQ ID NO: 117 and SEQ ID NO: 120;
(81) the fusion light chain includes SEQ ID NO: 50, SEQ ID NO: 104, SEQ ID NO: 85 and SEQ ID NO: 47; the fusion heavy chain includes SEQ ID NO: 49, SEQ ID NO: 111, SEQ ID NO: 85, SEQ ID NO: 48, SEQ ID NO: 74, SEQ ID NO: 91, SEQ ID NO: 117 and SEQ ID NO: 120;
(82) the fusion light chain includes SEQ ID NO: 50, SEQ ID NO: 104, SEQ ID NO: 85 and SEQ ID NO: 47; the fusion heavy chain includes SEQ ID NO: 49, SEQ ID NO: 111, SEQ ID NO: 85, SEQ ID NO: 48, SEQ ID NO: 74, SEQ ID NO: 91, SEQ ID NO: 115 and SEQ ID NO: 120;
(83) the fusion light chain includes SEQ ID NO: 147, SEQ ID NO: 104, SEQ ID NO: 85 and SEQ ID NO: 149; the fusion heavy chain includes SEQ ID NO: 146, SEQ ID NO: 111, SEQ ID NO: 85, SEQ ID NO: 148, SEQ ID NO: 82, SEQ ID NO: 91, SEQ ID NO: 117 and SEQ ID NO: 120;
(84) the fusion light chain includes SEQ ID NO: 149, SEQ ID NO: 104, SEQ ID NO: 85 and SEQ ID NO: 147; the fusion heavy chain includes SEQ ID NO: 148, SEQ ID NO: 111, SEQ ID NO: 85, SEQ ID NO: 146, SEQ ID NO: 82, SEQ ID NO: 91, SEQ ID NO: 117 and SEQ ID NO: 120;
(85) the fusion light chain includes SEQ ID NO: 147, SEQ ID NO: 104, SEQ ID NO: 85 and SEQ ID NO: 149; the fusion heavy chain includes SEQ ID NO: 146, SEQ ID NO: 111, SEQ ID NO: 85, SEQ ID NO: 148, SEQ ID NO: 82, SEQ ID NO: 91, SEQ ID NO: 115 and SEQ ID NO: 120;
(86) the fusion light chain includes SEQ ID NO: 149, SEQ ID NO: 104, SEQ ID NO: 85 and SEQ ID NO: 147; the fusion heavy chain includes SEQ ID NO: 148, SEQ ID NO: 111, SEQ ID NO: 85, SEQ ID NO: 146, SEQ ID NO: 82, SEQ ID NO: 91, SEQ ID NO: 115 and SEQ ID NO: 120.
(87) the fusion light chain includes SEQ ID NO: 147, SEQ ID NO: 104, SEQ ID NO: 85 and SEQ ID NO: 148; the fusion heavy chain includes SEQ ID NO: 146, SEQ ID NO: 111, SEQ ID NO: 85, SEQ ID NO: 149, SEQ ID NO: 87, SEQ ID NO: 91, SEQ ID NO: 117 and SEQ ID NO: 120;
(88) the fusion light chain includes SEQ ID NO: 149, SEQ ID NO: 104, SEQ ID NO: 85 and SEQ ID NO: 146; the fusion heavy chain includes SEQ ID NO: 148, SEQ ID NO: 111, SEQ ID NO: 85, SEQ ID NO: 147, SEQ ID NO: 82, SEQ ID NO: 91, SEQ ID NO: 117 and SEQ ID NO: 120;
(89) the fusion light chain includes SEQ ID NO: 147, SEQ ID NO: 104, SEQ ID NO: 85 and SEQ ID NO: 148; the fusion heavy chain includes SEQ ID NO: 146, SEQ ID NO: 111, SEQ ID NO: 85, SEQ ID NO: 149, SEQ ID NO: 82, SEQ ID NO: 91, SEQ ID NO: 115 and SEQ ID NO: 120; or
(90) the fusion light chain includes SEQ ID NO: 149, SEQ ID NO: 104, SEQ ID NO: 85 and SEQ ID NO: 146; the fusion heavy chain includes SEQ ID NO: 148, SEQ ID NO: 111, SEQ ID NO: 85, SEQ ID NO: 147, SEQ ID NO: 82, SEQ ID NO: 91, SEQ ID NO: 115 and SEQ ID NO: 120.

13. A pharmaceutical composition comprising the bispecific antibody of any one of claims 1 to 12, preferably, dosage form of the pharmaceutical composition includes a dosage form for gastrointestinal administration or a dosage form for parenteral administration; more preferably, dosage form of the pharmaceutical composition is an injection, including intravenous injection, intravenous drip, subcutaneous injection, local injection, intramuscular injection, intratumor injection, intraperitoneal injection, intracranial injection, or intracavity injection.

14. A conjugate or a fusion protein comprising the bispecific antibody of any one of claims 1 to 12.

15. The conjugate or fusion protein of claim 14, which comprises a substance A conjugated or fused to the antibody, and the substance A is selected from therapeutic agents, prodrugs, proteins (such as enzymes), viruses, lipids , biological response modifiers (such as immunomodulators), PEG, hormones, oligonucleotides, diagnostic agents, cytotoxic agents that can be drugs or toxins, ultrasound enhancers, non-radioactive markers, detectable markers, such as chemiluminescent labeling compounds (such as luminol, isoluminol, thermal acridinium esters, imidazoles, acridinium salts and oxalates), or fluorescence emitting metals (such as 152Eu, or lanthanide labels).

16. A polynucleotide encoding the bispecific antibody of any one of claims 1 to 12.

17. A cell comprising the polynucleotide of claim 16.

18. Use of the bispecific antibody of any one of claims 1 to 12 or the conjugate or fusion protein of any one of claims 14 to 15 in the treatment of tumors (cancers) or in the preparation of drugs for the treatment of tumors (cancers).

19. Use of the bispecific antibody of any one of claims 1 to 12 or the conjugate or fusion protein of any one of claims 14 to 15 in the preparation of reagents or kits for detecting tumors (cancers).
